# EUROPEAN PATENT APPLICATION

(11) **EP 4 224 020 A1**
(43) Date of publication of application: **09.08.2023**
(21) Application number: 21874342.5
(22) Date of filing: 24.09.2021
(51) Int. Cl.: F15B 11/08, F15B 13/02, F15B 21/00, F15B 21/02, F15B 19/00, C12Q 1/6869, C12M 1/36, C12M 1/34, C12M 1/04, C12M 1/00, F16K 11/065, F16K 11/08, F16K 31/02, B01L 3/00

(54) **FLOW PATH SELECTION VALUE, SYSTEM AND METHOD, STORAGE MEDIUM, AND APPLICATION**

(30) Priority: 29.09.2020 CN 202011052660; 29.09.2020 CN 202011052677; 31.12.2020 CN 202011617667; 31.12.2020 CN 202011617771
(71) Applicant: GeneMind Biosciences Company Limited, Luohu District, Shenzhen, Guangdong 518000 (CN)
(72) Inventor: WAN, Xuefeng, Shenzhen, Guangdong 518000 (CN); WU, Ping, Shenzhen, Guangdong 518000 (CN); JIANG, Zefei, Shenzhen, Guangdong 518000 (CN)
(74) Representative: Schiweck Weinzierl Koch Patentanwälte Partnerschaft mbB
(86) International application number: PCT/CN2021/120191
(87) International publication number: WO 2022/068681

(57) **Abstract**

A liquid path system (12), comprising a flow path selection valve (10), a pump assembly (14), and a fluid network (60). The fluid network (60) comprises a reservoir (64), a first flow channel, a second flow channel, and a reaction device (62). The first flow channel and the second flow channel are each independently in flow communication with the reservoir (64) and the reaction device (62). The pump assembly (14) is in communication with the flow path selection valve (10). The pump assembly (14) is in communication with the fluid network (60). The flow path selection valve (10) is configured to rotate between a first valve position and a second valve position. In the case that the flow path selection valve (10) is at the first valve position, the pump assembly (14) induces the liquid in the reservoir (64) to flow to the reaction device (62) through the first flow channel, and in the case that the flow path selection valve (10) is at the second valve position, the pump assembly (14) induces the liquid in the reservoir (64) to flow to the reaction device (62) through the second flow channel.

## Description

### FIELD

The present application relates to the field of machinery and fluid technology, and more particularly, to a flow path selection valve, a system, a method, a storage medium and use.

### BACKGROUND

In the related art, the nucleic acid detection of a nucleic acid sample placed on a solid substrate, for example, the nucleic acid sequence determination (sequencing) based on chip detection, generally includes what are called sample processing before loading on a machine and on-line sequencing, where the sample processing before loading on a machine is to process the nucleic acid sample under test to meet the requirements for on-line sequencing, for example, to attach the nucleic acid sample under test to the surface of the solid substrate, and the on-line sequencing refers to putting the processed nucleic acid sample under test into a sequencer for sequencing.

Current commercial sequencing platforms typically include two or more independent or functionally linked apparatuses to accomplish the sample processing and sequencing described above. For a sequencing platform including two matched apparatuses, typically, one of the apparatuses is for processing a nucleic acid sample under test, including attaching it to a reaction device, such as a chip, and the apparatus is often referred to as a hybridization instrument, a sample loading instrument, a library construction instrument, a sample processing instrument or a sample preparation instrument; the other apparatus detects the chip connected to the nucleic acid sample under test and output by the last apparatus to perform nucleic acid sequence determination, and the apparatus is the most important hardware of the sequencing platform, namely what is generally called a sequencer.

For current commercial sequencing platforms, these independent and functionally linked apparatuses can be sold or purchased together or separately/in part. Purchasing multiple associated apparatuses is generally expensive, and purchasing one or a part of the apparatuses, such as purchasing the sequencer only, requires additional manual operations or commercial sample processing service and usually requires more user operations or involvement.

Given the development of sequencing technologies, automation and other related technologies and the expectation for simple operation and the like from the market, a sequencer integrating at least a part of the sample processing before loading on a machine and sequencing functions is a development direction (hereinafter, the sequencer with integrated functions is referred to as an integrated sequencer or an integrated sequencing platform). Particularly, compared with a sequencing platform before integration, the integrated sequencer is not weakened in performance and is equivalent or smaller in volume and simpler and more convenient to operate.

However, at present, there are few mature integrated sequencers with integrated functionality due to at least the following factor: sample processing before loading on a machine and sequencing generally include a plurality of steps and a plurality of reactions and relate to switching of a plurality of reagents, a plurality of serial or parallel liquid inlet and outlet sequence control and microfluidic control.

Therefore, how to construct a system that integrates at least a part of the sample processing before loading on a machine and sequencing functions and allow the system or an integrated sequencer including the system to have performance equivalent to that before integration, exhibit a certain quality level and maintain cost in a certain range is worth attention.

### SUMMARY

In the process of designing and testing an integrated fluid path for sample processing before loading on a machine and on-line sequencing, the inventors of the present application find that an integrated fluid path capable of accurately controlling a trace amount of fluid, particularly an integrated fluid path capable of independently or alternately controlling reactions in a plurality of reactors (reaction devices/reaction cells) and/or controlling fluid environments of reactions of different stages in different regions of one reactor, involves a considerable number of valve bodies, and thus the associated tubes are large in number and are relatively complicated due to interlacing, the integrated fluid path thus constructed occupies a relatively large space, and it is difficult to achieve good cooperation at a predicted volume when the integrated fluid path is matched with other functional modules or systems to construct a complete machine (integrated sequencer).

Accordingly, embodiments of the present application provide a flow path selection valve, a system, a method, a storage medium and use.

A fluid path system according to an embodiment of the present application includes a fluid network, a pump assembly in communication with the fluid network, and a flow path selection valve. The fluid network includes a reservoir, a first flow channel, a second flow channel, and a reaction device, and the first flow channel and the second flow channel are each independently in flow communication with the reservoir and the reaction device. The flow path selection valve is configured to rotate between a first valve position and a second valve position. In the case that the flow path selection valve is at the first valve position, the pump assembly induces liquid in the reservoir to flow toward the reaction device through the first flow channel, and in the case that the flow path selection valve is at the second valve position, the pump assembly induces liquid in the reservoir to flow toward the reaction device through the second flow channel.

In the fluid path system according to any one of the embodiments described above, the flow path selection valve may be used to perform independent or parallel three-way control on the first flow channel and the second flow channel, and it may be avoided that a plurality of three-way valves are simultaneously provided in the fluid path system, so that the fluid path system has decreased cost, reduced size, less reagent consumption and improved reliability, and is convenient to maintain, repair and control. The fluid path system according to any one of the embodiments described above is particularly suitable for fluid networks involving multi-channel control, and systems requiring high precision in fluid control and delivery, such as sequencing systems (nucleic acid sequence determination systems).

The detection system according to an embodiment of the present application includes the fluid path system according to any one of the embodiments described above, a detection assembly, and a controller. The detection assembly is configured to detect a signal from the reaction device during a designated reaction, and the controller is configured to control the pump assembly and the flow path selection valve to allow the liquid in the reservoir to flow toward the reaction device through the first flow channel, to control the pump assembly and the flow path selection valve to allow the liquid in the reservoir to flow toward the reaction device through the second flow channel, and to control the detection assembly to detect a signal from the reaction device when the liquid in the reservoir flows through the reaction device or after the liquid in the reservoir flows through the reaction device. A detection system including any of the fluid path systems described above enables independent control of different reaction processes of different parts/regions of the reaction device, and is low in manufacturing cost.

Provided is use of the fluid path system according to any one of the embodiments above or the detection system according to any one of the embodiments above in nucleic acid sequence determination.

An embodiment of the present application provides a flow path selection valve. The flow path selection valve is configured to switch between a first valve position and a second valve position and is provided with a common port, a plurality of first ports, a plurality of second ports, and a plurality of communication grooves. The communication groove selectively allows the common port to be in communication with the first port or allow the first port to be in communication with the second port, the first port is in communication with the second port through the communication groove in the case that the flow path selection valve is at the first valve position, and the first port is in communication with the common port through the communication groove in the case that the flow path selection valve is at the second valve position. The flow path selection valve enables three-way control over a plurality of fluid paths (flow paths), and is particularly suitable for systems that include a plurality of fluid paths and require independent or parallel control of the plurality of fluid paths.

In the flow path selection valve according to any one of the embodiments described above, each first port is in communication with the corresponding second port through the corresponding communication groove in the case that the flow path selection valve is at the first valve position, and each first port is in communication with the common port through the corresponding communication groove in the case that the flow path selection valve is at the second valve position, so that the flow path selection valve has two or more channels. The flow path selection valve is suitable for any fluid path system requiring the use of a three-way valve, in particular a plurality of three-way valves, for example, a fluid path system involving the change of the direction of one or more flow paths and/or the dispensing of a plurality of liquids/solutions. The application of the flow path selection valve to a fluid path system including a plurality of fluid paths/flow paths enables independent or parallel three-way control on the plurality of fluid paths, and it may be avoided that a plurality of three-way valves are simultaneously provided in the fluid path system, so that the fluid path system has decreased cost, reduced size, less reagent consumption and improved reliability, and is convenient to maintain, repair and control. Any of the flow path selection valves and/or any of the fluid path systems including the flow path selection valve described above are particularly suitable for systems including a plurality of flow paths and requiring high precision in fluid control and delivery, such as sequencing systems (nucleic acid sequence determination systems). A sequencing system including any of the flow path selection valves described above enables independent control of different reaction processes of different parts/regions of the flow cell, and is low in manufacturing cost.

The fluid path system according to an embodiment of the present application includes: a plurality of flow paths, a flow path selection valve connected to the flow paths, a pump, and a control circuit. The plurality of flow paths are in fluid connection with a flow cell to support a target analyte when the flow cell is installed in the fluid system. The flow path selection valve includes a manifold provided with a common port, a plurality of first ports, and a plurality of second ports, and a first valve element provided with a communication groove, the first valve element may rotate or slide relative to the manifold to switch the flow path selection valve between a first valve position and a second valve position and thereby to select between the flow paths, the first port is in communication with the second port through the communication groove in the case that the flow path selection valve is at the first valve position, and the first port is in communication with the common port through the communication groove in the case that the flow path selection valve is at the second valve position. The pump is in fluid connection with the flow cell when the flow cell is installed in the fluid system and allows liquid to flow through a flow path selected by the flow path selection valve during an analysis operation. The control circuit is operatively coupled to the flow path selection valve and is configured to control the flow path selection valve to select a designated flow path.

The sequencing system according to an embodiment of the present application includes the fluid path system according to any one of the embodiments described above. In the fluid path system according to the embodiment described above, the flow path selection valve can be used to perform independent or parallel three-way control on the plurality of fluid paths, and it may be avoided that a plurality of three-way valves are simultaneously provided in the fluid path system, so that the fluid path system has decreased cost, reduced size, less reagent consumption and improved reliability, and is convenient to maintain, repair and control. The fluid path system described above is particularly suitable for systems including a plurality of flow paths and requiring high precision in fluid control and delivery, such as sequencing systems (nucleic acid sequence determination systems). A sequencing system including the fluid path system described above enables independent control of different reaction processes of different parts/regions of the flow cell, and is low in manufacturing cost.

Provided is a method for controlling a system to achieve sequencing according to an embodiment of the present application. The system includes a plurality of flow paths, a flow cell connected to the plurality of flow paths, a flow path selection valve and a pump, a first reservoir, and a second reservoir. The flow path selection valve includes a manifold and a first valve element, the manifold is provided with a common port, a plurality of first ports, and a plurality of second ports, the first valve element is provided with a communication groove, and the first valve element may rotate or slide relative to the manifold, so that the communication groove selectively allows the common port to be in communication with the first port, or allows the first port to be in communication with the second port, thereby selecting different flow paths. The first reservoir stores a sample solution under test and is connected to the second ports. The second reservoir stores a reaction reagent and is connected to the common port. The first reservoir carries a first reaction solution, and the first reaction liquid contains a nucleic acid molecule. The second reservoir carries a second reaction solution, and the second reaction solution contains components necessary for nucleic acid sequencing. The pump is configured to allow the liquid to flow through the flow path selected by the flow path selection valve. The method includes: switching the flow path selection valve to a first valve position, so that the communication groove allows the first port to be in communication with the second port, and thus allows the first reservoir to be in communication with the flow cell; controlling the pump to operate to allow the first reaction solution to enter the flow cell to perform a first reaction in the case that the flow path selection valve is at the first valve position, the first reaction including attaching at least a portion of the nucleic acid molecule to the flow cell; switching the flow path selection valve to a second valve position, so that the communication groove allows the first port to be in communication with the common port, and thus allows the second reservoir to be in communication with the flow cell; controlling the pump to operate to allow the second reaction solution to enter the flow cell to perform a second reaction in the case that the flow path selection valve is at the second valve position, the second reaction including allowing the nucleic acid molecule in the flow cell after the first reaction is performed to interact with the second reaction solution to perform a polymerization reaction and detecting a signal from the reaction, so as to achieve sequence determination of the nucleic acid molecule.

A method for determining a nucleic acid sequence according to an embodiment of the present application includes: rotating a flow path selection valve provided with a first flow channel and a second flow channel to a first valve position to allow a first reservoir to be in communication with a reaction device, the first reservoir carrying a first reaction solution, and the first reaction solution containing a nucleic acid molecule; allowing the first reaction solution to enter the reaction device through the first flow channel to perform a first reaction in the case that the flow path selection valve is at the first valve position, the first reaction including attaching at least a portion of the nucleic acid molecule to the reaction device; rotating the flow path selection valve to a second valve position to allow a second reservoir to be in communication with the reaction device, the second reservoir carrying a second reaction solution, and the second reaction solution containing components necessary for nucleic acid sequencing; and allowing the second reaction solution to enter the reaction device through the second flow channel to perform a second reaction in the case that the flow path selection valve is at the second valve position, the second reaction including allowing the nucleic acid molecule in the reaction device after the first reaction is performed to interact with the second reaction solution to perform a polymerization reaction and detecting a signal from the reaction, so as to achieve sequence determination of the nucleic acid molecule.

The method according to any one of the embodiments described above implements multiple reactions by designing and controlling a fluid path system, including configuring a flow path selection valve containing a plurality of independent flow channels and controlling the flow path selection valve to be at a first valve position or a second valve position to allow a solution reservoir to be in communication with a reaction device through a designated flow channel to perform various reactions. The method integrates and simplifies the fluid path system of various reactions by configuring a specific flow path selection valve, so that the simple operation of the flow path selection valve or the fluid path system containing the flow path selection valve can realize various reactions, and it is particularly suitable for an automated biomolecule detection system involving various reactions, such as a nucleic acid sequencing system.

By allowing the flow path selection valve to be at a designated valve position and thereby allowing designated flow channels to be accessible for different reactions, the method enables nucleic acid sequencing involving a solid carrier, including attaching the nucleic acid to or immobilizing it on the surface of the solid carrier and performing sequence determination on the nucleic acid molecule on the surface of the solid carrier using sequencing by synthesis (SBS) or sequencing by ligation (SBL). The hardware configuration required for automatically realizing the method is simple and easy to realize, which is beneficial to batch manufacturing and industrialization.

The nucleic acid sequence determination system according to an embodiment of the present application includes a fluid path system, a detection assembly and a controller connected to each other. The fluid path system includes a fluid network and a pump assembly in communication with the fluid network. The fluid network includes a first reservoir, a second reservoir, a flow path selection valve, and a reaction device. The flow path selection valve is provided with a first flow channel and a second flow channel, the first flow channel allows the first reservoir to be in communication with the reaction device, and the second flow channel allows the second reservoir to be in communication with the reaction device. The detection assembly is configured to detect a signal from the reaction device during a designated reaction. The controller is configured to control the fluid path system and the detection assembly to perform the steps of the nucleic acid sequence determination method according to any one of the embodiments described above.

A computer-readable storage medium according to an embodiment of the present application is configured to store a program executed by a computer, and executing the program includes implementing the nucleic acid sequence determination method according to any one of the embodiments described above.

The present application provides a system configured to perform the nucleic acid sequence determination method according to any one of the embodiments described above. A computer program product including instructions according to an embodiment of the present application includes performing the nucleic acid sequence determination method according to any one of the embodiments described above when the program is executed by a computer. A nucleic acid sequence determination system according to an embodiment of the present application includes the computer program product according to any one of the embodiments described above.

The method, system, computer-readable storage medium, and computer program product for determining nucleic acid sequences according to any one of the embodiments described above, in which the first flow channel and the first flow channel are controlled independently or in parallel by controlling the flow path selection valve, are particularly suitable for fluid systems involving multi-site three-way control, and can effectively reduce the number of valve bodies that need to be provided in a fluid path involving multi-site flow splitting and flow confluence, thereby enabling the performing of various reactions while facilitating the arrangement of corresponding hardware structures for automation and industrialization. Generally, the fluid path system or apparatus including the flow path selection valve that can realize the method described above features low manufacturing cost, small size, low reagent consumption, high reliability, and convenience in maintenance, repair, and control; by using the automated sequencing system including any of the flow path selection valves or any of the fluid path systems, the flow channels are controlled and selected by allowing the flow path selection valve to be in different valve positions, so that the flow directions of different liquids can be independently controlled and operated, and the sequence determination of nucleic acid molecules is easier to perform.

The additional aspects and advantages of the embodiments of the present application will be partially set forth in the following description, and will partially become apparent from the following description or be appreciated by practice of the embodiments of the present application.

### BRIEF DESCRIPTION OF THE DRAWINGS

FIG. 1 is an exploded schematic view of a flow path selection valve according to an embodiment of the present application;
FIG. 2 is a structural schematic view of a fluid path system according to an embodiment of the present application;
FIG. 3 is a three-dimensional perspective view of the fitting of a stator and a rotor according to an embodiment of the present application;
FIG. 4 is a schematic view of the rotor at a first valve position according to an embodiment of the present application;
FIG. 5 is a schematic view of the rotor at a second valve position according to an embodiment of the present application;
FIG. 6 is a three-dimensional perspective view of the stator according to an embodiment of the present application;
FIG. 7 is a three-dimensional perspective view of the rotor according to an embodiment of the present application;
FIG. 8 is a structural schematic view of a part of the interior of the flow path selection valve according to an embodiment of the present application;
FIG. 9 is a schematic plan view of the flow path selection valve according to an embodiment of the present application;
FIG. 10 is a cross-sectional schematic view of the flow path selection valve taken along direction A-A of FIG. 9;
FIG. 11 is another exploded schematic view of the flow path selection valve according to an embodiment of the present application;
FIG. 12 is another cross-sectional schematic view of the flow path selection valve according to an embodiment of the present application;
FIG. 13 is a bottom view of the fitting of the stator and a valve head according to an embodiment of the present application;
FIG. 14 is a side view of the fitting of the stator and the valve head according to an embodiment of the present application;
FIG. 15 is a three-dimensional perspective view of a stator according to another embodiment of the present application;
FIG. 16 is a three-dimensional perspective view of a rotor according to another embodiment of the present application;
FIG. 17 is another structural schematic view of the fluid path system according to an embodiment of the present application;
FIG. 18 is yet another structural schematic view of the fluid path system according to an embodiment of the present application;
FIG. 19 is yet another structural schematic view of the fluid path system according to an embodiment of the present application;
FIG. 20 is a schematic flow chart of a sequencing method according to an embodiment of the present application;
FIG. 21 is a block diagram of a nucleic acid sequence determination system according to an embodiment of the present application;
FIG. 22 is another block diagram of the nucleic acid sequence determination system according to an embodiment of the present application;
FIG. 23 is another schematic flow chart of the sequencing method according to an embodiment of the present application;
FIG. 24 is yet another schematic flow chart of the sequencing method according to an embodiment of the present application;
FIG. 25 is an exploded schematic view of a flow path selection valve according to an embodiment of the present application;
FIG. 26 is a structural schematic view of a fluid path system according to an embodiment of the present application;
FIG. 27 is a three-dimensional perspective view of the flow path selection valve according to an embodiment of the present application;
FIG. 28 is a three-dimensional perspective view of a manifold according to an embodiment of the present application;
FIG. 29 is a side view of the manifold according to an embodiment of the present application;
FIG. 30 is a cross-sectional schematic view of the manifold taken along direction A-A of FIG. 29;
FIG. 31 is a schematic plan view of a first valve element according to an embodiment of the present application;
FIG. 32 is a schematic plan view of a second valve element according to an embodiment of the present application;
FIG. 33 is a schematic view of the fitting of the first valve element and the second valve element while being at a first position according to an embodiment of the present application;
FIG. 34 is a schematic view of the fitting of the first valve element and the second valve element while being at a second position according to an embodiment of the present application;
FIG. 35 is a three-dimensional perspective view of a slide block according to an embodiment of the present application;
FIG. 36 is a structural schematic view of a housing according to an embodiment of the present application;
FIG. 37 is a structural schematic view of a flow cell assembly according to an embodiment of the present application;
FIG. 38 is a structural schematic view of a fluid path system according to an embodiment of the present application;
FIG. 39 is a structural schematic view of a system according to an embodiment of the present application;
FIG. 40 is a schematic flow chart of a method according to an embodiment of the present application;
FIG. 41 is a block diagram of the fluid path system according to an embodiment of the present application;

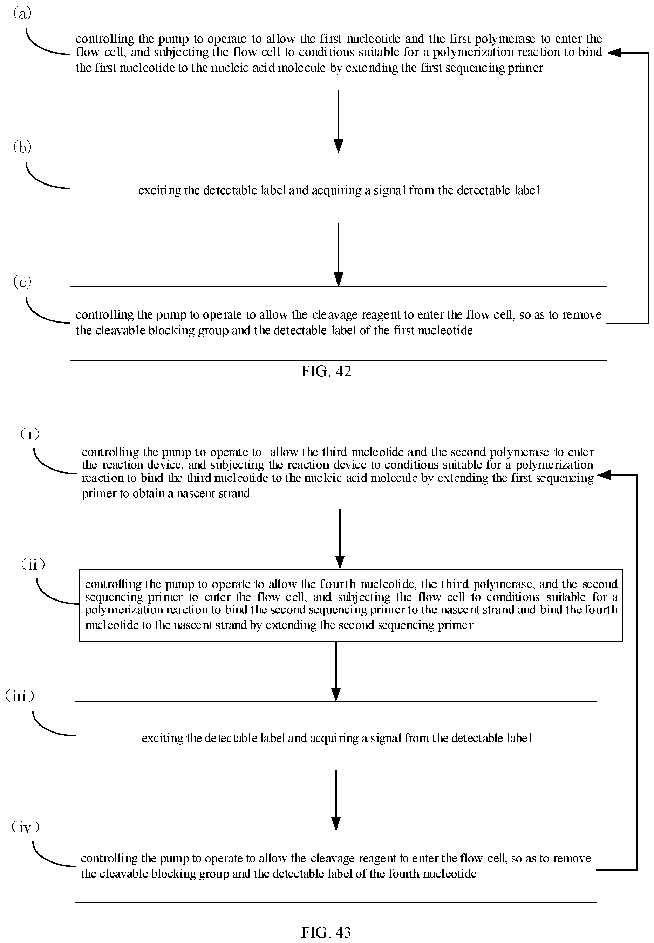

FIG. 42 is a schematic flow chart of a method according to an embodiment of the present application;
and
FIG. 43 is a schematic flow chart of a method according to an embodiment of the present application.

Description of reference numerals for main elements:
Flow path selection valve 10, fluid path system 12, pump assembly 14, common port 16, first port 18, second port 20, communication groove 21, first communication groove 22, stator 24, rotor 26, second communication groove 28, stator end face 30, rotor end face 32, valve body 34, valve head 36, first interface 38, second interface 40, third interface 42, recess 44, flange 46, top surface 48, side surface 50, positioning structure 52, end cap 53, positioning pin 54, first fastener 55, drive component 56, second fastener 58, fluid network 60, reaction device 62, reservoir 64, first reservoir 66, second reservoir 68, first reaction device 70, second reaction device 72, first reaction region 78, second reaction region 80, three-way valve 82, multi-way valve 84, liquid inlet 86, liquid outlet 88, liquid trap 89, nucleic acid sequence determination system 90, detection assembly 92, controller 94, first control module 96, second control module 98, third control module 100, fourth control module 102, third reservoir 104, and fourth reservoir 106.

Flow path selection valve 120, fluid path system 122, pump 142, common port 162, first port 182, second port 220, communication groove 212, first communication groove 222, manifold 242, first valve element 262, second communication groove 232, second valve element 282, first channel 320, second channel 322, third channel 342, cavity 362, first connection port 382, second connection port 420, third connection port 422, fastener 552, drive component 562, slide block 572, housing 582, guide groove 592, connection part 620, guide rail part 612, flow cell assembly 12012, a plurality of flow paths 12022, flow cell 622, control circuit 632, reservoir 642, first reservoir 662, second reservoir 682, first flow cell 720, second flow cell 722, first reaction region 782, second reaction region 820, reagent selection valve 842, liquid inlet 862, liquid outlet 882, liquid trap 892, and sequencing system 920.

### DETAILED DESCRIPTION

Embodiments of the present application will be further described with reference to the drawings. The same or similar reference numerals in the drawings represent the same or similar elements or elements having the same or similar functions all the way. In addition, the embodiments of the present application described below in conjunction with the drawings are exemplary and are only used for explaining the embodiments of the present application, and are not to be construed as limiting the present application.

In the present application, the terms "first" and "second" are used for description purpose only rather than being construed as indicating or implying relative importance or sequence or implicitly indicating the number of indicated technical features. Therefore, features defined with "first" and "second" may explicitly or implicitly include one or more features. In the description of the present application, unless otherwise defined, "a plurality of' means two or more than two.

In the description of the present application, unless otherwise clearly specified and defined, "install" and "connect" should be comprehended in their broad sense. For example, "connect" may be "fixedly connect", "detachably connect" or "integrally connect"; "mechanically connect", "electrically connect" or "communicate with each other"; or "directly interconnect", "indirectly interconnect via an intermediate", "the communication between the interiors of two elements" or "the interaction between two elements". For those of ordinary skill in the art, the specific meanings of the aforementioned terms in the present application can be understood according to specific conditions.

In this application, unless otherwise clearly specified and defined, a first feature being "above" or "under" a second feature may refer to that the first feature and the second feature are in direct contact, or the first feature and the second feature are in indirect contact via an intermediate. Also, a first feature being "on", "over" and "above" a second feature may refer to that the first feature is right above or obliquely above the second feature, or simply mean that the first feature is at a higher horizontal position than the second feature. A first feature being "under", "beneath" and "below" a second feature may refer to that the first feature is right below or obliquely below the second feature, or simply mean that the first feature is at a lower horizontal position than the second feature.

In the present application, the "nucleic acid sequence determination", "sequence determination" and "sequencing" are equivalent and include DNA sequencing and/or RNA sequencing, including long fragment sequencing and/or short fragment sequencing. The "sequencing determination reaction" is the same as the sequencing reaction. Generally, in nucleic acid sequence determination, a base/nucleotide on a template nucleic acid can be identified/determined by one cycle of sequencing, and the base is selected from at least one of A, T, C, G and U. In sequencing by SBS and/or SBL, the one cycle of sequencing includes extension (base extension), signal detection (e.g., photographing/image acquisition) and group cleavage (cleavage of detectable and/or blocking groups). The "nucleotide analog", i.e., a substrate, also called a reversible terminator, is an analog of nucleotides A, T, C, G and/or U, and is capable of pairing with a specific type of base following the complementary base pairing rule and blocking or inhibiting the binding of the nucleotide/substrate to the next nucleotide position of the template strand. One cycle of sequencing includes one or several repeated reactions consisting of extension reaction (base extension), signal detection and group cleavage. For example, when four nucleotide analogs carry the same detectable label, e.g., carry the same fluorescent molecule, one cycle of sequencing includes four repeated reactions sequentially performed and corresponding to the four nucleotides. For another example, when four nucleotide analogs carry two detectable labels that can be detected and differentiated from each other, and every two nucleotide analogs carry the same detectable label, one cycle of sequencing includes two repeated reactions, and namely, two nucleotides carrying different detectable labels are subjected to extension reaction, signal detection and group cleavage in the same reaction system, and the two repeated reactions compose one cycle of sequencing and complete the base type identification at one position of the template. For yet another example, when four nucleotide analogs carry four detectable labels that are signal-detectable and can be differentiated from each other, respectively, or three of the four nucleotide analogs carry three detectable labels that are signal-detectable and can be differentiated from each other, respectively, and the other nucleotide analog does not carry a detectable label, one cycle of sequencing includes one repeated reaction of the four nucleotide analogs in the same reaction system.

### Embodiment 10

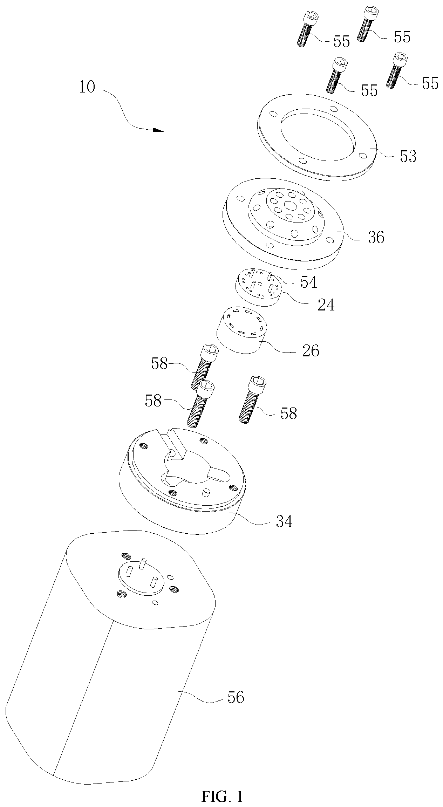

Referring to FIGs. 1-2, an embodiment of the present application provides a flow path selection valve 10, where the flow path selection valve 10 is provided with a plurality of flow channels, and the flow path selection valve 10 may be applied to a fluid path system 12 or the fluid path system 12 includes the flow path selection valve 10, thus achieving flow confluence, flow splitting, flow path switching and/or flow rate control.

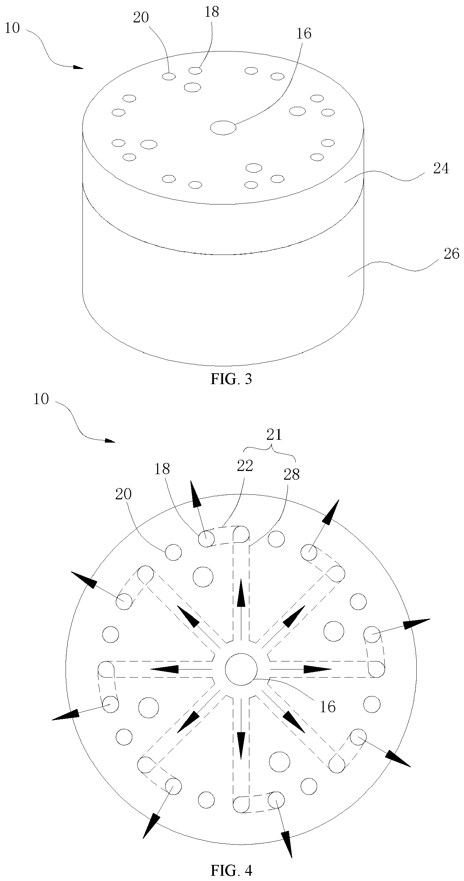

Referring to FIGs. 3-5, the flow path selection valve 10 according to an embodiment of the present application is configured to rotate between a first valve position and a second valve position, and the flow path selection valve 10 has a common port 16, a plurality of first ports 18, a plurality of second ports 20, and a plurality of communication grooves 21. The communication groove 21 may selectively allow the common port 16 to be in communication with a first port 18, or allow a first port 18 to be in communication with a second port 20.

In the case that the flow path selection valve 10 is at the first valve position, the first port 18 is in communication with the second port 20 through the communication groove 21 to form a first flow channel. In the case that the flow path selection valve 10 is at the second valve position, the first port 18 is in communication with the common port 16 through the communication groove 21 to form a second flow channel.

In the flow path selection valve 10 and the fluid path system 12 according to an embodiment of the present application, each first port 18 is in communication with the corresponding second port 20 through the corresponding communication groove 21 in the case that the flow path selection valve 10 is at the first valve position, and each first port 18 is in communication with the common port 16 through the corresponding communication groove 21 in the case that the flow path selection valve 10 is at the second valve position, so that the flow path selection valve 10 has two or more channels. The flow path selection valve 10 is suitable for any fluid path system requiring the use of a three-way valve, in particular a plurality of three-way valves, for example, a fluid path system involving the change of the direction of one or more flow paths and/or the dispensing of a plurality of liquids/solutions. The application of the flow path selection valve to a fluid path system including a plurality of fluid paths/flow paths enables independent or parallel three-way control on the plurality of fluid paths, and it may be avoided that a plurality of three-way valves are simultaneously provided in the fluid path system 12, so that the fluid path system 12 has decreased cost, reduced size, less reagent consumption and improved reliability, and is convenient to maintain, repair and control.

Specifically, the number of the common ports 16 is one, and the common port 16 may serve as a liquid inlet or outlet of the flow path selection valve 10. In other words, the liquid may enter or flow out from the flow path selection valve 10 from the common port 16, thereby achieving flow splitting or flow confluence. Here, the common port 16 serves as the liquid inlet of the flow path selection valve 10, and the liquid may enter the flow path selection valve 10 from the common port 16. The shape of the common port 16 may be a regular shape such as circular or polygonal, or may be an irregular shape. In an embodiment of the present application, the common port 16 is circular in shape to facilitate the forming and manufacturing of the common port 16 and/or connecting of it to a common tube.

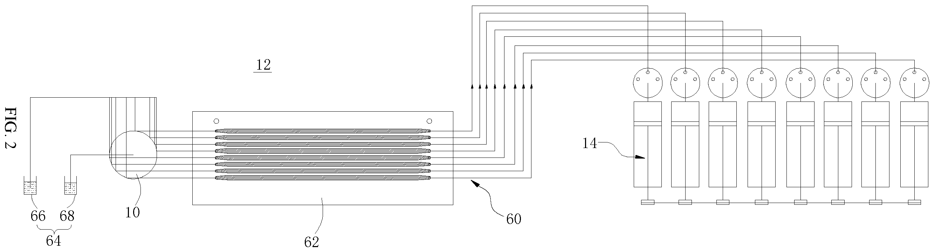

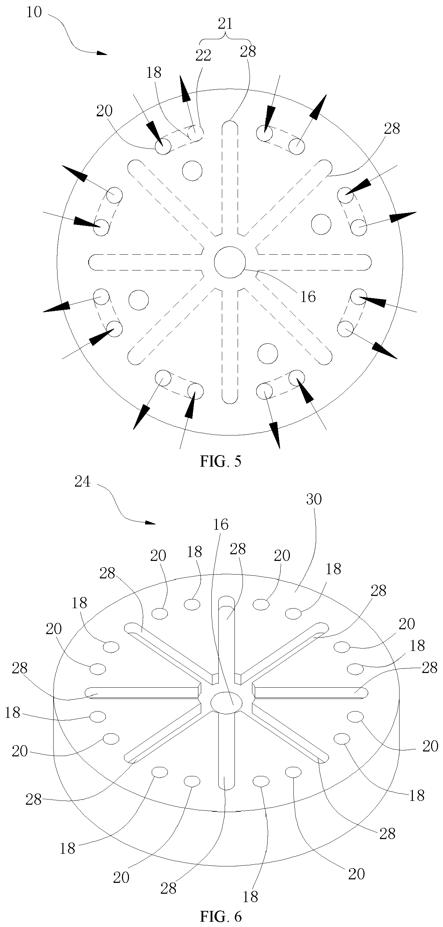

The plurality of second ports 20 correspond to the plurality of first ports 18 in a one-to-one manner. The plurality of communication grooves 21 correspond to the plurality of first ports 18 in a one-to-one manner. The number of the first ports 18 may be 2, 3, 4, 5, 6, 7, 8, or more. In the embodiment shown in FIG. 6, the number of the first ports 18 is 8 to fit an element or device containing 8 outlets or inlets upstream or downstream of the flow path selection valve 10. As shown in FIG. 2, the liquid enters the flow path selection valve 10 from the common port 16, flows out from 8 first ports 18, and then enters a reaction device 62 containing 8 channels or 8 reaction regions, thus enabling the parallel control of the feeding liquid of the 8 channels or 8 reaction regions of the reaction device 62. The shape of the first port 18 may be a regular shape such as circular or polygonal, or may be an irregular shape. In an embodiment of the present application, the first port 18 is circular. The first port 18 may serve as a liquid outlet or an inlet of the flow path selection valve 10.

Similarly, the number of the second ports 20 is not limited in this embodiment, and the number of the second ports 20 may be 2, 3, 4, 5, 6, 7, 8, or more. In the embodiment shown in FIG. 6, the number of the second ports 20 is 8 to fit an element or device including 8 outlets or inlets upstream or downstream of the flow path selection valve 10, such as the reaction device 62 including 8 channels or 8 reaction regions downstream of the flow path selection valve 10 in FIG. 2, thus enabling the parallel control of the feeding liquid of the 8 channels or 8 reaction regions of the reaction device 62. The number of second ports 20 is equal to the number of first ports 18. The shape of the second port 20 may be a regular shape such as circular or polygonal, or may be an irregular shape. In an embodiment of the present application, the second port 20 is circular. The second port 20 may serve as a liquid inlet or an outlet of the flow path selection valve 10. For ease of manufacture, the second port 20 may have a same size as the first port 18.

The number of the communication grooves 21 is the same as the number of the first ports 18 and/or the second ports 20. In an embodiment of the present application, the number of the communication grooves 21 is also 8. The communication groove 21 is not limited in shape as long as it allows the first port 18 to be in communication with the second port 20 when the flow path selection valve 10 is at the first valve position, and it may be, for example, arc-shaped or rectangular.

As shown in FIG. 4, when the flow path selection valve is at the second valve position, the common port 16 is in communication with each of the first ports 18 through a corresponding communication groove 21, and at this time, the liquid can enter the flow path selection valve 10 from the common port 16, pass through the communication grooves 21, and then flow out from the first ports 18. In other words, when the flow path selection valve is at the second valve position, the liquid enters the flow path selection valve 10 from the common port 16 and flows out from a plurality of first ports 18. That is, the flow path selection valve 10 has a function of one-channel liquid feeding and multi-channel liquid discharging.

As shown in FIG. 5, when the flow path selection valve is at the first valve position, each of the first ports 18 is in communication with a corresponding second port 20, and at this time, the liquid enters the flow path selection valve 10 from the first port 18 and flows out from the corresponding second port 20. That is, the flow path selection valve 10 also has a function of simultaneous multi-channel liquid feeding and simultaneous multi-channel liquid discharging.

The angle between the first valve position and the second valve position is not specifically limited in the embodiments of the present application. Generally, the angle between the first valve position and the second valve position is related to the positions and number of the first port 18, the second port 20, and/or the communication groove 21. In one example, the number of the first ports 18, the number of the second ports 20, and the number of the communication grooves 21 are all 8, and when the flow path selection valve 10 is at the first valve position, the flow path selection valve 10 may be rotated to the second valve position by 15 degrees clockwise or counterclockwise.

In an embodiment of the present application, in the case that the flow path selection valve 10 is at the first valve position, the first ports 18 and the second ports 20 are separated from the common port 16; in the case that the flow path selection valve 10 is at the second valve position, the common port 16 and the first ports 18 are separated from the second ports 20 to form independent first flow channels or second flow channels. The number of the first flow channels and the number of the second flow channels are both more than one, which is beneficial to the parallel control of liquid feeding and liquid discharging of a plurality of reaction regions and also the independent control of liquid feeding and liquid discharging of different reaction processes of the reaction regions.

In some embodiments, the flow path selection valve 10 may be at a third valve position, and in this case, the common port 16, the plurality of first ports 18, and the plurality of second ports 20 are separated from one another. As such, in the case that the flow path selection valve 10 is at the third valve position, the flow path selection valve 10 may also have the function of controlling the liquid shutoff.

In this embodiment, the flow path selection valve 10 has three modes, that is, a mode in which the first port 18 is in communication with the second port 20 when the flow path selection valve 10 is at the first valve position; a mode in which the common port 16 is in communication with the first port 18 when the flow path selection valve 10 is at the second valve position; and a mode in which the common port 16, the first ports 18, and the second ports 20 are separated from one another when the flow path selection valve 10 is at the third valve position.

Unless otherwise stated, reference herein to a plurality of ports being "separated" means that the plurality of ports cannot be in communication with one another, or that the liquid cannot enter from designated port or ports and flow out from other designated port or ports.

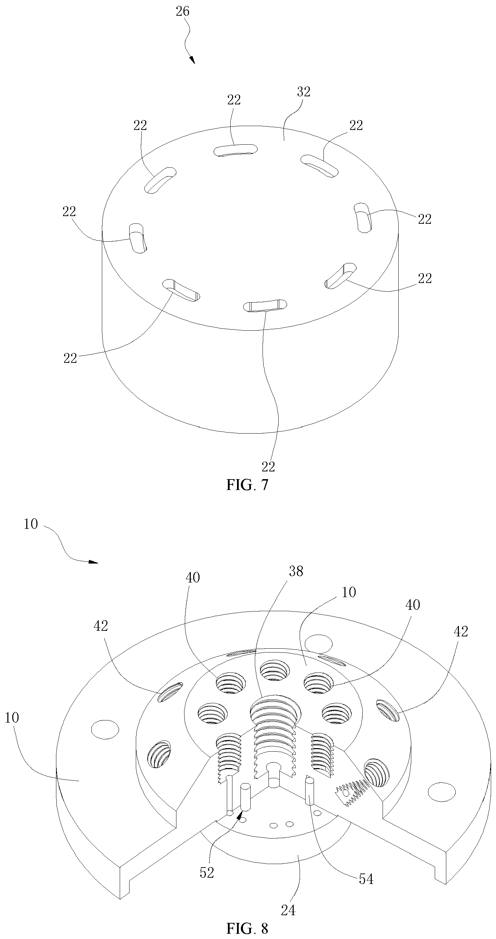

Referring to FIG. 6 and FIG. 7, in certain embodiments, the flow path selection valve 10 includes a stator 24 and a rotor 26 provided opposite to the stator 24, and the stator 24 has the common port 16, the plurality of first ports 18, and the plurality of second ports 20. The rotor 26 is provided with at least a part of the plurality of communication grooves 21. As such, during the rotation of the rotor 26, the communication groove 21 may allow the common port 16 to be in communication with the first port 18, or may allow the first port 18 to be in communication with the second port 20, so that liquid may enter the flow path selection valve 10 from the stator 24 and flow out from the flow path selection valve 10 through the stator 24.

Specifically, the rotor 26 and the stator 24 are coaxially arranged, or a central axis of the rotor 26 and a central axis of the stator 24 coincide. It will be appreciated that the rotor 26 may rotate relative to the stator 24. Further, the rotor 26 rotates relative to the stator 24 between the first valve position and the second valve position.

Unless otherwise stated, rotation of the flow path selection valve 10 between the first valve position and the second valve position is generally accomplished by rotating the rotor 26 of the flow path selection valve 10, not by rotating the flow path selection valve 10 as a whole.

In addition, the rotor 26 being provided with at least a part of the plurality of communication grooves 21 means that some of or all of the plurality of communication grooves 21 may be provided on the rotor 26 and a part of the structure or the entire structure of the communication grooves 21 may be provided on the rotor 26.

In an embodiment of the present application, the communication groove 21 may have a bent shape, a curved shape, or the like, and the specific shape of the communication groove 21 is not limited herein. In certain embodiments, the communication groove 21 includes a first communication groove 22 and a second communication groove 28 capable of being in communication with each other, and the first communication groove 22 and the second communication groove 28 are provided on the rotor 26 and the stator 24, respectively. As such, the communication groove 21 is more easily formed.

In an embodiment of the present application, a plurality of first communication grooves 22 are arranged at intervals along a circumferential direction of the rotor 26. A plurality of second communication grooves 28 are radially arranged centering on the common port 16.

In other embodiments, the first communication groove 22 may be omitted, and in this case, the communication groove 21 includes the second communication groove 28. Since the second communication groove 28 is provided on the stator 24, the entire structure of the communication groove 21 is provided on the stator 24, or the stator 24 is provided with the communication groove 21. In this case, the first port 18 and the second port 20 may both be provided on the rotor 26, and the common port 16 is provided on the stator 24. The communication groove 21 may have a first portion and a second portion, and the first portion may be provided to be separated from the second portion. One end of the first portion may be in communication with the common port 16, and the other end is in selective communication with the first port 18 during rotation of the rotor 26. The second portion is in selective communication with the first port 18 and the second port 20 during rotation of the rotor 26, thereby allowing the first port 18 to be in communication with the second port 20.

Of course, similarly, the second communication groove 28 may be omitted, and in this case, the communication groove 21 includes the first communication groove 22. Since the first communication groove 22 is provided on the rotor 26, the entire structure of the communication groove 21 is provided on the rotor 26, or in other words, the rotor 26 is provided with the communication groove 21. In this case, the first port 18, the second port 20, and the common port 16 may be all provided on the stator 24. The communication groove 21 may have a first portion and a second portion, and the first portion may be provided to be separated from the second portion. One end of the first portion may be in communication with the common port 16, and the other end is in selective communication with the first port 18 during rotation of the rotor 26. The second portion is in selective communication with the first port 18 and the second port 20 during rotation of the rotor 26, thereby allowing the first port 18 to be in communication with the second port 20.

In certain embodiments, one communication groove 21 is composed of one first communication groove 22 and one second communication groove 28. One first communication groove 22 has two ends, one end of each second communication grooves 28 is in communication with one first port 18, and the other end is in selective communication with one second port 20; one second communication groove 28 has two ends, one end of each second communication groove 28 is in communication with the common port 16, and the other end is in selective communication with one first port 16. As such, the design of the second communication groove 28 allows the common port 16 to be easily in communication with the first port 18 by rotating the flow path selection valve 10, and further, the communication groove 21 including the first communication groove 22 and the second communication groove 28 enables easy control of the flow path selection valve 10, so that the rotation of the flow path selection valve 10 can achieve the designated functions described above and the manufacturing is also facilitated.

Specifically, the number of the flow channels 28 is the same as the number of the first ports 18, and in an embodiment of the present application, the number of the second communication grooves 28 is 8. Of course, in other embodiments, the number of the flow channels 28 may be 2, 3, 4, 5, 6, etc. Referring to FIG. 6 and FIG. 7, in certain embodiments, the stator 24 includes a stator end face 30, the rotor 26 includes a rotor end face 32, and the stator end face 30 and the rotor end face 32 are abutted together. The second communication groove 28 is formed in the stator end face 30. The first communication groove 22 is formed in the rotor end face 32. As such, the liquid leakage can be prevented as the rotor end face 32 and the stator end face 30 is attached. The second communication groove 28 is formed in the stator end face 30, and the first communication groove 22 is formed in the rotor end face 32, so that it is easier to manufacture and form the second communication groove 28 and the first communication groove 22, and the manufacturing cost of the stator 24 and the rotor 26 can be reduced.

In certain embodiments, the plurality of first ports 18 and the plurality of second ports 20 are all provided around the common port 16. As such, the liquid entering the common port 16 may flow around to flow out from the first ports 18, facilitating a more uniform flow of liquid. In addition, the surrounding of the common port 16 by the second ports 20 may prevent the second ports 20 from interfering with the common port 16.

In certain embodiments, the plurality of first ports 18 are arranged at intervals along a circumferential direction of the stator 24 or rotor 26. As such, the arrangement of the first ports 18 can allow reasonable utilization of the space of the stator 24 and avoid the concentration of the first ports 18, so that the size of the stator 24 can be reduced. Further, the plurality of first ports 18 are arranged at uniform intervals along a circumferential direction of the stator 24, or the distance between two adjacent first ports 18 is equal along a circumferential direction of the stator 24.

In certain embodiments, the plurality of second ports 20 are arranged at intervals along a circumferential direction of the stator 24 or rotor 26. As such, the arrangement of the second ports 20 can allow reasonable utilization of the space of the stator 24 and avoid the concentration of the second ports 20, so that the size of the stator 24 can be reduced. Further, the plurality of second ports 20 are arranged at uniform intervals along a circumferential direction of the stator 24, or the distance between two adjacent second ports 20 is equal along a circumferential direction of the stator 24.

As shown in FIG. 6, in certain embodiments, the plurality of first ports 18 and the plurality of second ports 20 are located on the same circumference. In other words, the plurality of first ports 18 and the plurality of second ports 20 are arranged in a circular pattern. As such, it is easier to provide the first ports 18 and the second ports 20. Further, the first ports 18 and the second ports 20 are alternately arranged, which makes it easier for the flow path selection valve 10 to switch from the first valve position to the second valve position during rotation of the rotor 26.

In certain embodiments, the flow path selection valve 10 has a central axis, and the plurality of first ports 18 are arranged at intervals on a circular plane having the central axis of the flow path selection valve 10 as an axis. As such, it is easier to provide the first ports 18 and the second ports 20.

Further, the plurality of second ports 20 are arranged at intervals on a circular plane having the central axis of the flow path selection valve 10 as an axis.

Referring to FIG. 1, in certain embodiments, the flow path selection valve 10 includes a valve body 34, and the stator 24 and the rotor 26 are both received in the valve body 34. As such, the valve body 34 acts as a carrier for the flow path selection valve 10, and the valve body 34 may facilitate installation of the stator 24, the rotor 26, and other components of the flow path selection valve 10. Specifically, the valve body 34 may be made of metal or the like, so that the strength of the valve body 34 may be increased, and thereby the valve body 34 may protect the rotor 26 and the stator 24, thus increasing the service life of the flow path selection valve 10. The appearance of the valve body 34 is in a cylindrical shape, a rectangular shape, or the like, the appearance of the valve body 34 may be designed to have different shapes according to actual requirements, and the appearance of the valve body 34 is not limited herein.

Referring to FIG. 1 and FIG. 8, in certain embodiments, the flow path selection valve 10 includes a valve head 36. The valve head 36 covers the stator 24 and is provided on the valve body 34, the valve head 36 has a first interface 38, a second interface 40, and a third interface 42, the first interface 38 is in communication with the common port 16, the second interface 40 is in communication with the first port 18, and the third interface 42 is in communication with the second port 20. As such, the interfaces of the valve head 36 may be connected to external tubes, so that the flow path selection valve 10 may control the dispensing of liquid.

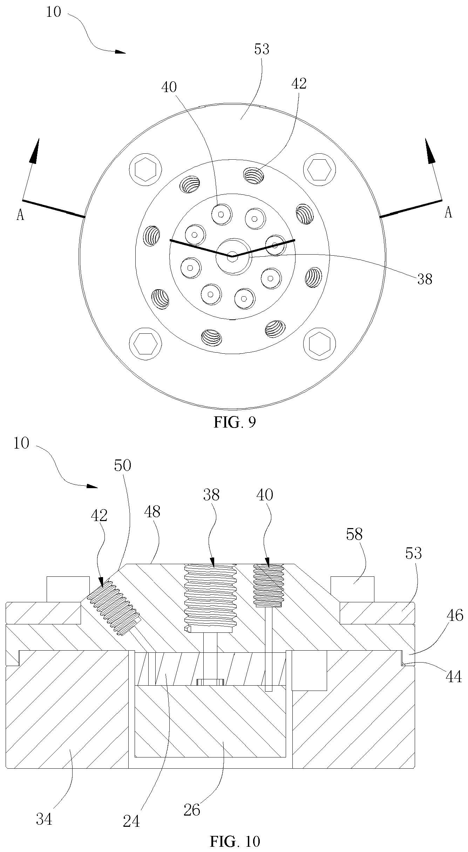

Specifically, referring to FIG. 9 and FIG. 10, the edge of the valve body 34 is provide with recesses 44, and the edge of the valve head 36 is provided with flanges 46, and the flange 46 is caught in the groove 44 to allow the valve head 36 to form a more compact fit with the valve body 34.

The first interface 38 may be a threaded interface, or an external tube or other components may be in thread connection with the flow path selection valve 10. The threaded interface may not only prevent liquid from leaking out of the first interface 38, but also allow the external tubes to be quickly removed from and installed with the flow path selection valve 10. Similarly, the second interface 40 and the third interface 42 may also be threaded interfaces.

The valve head 36 is a cap-like part, and the valve head 36 includes a top surface 48 and a side surface 50 connected to the top surface 48, and the side surface 50 is provided obliquely with respect to the top surface 48. The first interface 38 and the second interface 40 are both provided on the top surface 48, and the third interface 42 is provided on the side surface 50. Therefore, the interfaces are dispersedly positioned, and mutual interference among the interfaces is avoided.

Referring to FIG. 8, in certain embodiments, the flow path selection valve 10 includes a positioning structure 52, and the positioning structure 52 is configured to position the valve head 36 and the stator 24. As such, the positioning structure 52 enables more accurate positioning of the valve head 36 and the stator 24, such that the interfaces on the valve head 36 may be in communication with corresponding ports, thus facilitating fluid flow.

Specifically, in one example, the positioning structure 52 includes a positioning pin 54, and the positioning pin 54 is inserted in the valve head 36 and the stator 24. In an embodiment of the present application, the number of the positioning pins 54 is more than one, and the plurality of positioning pins 54 can limit the degree of freedom of the valve head 36 and the stator 24, so that the valve head 36 is positioned on the stator 24.

As shown in FIG. 1 and FIG. 10, in certain embodiments, the flow path selection valve 10 further includes an end cap 53 and a first fastener 55, and the valve head 36 is secured to the valve body 34 through the end cap 53 and the first fastener 55. The end cap 53 may be in the form of a ring-shaped sheet, and the first fastener 55 may be a screw.

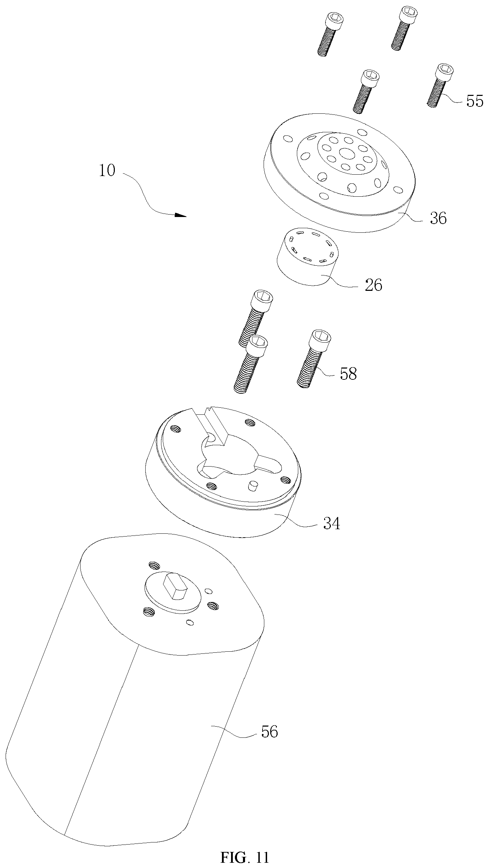

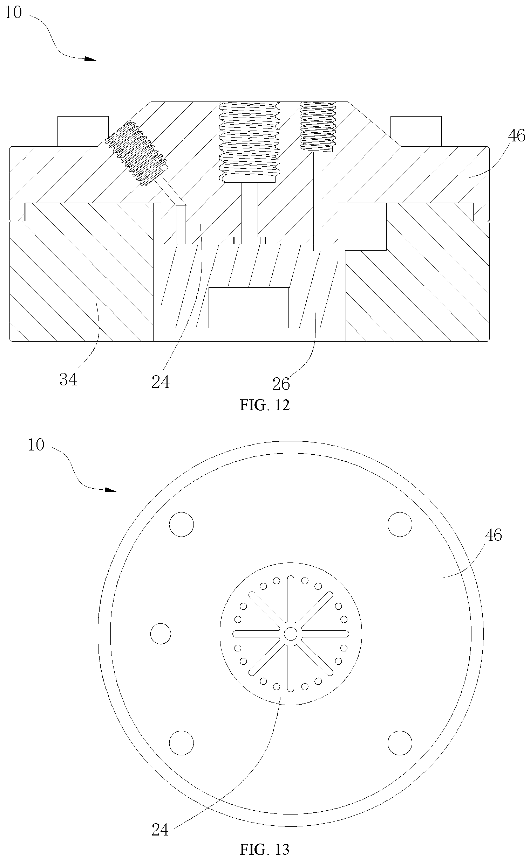

In one example, during the assembly of the flow path selection valve 10, first the stator 24 and the rotor 26 are installed in the valve body 34, then the valve head 36 is installed on the valve body 34 and the positioning of the valve head 36 and the stator 24 is performed, then the end cap 53 is placed on the valve head 36, and finally the first fastener 55 passes through the end cap 53 and the valve head 36 and is tightly threaded into the valve body 34, thus securing the valve head 36 to the valve body 34. Referring to FIGs. 11-14, in certain embodiments, the valve head 46 and the stator 24 may be an integrated structure. In other words, the valve head 46 and stator 24 may not be detachable. For example, the valve head 46 and the stator 24 may be formed by removing material from the same substrate. In such embodiments, the positioning structure 52 may be omitted. In other words, the valve head and the stator need not be installed together using the positioning structure 52.

It should be noted that reference is made to the same or similar parts of the flow path selection valve of the embodiments of FIGs. 1-10 for other explanations of the flow path selection valve of the embodiments of FIGs. 11-14, which will not be repeated herein.

Referring again to FIG. 1, in certain embodiments, the flow path selection valve 10 includes a drive component 56 connected to the rotor 26, the drive component 56 is provided on the valve body 34, and the drive component 56 is used to drive the rotor 26 to rotate. As such, the drive component 56 may drive the rotor 26 to rotate such that the rotor 26 may be at different locations to enable the flow path selection valve 10 to achieve different functions.

Specifically, the drive component 56 may be a motor, and a rotating portion of the motor is connected to the rotor 26. The drive component 56 may be secured to the valve body 34 through a second fastener 58, such that the relative position between the drive component 56 and the valve body 34 is fixed.

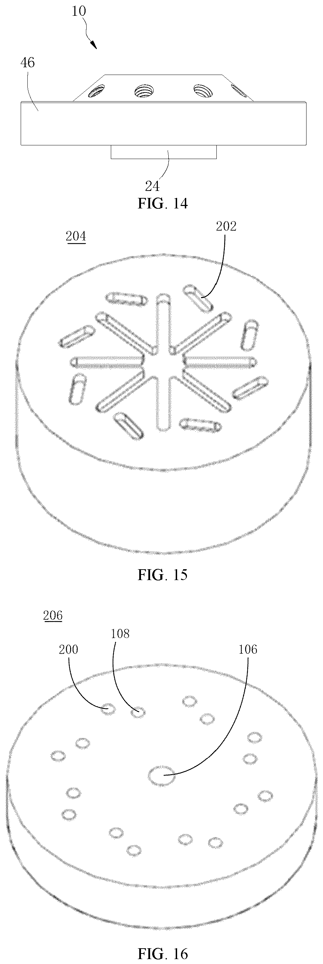

Referring to FIGs. 15-16, FIGs. 15-16 are three-dimensional perspective views of a stator 204 and a rotor 206, respectively, according to another embodiment of the present application. In this embodiment, the rotor 206 has a common port 106, a plurality of first ports 108, and a plurality of second ports 200, and the stator 204 is provided with at least a part of a plurality of communication grooves 202. In other words, the common port 106, the first ports 108, and the second ports 200 are formed on the rotor 206, and at least a part of the plurality of communication grooves 202 are formed on the stator 204. In this embodiment, reference may be made to the explanations of the stator 24 and the rotor 26 of any of the above embodiments for other explanations of the common port 106, the first port 108, and the second port 200, which will not be repeated herein. For example, the stator 204 being provided with at least a part of the plurality of communication grooves 202 means that some of or all of the plurality of communication grooves 202 may be provided on the stator 204 and a part of the structure or the entire structure of the communication grooves 202 may be provided on the stator 204.

Referring again to FIG. 2, the fluid path system 12 according to one embodiment of the present application includes the flow path selection valve 10 according to any one of the embodiments described above, a pump assembly 14, and a fluid network 60. The fluid network 60 includes a reservoir 64, a first flow channel, a second flow channel, and a reaction device 62, the first flow channel and the second flow channel are each independently in flow communication with the reservoir 64 and the reaction device 62, and the pump assembly 14 is in communication with the flow path selection valve 10. The pump assembly 14 is in communication with the fluid network 60. The flow path selection valve 10 is configured to rotate between a first valve position and a second valve position. In the case that the flow path selection valve 10 is at the first valve position, the pump assembly 14 induces liquid in the reservoir 64 to flow toward the reaction device 62 through the first flow channel, and in the case that the flow path selection valve 10 is at the second valve position, the pump assembly 14 induces liquid in the reservoir 64 to flow toward the reaction device 62 through the second flow channel.

In the fluid path system 12 according to an embodiment of the present application, the flow path selection valve 10 may be used to perform independent or parallel three-way control on the first flow channel and the first flow channel, and it may be avoided that a plurality of three-way valves are simultaneously provided in the fluid path system 12, so that the fluid path system 12 has decreased cost, reduced size, less reagent consumption and improved reliability, and is convenient to maintain, repair and control. The fluid path system 12 described above is particularly suitable for systems requiring high precision in fluid control and delivery, such as sequencing systems.

Specifically, the pump assembly 14 may provide power for the fluid path system 1212, so as to enable the liquid to flow. The pump assembly 14 may include a plurality of pumps, e.g., two. One of the pumps may be in communication with the common port 16, and the other pump is in communication with the second port 20, to achieve flow splitting or flow confluence. The pump in communication with the common port 16 may provide power for the liquid to move from the common port 16 to the first port 18, and the pump in communication with the second port 20 may provide power for the liquid to move from the second port 20 to the first port 18.

In a specific embodiment, the number of the first ports 18 is 8, and the pump assembly 14 includes 8 pumps with the number equal to the number of the first ports 18. For example, the pump assembly is a negative pressure octuple pump, which is located downstream of the fluid path system 1212, specifically downstream of the reaction device 62, and is capable of independently providing negative pressure to the fluid in the one-inlet-eight-out first flow channels and the eight-inlet-eight-out second flow channels after passing through the flow path selection valve 10, so that the power of the fluid in flow channels of the fluid network 60 can be independently controlled, which facilitates fine control of the flow rate and/or flow velocity of the fluid in the flow channels.

The reservoir 64 can store a plurality of solutions, including a reaction solution, a buffer solution, a cleaning solution, and/or purified water, and other solutions, including reagents for different reactions or different steps of a reaction. The fluid path system 12 including the pump assembly 14 may allow one or more solutions to flow toward the reaction device 62 sequentially or simultaneously.

In certain embodiments, the reservoir 64 includes a first reservoir 66 and a second reservoir 68. The first reservoir 66 carries a biological sample solution and the second reservoir 68 carries a reaction solution. In the case that the flow path selection valve 10 is at the first valve position, the flow path selection valve 10 allows the first reservoir 66 to be in communication with the reaction device 62, and the pump assembly 14 induces the biological sample solution to flow toward the reaction device 62 through the first flow channel. In the case that the flow path selection valve 10 is at the second valve position, the flow path selection valve 10 allows the second reservoir 68 to be in communication with the reaction device 62, and the pump assembly 14 induces the reaction solution to flow toward the reaction device 62 through the second flow channel.

That is, the flow path selection valve 10 may allow the reaction solution and the biological sample solution to enter the reaction device 62 independently and in a time-sharing manner to sequentially perform corresponding reactions, such as a sample loading reaction (immobilization and/or hybridization of nucleic acid) and a sequencing reaction.

Illustratively, the first reaction is, for example, an immobilization and/or hybridization reaction, that is, a nucleic acid under test is immobilized on or connected to a channel or reaction region of the reaction device 62. The biological sample solution is a solution containing the nucleic acid under test, and the biological sample solution may sequentially pass through the second port 20 and the first port 18 and enter the reaction device 62 to perform the first reaction. In one example, the reaction device 62 is a sandwich-like structure with three layers (upper, middle and lower layers), or a structure with upper and lower layers. The upper layer (near the objective lens) is a transparent glass layer, the middle layer or the lower layer is a transparent glass layer or an opaque substrate layer, and the middle layer or the lower layer is provided with a plurality of channels arranged in an array. The channels can receive liquid to provide physical space for reaction, each channel is provided with an independent liquid inlet and an independent liquid outlet, the number of the channels is equal to that of the first ports 18, and a plurality of biological sample solutions can simultaneously and independently enter one channel of the reaction device 62 through one second port 20 and one first port 18. Thus, the flow path selection valve 10 or the fluid path system 12 containing the flow path selection valve 10 can perform loading and detection assays of a plurality of biological samples without the need to incorporate other means (e.g., labeling different biological samples). Specifically, the reaction device 62 herein is, for example, a solid substrate having a functional group on the surface and/or a solid substrate having a probe attached to the surface, and the solid substrate having a functional group on the surface and/or the solid substrate having a probe attached to the surface are also generally referred to as a chip or a microsphere. For example, the channel has a functional group on or a probe (oligonucleotide) attached to the upper surface (the lower surface of the upper glass layer) and/or the lower surface (the upper surface of the middle or lower layer structure), the functional group is capable of binding to the nucleic acid under test, and/or at least a portion of the probe is capable of complementarily pairing with the nucleic acid under test, to immobilize the nucleic acid under test on or attach it to the surface of the solid substrate, and to perform a subsequent detection and analysis, such as a sequencing reaction, on the nucleic acid under test immobilized on or attached to the surface of the solid substrate.

The second reaction is, for example, a sequencing reaction, i.e., a nucleic acid sequence determination reaction, and more specifically, a sequencing-by-synthesis reaction using a reversible terminator based on chip detection, and accordingly, the reaction solution includes one or more substrate (reversible terminator), a polymerase catalyst, a cleavage reagent (group cleavage reagent), an imaging reagent, a cleaning reagent, and the like. The reagents/reaction solutions may sequentially or simultaneously enter the reaction device 62 through the common port 16 and the first port 18 to perform the second reaction. Specifically, the corresponding reagents may sequentially or simultaneously pass through the flow path selection valve 10 and flow into the reaction device 62 to perform a plurality of reaction steps in the reaction device 62 and thereby achieve the sequencing reaction; the reaction device 62 is, for example, a solid substrate having a nucleic acid under test attached to the surface, and the solid substrate having a nucleic acid under test attached to the surface is, for example, a chip or a microsphere.

The first reaction and the second reaction described above can be performed in the reaction device 62 by controlling one flow path selection valve 10 to switch the flow channels, so that a nucleic acid sequence determination system (integrated system) including the first reaction and the second reaction can have a simpler structure. Thus, the first reaction and the second reaction do not need to be performed in different systems/apparatuses/devices, and the user operation is simpler and more convenient. Besides, the cost of constructing the integrated nucleic acid sequence determination system is far lower than the sum of the cost of constructing a nucleic acid sequence determination system (a sample loading device) for independently performing the first reaction and the cost of constructing a nucleic acid sequence determination system for independently performing the second reaction.

In an embodiment of the present application, the first reaction includes a reaction for attaching a biomolecule to the reaction device 62, including, for example, an immobilization, hybridization, or sampling reaction. The biomolecule includes DNA and/or RNA and the like, including ribonucleotides, deoxyribonucleotides and analogs thereof, including A, T, C, G, and U and analogs thereof. Among them, C represents cytosine or a cytosine analog, G represents guanine or a guanine analog, A represents adenine or an adenine analog, T represents thymine or a thymine analog, and U represents uracil or a uracil analog. The reaction device 62 provides a place for biochemical reactions, and the reaction device 62 may specifically be a chip. The reaction device 62 is detachably connected to the fluid path system 12.

The second reaction includes a reaction for detecting the biomolecule attached to the reaction device 62. For example, the biomolecule is a nucleic acid, and the second reaction may be a sequence determination reaction, i.e., generally so-called sequencing, including the determination of the primary structure or sequence of DNA, RNA, or the like, including the determination of the order of nucleotides/bases of a given nucleic acid fragment. The second reaction may include one or more sub-reactions. In one example, a DNA is subjected to sequence determination. The second reaction is sequencing based on sequencing by synthesis or sequencing by ligation, specifically, for example, sequencing-by-synthesis based on chip detection using a modified nucleotide with a detectable label, such as dNTP or a dNTP analog with a detectable label. The sequencing includes a plurality of sub-reactions, including base extension, signal acquisition, and detection group cleavage, to determine the base type of a position on the nucleic acid sequence under test, and performing the plurality of sub-reactions once may be referred to as performing one repeated reaction or one cycle of reaction. A sequencing includes multiple repeated reactions or multiple cycles of reactions to determine the order of nucleotides/bases of at least a fragment of the nucleic acid molecule (template). The modified nucleotide carries a fluorescent molecule capable of being excited to fluoresce in a particular situation for detection by an optical system. When bound to a nucleic acid under test, the modified nucleotide label is capable of preventing a base/nucleotide from binding to the next position in the nucleic acid under test. For example, the modified nucleotide label is a dNTP with a chemically cleavable moiety at the 3' hydroxyl terminus or a dNTP with a molecular conformation that interferes with the binding of the next nucleotide to the nucleic acid under test, and the dNTP or dNTP analog is four deoxyribonucleotides containing bases A, T/U, C, and G, respectively.

For sequencing by synthesis (SBS) or sequencing by ligation (SBL) based on chip detection, under the action of polymerase or ligase, the base extension includes enabling nucleotides (including modified nucleotides) to bind to a nucleic acid molecule under test based on the complementary base pairing rule in the reaction device 62 on which the nucleic acid molecule under test is immobilized, and acquiring corresponding reaction signals. The modified nucleotide may be a nucleotide with a detectable label that allows the modified nucleotide to be detected under certain circumstances, e.g., a nucleotide with a fluorescent molecular label that fluoresces when excited by a laser of a particular wavelength. Generally, for SBS, the modified nucleotide also has the function of inhibiting the binding of other nucleotides to the next position of the same nucleic acid molecule. For example, the modified nucleotide carries a blocking group that can prevent the binding of other nucleotides to the next position of the template, so that each extension is a single base extension, thus enabling the acquisition of the corresponding signal from the single base extension. The blocking group is, for example, a modified azido group (-N₃) attached to the 3' position of the glycosyl of the nucleotide.

In the detection and analysis of a biomolecule, generally, first the biomolecule is attached to the reaction device 62, and then the biomolecule attached to the reaction device 62 is detected. Specifically, in any one of the specific embodiments described above, the first reaction is performed before the second reaction, that is, the sequencing reaction is performed after the nucleic acid under test is attached to the reaction device 62. As such, using the fluid path system 12, multiple types of reactions, including sampling and sample detection, can be performed in a single nucleic acid sequence determination system.

In certain specific embodiments, the first reaction is a sampling reaction, the second reaction is a sequencing reaction, and the first reaction is performed before the second reaction. The nucleic acid molecule under test is contained in a trace amount of biological sample solution, for example, a biological sample solution at the microliter level, for example, 20 microliters of a biological sample solution. Before the first reaction is performed, the fluid path system 12 is cleaned using a cleaning solution, for example, a solution that does not affect the subsequent reaction, and the fluid path system 12 is filled with the cleaning solution. Before the first reaction is started, air is injected to fill a section of the fluid path system to separate the biological sample solution which will flow into the fluid path system subsequently from the cleaning solution already in the fluid path system, thus avoiding a situation where the trace amount of biological sample is diffused and/or diluted and thereby the attachment of the nucleic acid molecule under test to the reaction device 62 and the subsequent detection of the nucleic acid molecule under test is affected. Moreover, the separation of the biological sample solution which will flow into the fluid path system subsequently from the cleaning solution already in the fluid path system facilitates the observation of the sample injection condition, the observation of whether the reaction device 62, such as a chip containing a plurality of channels, is normal or not, and whether the fluid path system 12 is normal or not, and the like.

The substrate may be any solid support useful for immobilizing nucleic acid sequences, such as nylon membranes, glass slides, plastics, silicon wafers, magnetic beads, and the like. Probes may be randomly distributed on the surface of the substrate. The probe may be a fragment of DNA and/or RNA sequence and the like, and it also is called a primer, a capture chain or an immobilization chain. The first reaction may allow the biomolecule to be fixedly attached to the probe, for example, based on the complementary base pairing rule, so that the biomolecule is attached to the reaction device 62. Acquiring signals includes acquiring signals emitted by the modified nucleotide bound to the nucleic acid molecule. For example, a laser irradiation is performed on a particular region in the reaction device 62 after the base extension using an optical imaging assembly/system, a fluorescent molecular label in the particular region is excited to emit fluorescence, and then photographing/image acquisition is performed on the region to record the biochemical reaction signals as image information. Sequence determination involves converting the image information obtained from multiple cycles of /multiple repeated reactions into sequence information, i.e., determining the base type based on the image information, commonly referred to as base-calling.

Group cleavage includes removing the detectable label and/or blocking group on the modified nucleotide bound to the nucleic acid molecule after the base extension to allow other nucleotides (including modified nucleotides) to bind to the next position of the nucleic acid molecule for the next repeated reaction or the next cycle of reaction.

After the previous cycle or previous sub-reaction or previous step is completed and before the next cycle or subsequent sub-reaction or next step is started, a cleaning reagent may also be introduced to remove residual unreacted substances or substances that will interfere with the reaction or signal acquisition in the reaction device 62 or in the fluid path system 12.

In certain embodiments, the flow path selection valve 10 is provided upstream of the reaction device 62. As such, the flow path selection valve 10 may control the entering of the solution into the reaction device 62, and allows the fluid path system 12 to control the entering and exiting of multiple solutions to achieve multiple types of reactions only by using one power assembly (e.g., a pump assembly) or only by providing power in one direction, which is beneficial to further reducing the size and improving the integration degree of the fluid path system 12, thereby being beneficial to industrialization.

In certain embodiments, the pump assembly 14 is provided downstream of the reaction device 62 to provide negative pressure. As such, the pump assembly 14 may generate negative pressure in elements such as the reaction device 62 and the flow path selection valve 10, so as to allow the solution to enter the reaction device 62. In addition, the negative pressure generated by the pump assembly 14 may remove the air in the reaction device 62, so as to prevent the air from affecting the normal reaction in the reaction device 62. The fluid path system 12 including the pump assembly 14 located downstream of the reaction device 62 may provide a uniform power direction for the entering and exiting of the liquid in multiple types of reactions, and is particularly suitable for the fluid path system 12 including pressure-sensitive elements/assemblies. For example, the reaction device 62 is a chip containing thin glasses with a multi-layer sheet structure bonded by glue, the chip may further contain independent multiple reaction regions/channels, and the change of the power/pressure direction is easy to deform the chip or cause fluid leakage or cross-channel.

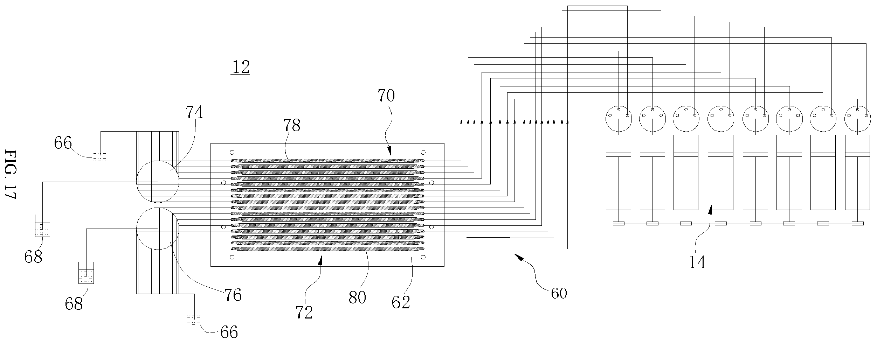

Referring to FIG. 17, in certain embodiments, the reaction device 62 includes a first reaction device 70 and a second reaction device 72. The flow path selection valve 10 includes a first flow path selection valve 74 and a second flow path selection valve 76. The first flow path selection valve 74 and the second flow path selection valve 76 are in communication with the first reaction device 70 and the second reaction device 72, respectively. As such, the first flow path selection valve 74 and the second flow path selection valve 76 may independently control the reactions in the first reaction device 70 and the second reaction device 72, which facilitates the alternation of different reactions or different steps/sub-reactions of a reaction in the first reaction device 70 and the second reaction device 72, thereby improving the reaction efficiency. In addition, the fluid path system 12 including the flow path selection valve 10, in combination with the plurality of reaction devices 62 or the reaction device 62 having a plurality of independent reaction regions, is beneficial to increasing the detection throughput and/or enabling the detection of a plurality of samples at a time.

Specifically, the first reaction device 70 and the second reaction device 72 may be of separate structures or may be of an integrated structure. In the example shown in FIG. 17, the first reaction device 70 and the second reaction device 72 are of an integrated structure. The first reaction device 70 and/or the second reaction device 72 may include one or more reaction regions. Each reaction region is capable of enabling reactions, and the reaction regions can be intersecting, sequential, or separate regions.

In the example of FIG. 17, the first reaction device 70 includes eight first reaction regions 78. The eight first reaction regions 78 correspond to the eight first ports 18 of the first flow path selection valve 74 in a one-to-one manner. The eight first reaction regions 78 are separately disposed. Similarly, the second reaction device 72 includes eight second reaction regions 80. The eight second reaction regions 80 correspond to the eight second ports of the second flow path selection valve 76 in a one-to-one manner. The eight second reaction regions 80 are separately disposed.

It should be noted that the first flow path selection valve 74 and the second flow path selection valve 76 may operate simultaneously, individually, or alternately, so that the reactions or steps in the first reaction device 70 and the second reaction device 72 may be performed alternately and in a time-sharing manner or simultaneously, which is beneficial to improving the reaction efficiency and reducing the consumption of the detection reagent and/or time.

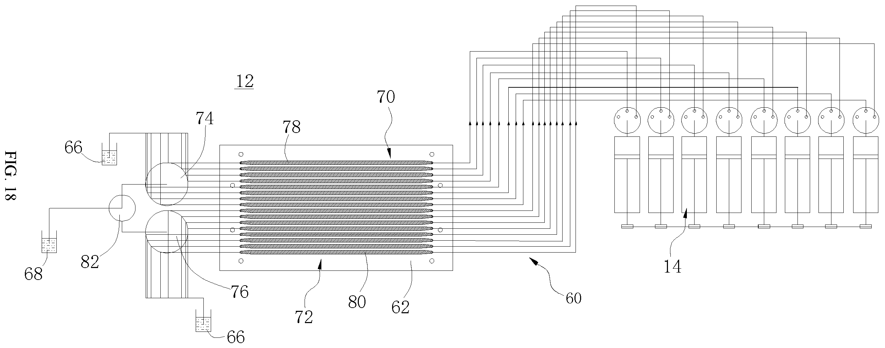

Referring to FIG. 18, in certain embodiments, the fluid path system 12 further includes a three-way valve 82 provided between the reservoir 64 and the flow path selection valve 10. The three-way valve 82 is configured to switch between a first position and a second position, the three-way valve 82 allows the reservoir 64 to be in communication with the first flow path selection valve 74 in the case that the three-way valve 82 is at the first position, and the three-way valve 82in communication with the second flow path selection valve 76 in the case that the three-way valve 82 is at the second position. As such, the three-way valve 82 enables time-sharing control of the liquid feeding of the first flow path selection valve 74 and the second flow path selection valve 76, so that alternate control of different types of reactions or different steps of the same type of reaction of the first reaction device 70 and the second reaction device 72 can be achieved, which is beneficial to improving reaction efficiency and saving time.

For example, one of the first reaction device 70 and the second reaction device 72 performs a first reaction, and the other performs a second reaction. For another example, one of the first reaction device 70 and the second reaction device 72 performs one step of the second reaction, and the other performs another step of the second reaction.

Further, in certain embodiments, the three-way valve 82 allows the reservoir 64 to be in communication with a common port of the first flow path selection valve 74 in the case that the three-way valve 82 is at the first position, and the three-way valve 82 allows the reservoir 64 to be in communication a common port of the second flow path selection valve 76 in the case that the three-way valve 82 is at the second position. As such, the liquid passing through the three-way valve 82 can enter the reaction device 62 through the second flow channel formed by the first flow path selection valve 74 or the second flow channel formed by the second flow path selection valve 76 to perform the corresponding reaction. For example, the liquid passing through the three-way valve 82 enters the first reaction device 70 through the second flow channel of the first flow path selection valve 74 to perform the second reaction (sequencing reaction).

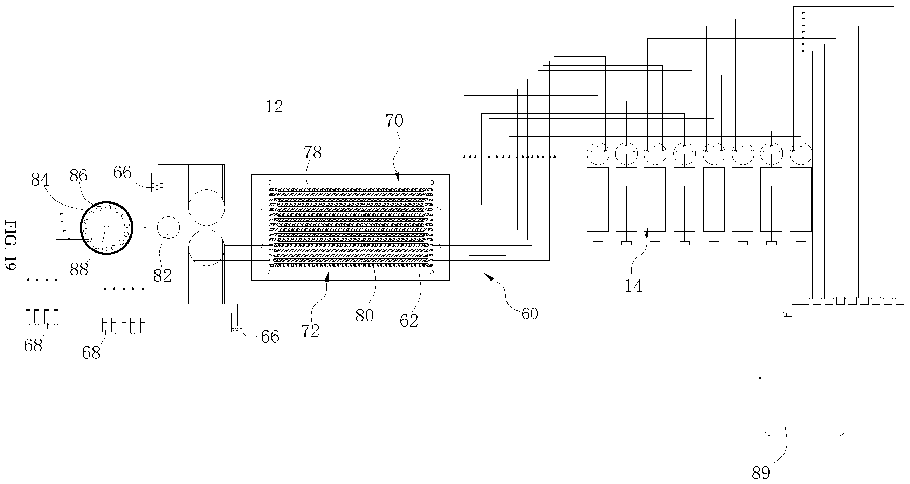

Referring to FIG. 19, in certain embodiments, the fluid path system 12 includes a multi-way valve 84. The multi-way valve 84 is provided with a plurality of liquid inlets 86 and one liquid outlet 88. The liquid outlet 88 is selectively in communication with one of the liquid inlets 86, and the liquid inlets 86 are in communication with the three-way valve 82. As such, the plurality of liquid inlets 86 of the multi-way valve 84 allow different liquids to enter the first reaction device 70 or the second reaction device 72, thereby enabling multiple cycles of reactions/multiple repeated reactions.

Illustratively, in the case that the three-way valve 82 allows the second flow channel of the first flow path selection valve 74 to be in communication with the multi-way valve 84, the first reaction device 70 may perform the second reaction, and at this time, when one of the liquid inlets 86 is switched to be in communication with the liquid outlet 88, the switching between multiple reagents may be performed, thus performing a designated process or step of the second reaction in the second reaction device 72. By switching the communication positions of the multi-way valve 84, the multiple reagents passing through different liquid inlets 86 may enter the first reaction device 70 in a designated order to perform the second reaction.

It can be understood that in the case that the three-way valve 82 allows the second flow channel of the second flow path selection valve 76 to be in communication with the multi-way valve 84, the reagent passing through the multi-way valve 84 and the three-way valve 82 can enter the second reaction device 72 to perform the second reaction.

As shown in FIG. 19, in certain embodiments, the fluid path system 12 includes a liquid trap 89. The liquid trap 89 collects the liquid flowing out of the reaction device 62. For example, the liquid trap 89 collects the liquid after the first reaction and the second reaction.

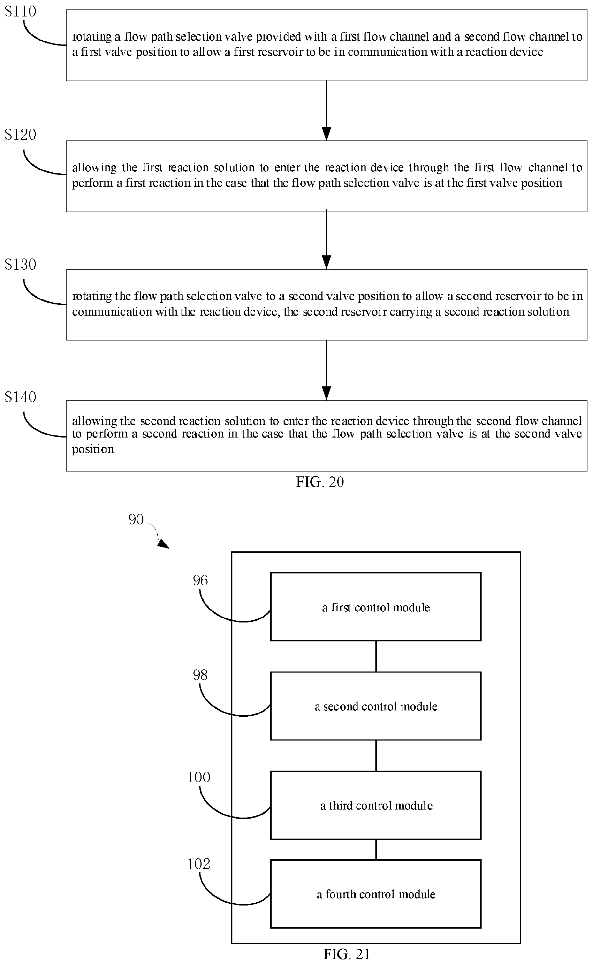

Referring to FIG. 20, the present application also provides a method for determining a nucleic acid sequence, which includes: S 110, rotating a flow path selection valve 10 provided with a first flow channel and a second flow channel to a first valve position to allow a first reservoir 66 to be in communication with a reaction device 62, the first reservoir 66 carrying a first reaction solution, and the first reaction solution containing a nucleic acid molecule; S 120, allowing the first reaction solution to enter the reaction device 62 through the first flow channel to perform a first reaction in the case that the flow path selection valve 10 is at the first valve position, the first reaction including attaching at least a portion of the nucleic acid molecule to the reaction device 62; S 130, rotating the flow path selection valve 10 to a second valve position to allow a second reservoir 68 to be in communication with the reaction device 62, the second reservoir 68 carrying a second reaction solution, and the second reaction solution containing components necessary for nucleic acid sequencing; and S 140, allowing the second reaction solution to enter the reaction device 62 through the second flow channel to perform a second reaction in the case that the flow path selection valve 10 is at the second valve position, the second reaction including allowing the nucleic acid molecule in the reaction device 62 after the first reaction is performed to interact with the second reaction solution to perform a polymerization reaction and detecting a signal from the reaction, so as to achieve sequence determination of the nucleic acid molecule. The method achieves the first reaction and the second reaction by allowing one flow path selection valve 10 to be at different valve positions to achieve the switching of different flow channels/reagents, and is particularly suitable for the operation control of a highly integrated system or apparatus.

The current next-generation high-throughput sequencing platforms or single-molecule sequencing platforms generally require processing of the sample under test before on-line sequencing. For example, in order to adapt to a designated sequencing platform, it is necessary to process the sample under test to convert it into a library adapted to the sequencing platform, and to load the library into a designated region, such as a reaction device 62, so as to place the reaction device 62 containing the sample under test into a sequencer for automated sequencing. Currently, in commercially available sequencing platforms, the processing of the sample under test before loading on a machine is generally separated from the on-line sequencing. For example, the sample processing/library preparation is performed manually in a reagent tube, or the processing and loading of the sample under test is performed on a sample processing apparatus. The method can achieve sample processing before loading on a machine and sequencing, and achieve the switching and the access control of different flow channels for a plurality of reagents by allowing the flow path selection valve 10 provided with a first flow channel and a second flow channel to be at different valve positions, and is particularly suitable for an integrated sequencing platform integrating the functions of sample processing before loading on a machine and sequencing.

Referring to FIG. 21, the method for determining a nucleic acid sequence described above can be implemented by a nucleic acid sequence determination system 90. Specifically, the nucleic acid sequence determination system 90 may include a first control module 96, a second control module 98, a third control module 100, and a fourth control module 102. The step S 110 can be implemented by the first control module 96, the step S120 can be implemented by the second control module 98, the step S130 can be implemented by the third control module 100, and the step S 140 can be implemented by the fourth control module 102.

In other words, the first control module 96 is configured to rotate the flow path selection valve 10 provided with a first flow channel and a second flow channel to the first valve position to allow a first reservoir 66 to be in communication with a reaction device 62. The first reservoir 66 carries a first reaction solution, and the first reaction solution contains a nucleic acid molecule. The second control module 98 is configured to allow the first reaction solution to enter the reaction device 62 through the first flow channel to perform a first reaction in the case that the flow path selection valve 10 is at the first valve position. The first reaction includes attaching at least a portion of the nucleic acid molecule to the reaction device 62. The third control module 100 is configured to rotate the flow path selection valve 10 to a second valve position to allow a second reservoir 68 to be in communication with the reaction device 62. The second reservoir 68 carries a second reaction solution, and the second reaction solution contains components necessary for nucleic acid sequencing. The fourth control module 102 is configured to allow the second reaction solution to enter the reaction device 62 through the second flow channel to perform a second reaction in the case that the flow path selection valve 10 is at the second valve position. The second reaction includes allowing the nucleic acid molecule in the reaction device 62 after the first reaction is performed to interact with the second reaction solution to achieve the sequence determination of the nucleic acid molecule.

The first to fourth control modules 96, 98, 100 and 102 are each independently a physical device such as an automated detecting instrument and/or a non-physical device such as a computer-executable program, and these control modules can independently control the operation of elements or assemblies such as flow path selection valves, pump assemblies, and detection assemblies. The control modules may be constituting parts of a system, or may be separate apparatuses or modules between which signal or data transmission is possible. They can be provided in the same space or in different spaces, and the space may be either a physical space or a digital space.

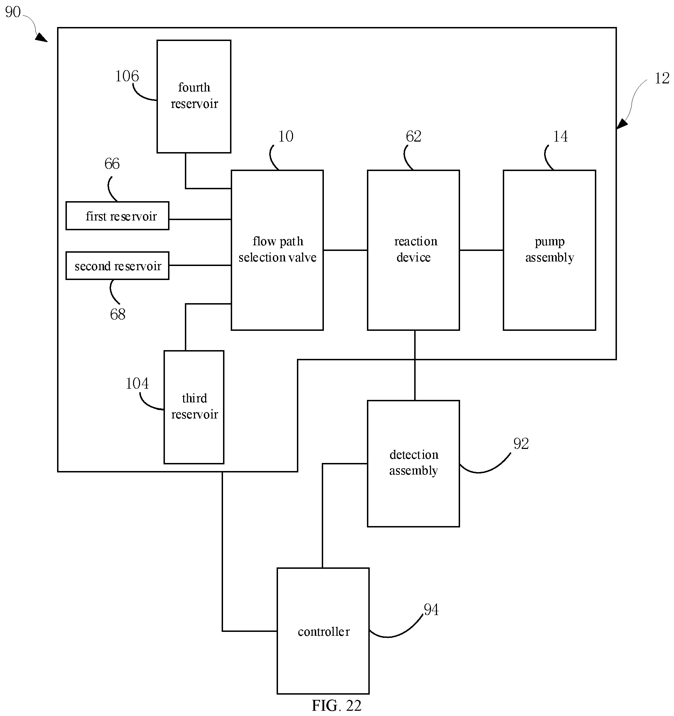

In the method for determining a nucleic acid sequence and the nucleic acid sequence determination system 90 described above, the flow path selection valve 10 may be used to perform independent or parallel three-way control on the first flow channel and the second flow channel, and it may be avoided that a plurality of three-way valves are simultaneously provided in the fluid path system, so that the fluid path system has decreased cost, reduced size, less reagent consumption and improved reliability, and is convenient to maintain, repair and control. In addition, the flow path selection valve 10 being at different positions can realize independent control and operation of the flow directions of different liquids, so that the sequence determination of nucleic acid molecules is easier to perform. Referring to FIG. 22, in certain embodiments, the method for determining a nucleic acid sequence further includes performing the following step after performing the step S120 and before performing the step S 140 (after performing the first reaction and before performing the second reaction): allowing a third reaction solution in a third reservoir 104 to enter the reaction device 62 through the first flow channel or the second flow channel to perform a third reaction, the third reaction including allowing the nucleic acid molecule in the reaction device 62 after the first reaction is performed to interact with the third reaction solution to achieve amplification of the nucleic acid molecule, and the third reaction solution containing components necessary for the amplification.

The "amplification" refers to cloning a nucleic acid molecule. For example, by polymerase chain reaction, the nucleic acid molecule is replicated to get thousands or millions of copies and thus becomes a cluster, and any one molecule of the thousands or millions of copies or the cluster is identical in sequence to the original nucleic acid molecule. As such, the signal from the nucleic acid molecule can be amplified by increasing the number of molecules, thus facilitating detection of the nucleic acid molecule; specifically, in subsequent sequencing, the signals emitted by the thousands or millions of molecules (clusters) are equal to the signal from the single nucleic acid molecule, which greatly enhances the signal of the molecule and is beneficial to detection.

The second generation high-throughput sequencing platforms currently on the market, such as ILLUMINA sequencing platform, Ion Torrent sequencing platform and BGI sequencing platform, all require magnifying of the signal from the molecule under test by amplification such as bridge PCR before sequencing to obtain a stronger signal that is easily identified for detection (or not susceptible to interference).

Referring to FIG. 22, in certain embodiments, the method for determining a nucleic acid sequence further includes performing the following step before performing the step S 120 (before performing the first reaction): allowing a cleaning solution in a fourth reservoir 106 to enter the reaction device 62 through the first flow channel. As such, the cleaning solution may rinse the reaction device 62, thereby preventing the first reaction solution from being contaminated by the residual substance of last reaction and/or reducing unnecessary consumption of the first reaction solution, e.g., a trace amount of biological sample solution (e.g., filling the tube of the fluid path system 12).

In certain embodiments, the method for determining a nucleic acid sequence further includes performing the following step before performing the step S 120 (before performing the first reaction): introducing air into the reaction device 62 through the first flow channel. As such, before the first reaction is started, air is injected to fill a section of the fluid path system to separate the first reaction solution which will flow into the fluid path system subsequently from the cleaning solution already in the fluid path system, thereby avoiding a situation where the trace amount of biological sample is diffused and/or diluted and thereby the attachment of the nucleic acid molecule under test to the reaction device 62 and the subsequent detection of the nucleic acid molecule under test is affected.

In certain embodiments, the method for determining a nucleic acid sequence further includes performing the following step before performing the step S 120 and/or before performing the step S 140 (before performing the first reaction and/or before performing the second reaction): allowing the cleaning solution in the fourth reservoir 106 to enter the reaction device 62 through the first flow channel or the second flow channel. As such, the cleaning solution may clean the reaction device 62, so that the subsequent first reaction solution and/or second reaction solution may be prevented from being affected by the residue of the last reaction or the last step.

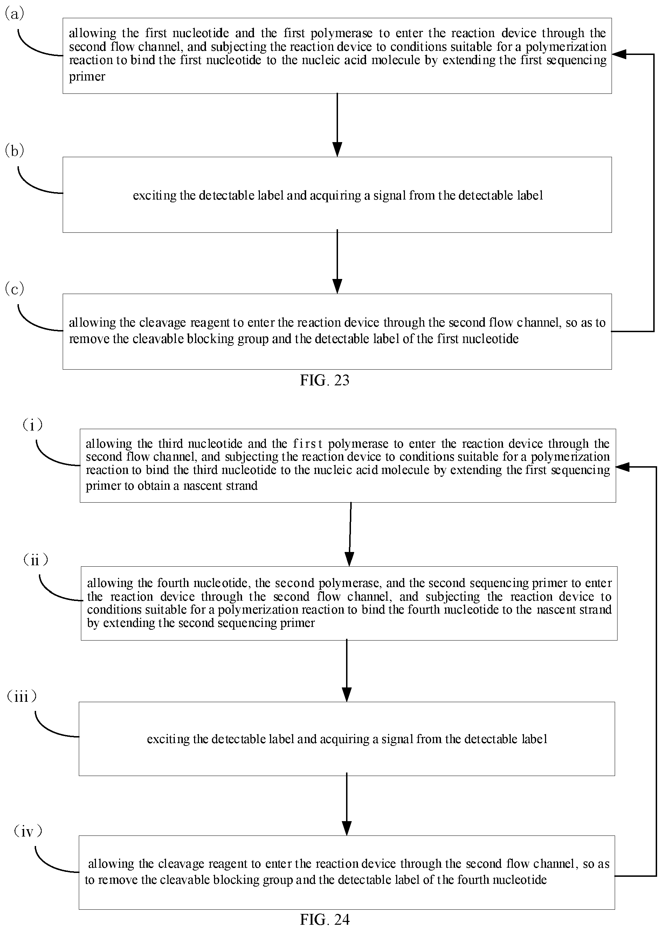

Referring to FIG. 23, in certain embodiments, the reaction device 62 has a solid carrier surface on which a first sequencing primer is immobilized. At least one end of the nucleic acid molecule includes at least a portion of a sequence capable of complementarily pairing with at least a portion of the first sequencing primer, and the first reaction includes complementary pairing of at least a portion of the nucleic acid molecule with the first sequencing primer for attachment to the reaction device 62.

The "first sequencing primer" is a fragment of oligonucleotide (a short nucleic acid sequence with known sequence), and such a known sequence immobilized on the surface of the chip is also commonly referred to as a "probe".

In certain embodiments, the second reaction solution includes a first nucleotide, a first polymerase, and a cleavage reagent. The step S140 includes: (a) allowing the first nucleotide and the first polymerase to enter the reaction device 62 through the second flow channel, and subjecting the reaction device 62 to conditions suitable for a polymerization reaction to bind the first nucleotide to a nucleic acid molecule by extending a first sequencing primer, the first nucleotide containing a base, a sugar unit, a cleavable blocking group, and a detectable label; (b) exciting the detectable label and acquiring a signal from the detectable label; (c) allowing the cleavage reagent to enter the reaction device 62 through the second flow channel, so as to remove the cleavable blocking group and the detectable label of the first nucleotide; and (d) repeating the steps (a)-(c) at least once.

Specifically, by "subjected to conditions suitable for a polymerization reaction", temperature conditions are generally involved in addition to the components/reagents required for the polymerization reaction (e.g., a polymerase, a reaction substrate, i.e., a nucleotide, and/or a sequencing primer). For example, the nucleic acid sequence determination system 90 may further include a temperature control system to control the temperature of the reaction device 62/reaction chamber to achieve "conditions suitable for a polymerization reaction". The detectable label is, for example, an optically detectable label, such as a fluorescent molecule. The cleavable blocking group may prevent/inhibit the binding of the other nucleotides (first nucleotide) in the reaction system to the next position of the nucleic acid molecule under test, either in a physically blocking manner, e.g., by attaching an azido group (-N₃) to the 3' position of the glycosyl, or in a non-physical blocking (virtual blocking) manner, e.g., by forming a spatial conformation that can prevent the extension from proceeding in the extension solution system.

In certain embodiments, the second reaction solution further includes a second nucleotide. The step S140 further includes performing the following step after (a): allowing the second nucleotide and the first polymerase to enter the reaction device 62 through the second flow channel, and subjecting the reaction device 62 to conditions suitable for a polymerization reaction to allow the second nucleotide to bind to the nucleic acid molecule by proceeding to extend a product after (a), the second nucleotide containing a base, a sugar unit, and a cleavable blocking group. Compared with the first nucleotide, the second nucleotide is a reversible terminator without a detectable label, and the reaction efficiency of the second nucleotide is generally higher than that of the first nucleotide for the same polymerase. This step facilitates the synchronization of the reactions of multiple nucleic acid molecules in a cluster, that is, it can eliminate or reduce the prephasing or phasing nucleic acid molecules in a cluster to some extent, and thus facilitates the sequencing reaction.

Referring to FIG. 24, in certain embodiments, the second reaction solution includes a third nucleotide, a fourth nucleotide, a second polymerase, a third polymerase, a cleavage reagent, and a second sequencing primer, and at least one end of the nucleic acid molecule includes at least a portion of a sequence capable of complementarily pairing with at least a portion of the second sequencing primer. Allowing the second reaction solution to enter the reaction device 62 through the second flow channel to perform the second reaction includes: (i) allowing the third nucleotide and the second polymerase to enter the reaction device 62, and subjecting the reaction device 62 to conditions suitable for a polymerization reaction to bind the third nucleotide to the nucleic acid molecule by extending the first sequencing primer to obtain a nascent strand, the third nucleotide being a nucleotide that carries neither a cleavable blocking group nor a detectable label; (ii) allowing the fourth nucleotide, the third polymerase, and the second sequencing primer to enter the reaction device 62 through the second flow channel, and subjecting the reaction device 62 to conditions suitable for a polymerization reaction to bind the second sequencing primer to the nascent strand and bind the fourth nucleotide to the nascent strand by extending the second sequencing primer, the fourth nucleotide containing a base, a sugar unit, a cleavable blocking group and a detectable label; (iii) exciting the detectable label and acquiring a signal from the detectable label; (iv) allowing the cleavage reagent to enter the reaction device 62 through the second flow channel to remove the cleavable blocking group and the detectable label of the fourth nucleotide; and (v) repeating the steps (ii)-(iv) at least once. As such, the second reaction can be performed through the steps (ii)-(v) to achieve determination of the nucleic acid sequence. The third nucleotide may be, for example, a natural nucleotide. The fourth nucleotide may be identical to the first nucleotide. The first to third polymerases may be the same or different. For example, they are different types of DNA polymerases or different mutants of the same type of DNA polymerase, and they are each capable of independently binding to a designated nucleotide and thereby effectively catalyzing the performance of a designated extension/polymerization reaction. Compared with the sequencing method of the previous embodiment, the method can determine the sequence of the other end of the nucleic acid molecule under test by synthesizing the complementary strand of the nucleic acid molecule under test, thus achieving sequencing of the other end of the nucleic acid molecule under test.

Similarly, in certain embodiments, the second reaction solution further includes a fifth nucleotide, and the method further includes after (ii), allowing the fifth nucleotide and the third polymerase to enter the reaction device 62 through the second flow channel, and subjecting the reaction device 62 to conditions suitable for a polymerization reaction to bind the fifth nucleotide to the nascent strand by proceeding to extend a product after (ii), the fifth nucleotide containing a base, a sugar unit, and a cleavable blocking group. Similar to, for example, the second nucleotide, the fifth nucleotide is a reversible terminator without a detectable label. This step facilitates the synchronization of the reactions of multiple nucleic acid molecules in a cluster, that is, it can eliminate or reduce the prephasing or phasing nucleic acid molecules in a cluster to some extent, and thus facilitates the sequencing reaction and the obtaining of longer reads.

It should be noted that the explanations of the technical features such as the structure, connection relationship and operation control of the flow path selection valve 10 and/or the fluid path system 12 in the embodiments described above are also applicable to the method for determining a nucleic acid sequence according to any one of the embodiments, and how to implement the method for determining a nucleic acid sequence according to any one of the embodiments by using the flow path selection valve 10 and the related elements/structural assemblies is not shown here. Those skilled in the art can understand how to utilize and control the flow path selection valve 10 and/or the fluid path system 12 and/or the sequencing system 90 in the embodiments described above to implement the corresponding sequencing method by way of the above description of the structure, connection relationship, function and operation manner of the flow path selection valve 10 and/or the fluid path system 12 in the above embodiments and the current illustration of the sequencing method.

Referring to FIG. 22, an embodiment of the present application further provides a nucleic acid sequence determination system 90 (sequencing system 90). The nucleic acid sequence determination system 90 includes the fluid path system 12, the detection assembly 92, and the controller 94 according to any one of the embodiments described above. Illustratively, the fluid path system 12 includes a first reservoir 66, a second reservoir 68, a flow path selection valve 10, and a reaction device 62. The detection assembly 92 is configured to detect a signal from the reaction device 62 during a designated reaction, and the controller 94 is configured to control the fluid path system 12 and the detection assembly 92 to perform the method for determining a nucleic acid sequence according to any one of the embodiments described above.

For example, the controller 94 is configured to: rotate a flow path selection valve 10 provided with a first flow channel and a second flow channel to a first valve position to allow a first reservoir 66 to be in communication with a reaction device 62, the first reservoir 66 carrying a first reaction solution, and the first reaction solution containing a nucleic acid molecule; allow the first reaction solution to enter the reaction device 62 through the first flow channel to perform a first reaction in the case that the flow path selection valve 10 is at the first valve position, the first reaction including attaching at least a portion of the nucleic acid molecule to the reaction device 62; rotate the flow path selection valve 10 to a second valve position to allow a second reservoir 68 to be in communication with the reaction device 62, the second reservoir 68 carrying a second reaction solution, and the second reaction solution containing components necessary for nucleic acid sequencing; and allow the second reaction solution to enter the reaction device 62 through the second flow channel to perform a second reaction in the case that the flow path selection valve 10 is at the second valve position, the second reaction including allowing the nucleic acid molecule in the reaction device 62 after the first reaction is performed to interact with the second reaction solution to perform a polymerization reaction and detecting a signal from the reaction, so as to achieve sequence determination of the nucleic acid molecule. The nucleic acid sequence determination system 90 described herein is, for example, a machine such as a sequencer or a sequencing platform.

For another example, the controller 94 is configured to control the pump assembly 14 and the flow path selection valve 10 to allow the liquid in the reservoir 64 to flow toward the reaction device 62 through the first flow channel, to control the pump assembly 14 and the flow path selection valve 10 to allow the liquid in the reservoir 64 to flow toward the reaction device 62 through the second flow channel, and to control the detection assembly 92 to detect a signal from the reaction device 62 when the liquid in the reservoir 64 flows through the reaction device 62 or after the liquid in the reservoir 64 flows through the reaction device 62.

For yet another example, the first reservoir 66 carries a biological sample solution, and the biological sample solution contains a nucleic acid molecule. The second reservoir 68 carries a reaction solution, and the reaction solution contains components necessary for a polymerization reaction. The controller 94 is configured to control the pump assembly 14 and the flow path selection valve 10 to allow the biological sample solution in the first reservoir 66 to enter the reaction device 62 to perform a first reaction, the first reaction including allowing at least a portion of the nucleic acid molecule to be attached to the reaction device 62, and the controller 94 is configured to control the pump assembly 14 and the flow path selection valve 10 to allow the reaction solution in the second reservoir 68 to enter the reaction device 62 to perform a second reaction after the first reaction is performed, and control the detection assembly 92 to acquire a signal produced by the second reaction, the second reaction including allowing the nucleic acid molecule in the reaction device 62 to interact with the reaction solution to perform a polymerization reaction.

In certain embodiments, the second reaction produces an optical signal, and the detection assembly 92 includes an imaging detector that detects the optical signal.

A computer-readable storage medium according to an embodiment of the present application is configured to store a program executed by a computer, and executing the program includes implementing the method for determining a nucleic acid sequence according to any one of the embodiments described above. It should be noted that the explanations of technical features in the method for determining a nucleic acid sequence in the embodiments described above are also applicable to the computer-readable storage medium for implementing the embodiment, and detailed descriptions of the computer-readable storage medium according to an embodiment of the present application are not shown here.

According to an embodiment of the present application, provided is a system configured to perform the method for determining a nucleic acid sequence according to any one of the embodiments described above. It should be noted that the explanations of the technical features of the method for determining a nucleic acid sequence, the flow path selection valve 10, and/or the fluid path system 12 in the embodiments described above are also applicable to the system for implementing the embodiment, and reference may be made to the method for determining a nucleic acid sequence, the flow path selection valve 10, and/or the fluid path system 12 with respect to the implementation of the system according to the embodiment of the present application, which is not shown here.

A computer program product including instructions according to an embodiment of the present application includes performing the method for determining a nucleic acid sequence according to any one of the embodiments described above when the program is executed by a computer. It should be noted that the explanations of technical features in the method for determining a nucleic acid sequence in the embodiments described above are also applicable to the computer program product for implementing the embodiment, and detailed descriptions of the computer program product according to an embodiment of the present application are not shown here. A nucleic acid sequence determination system according to an embodiment of the present application includes the computer program product described above.

### Embodiment 20:

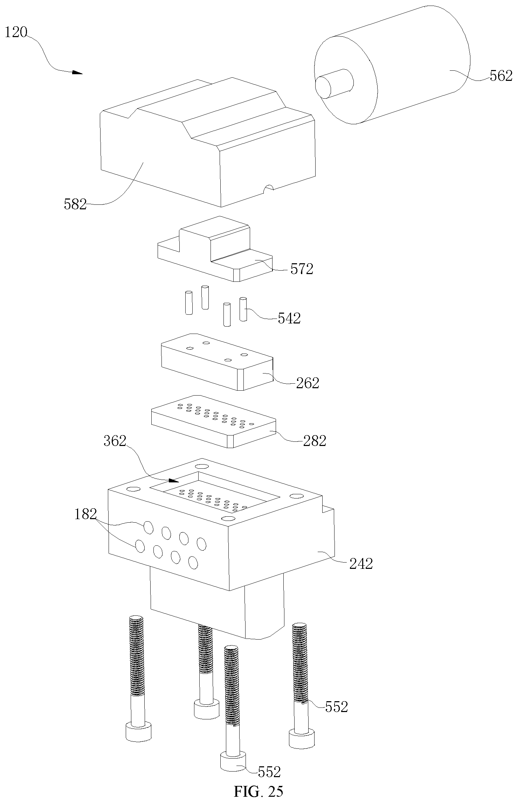

Referring to FIGs. 25-26, an embodiment of the present application provides a flow path selection valve 120. The flow path selection valve 120 may be applied to a fluid path system 122 or the fluid path system 122 includes the flow path selection valve 120, thus achieving flow confluence, flow splitting, flow path switching and/or flow rate control.

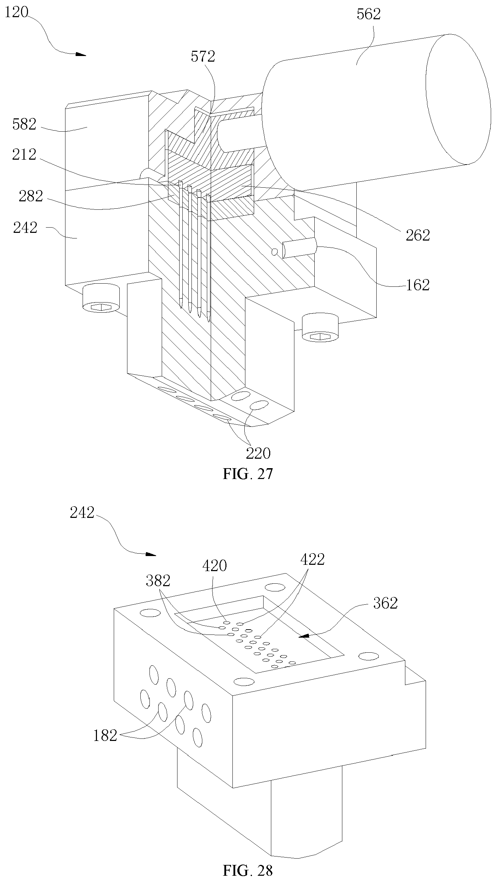

Referring to FIG. 25 and FIG. 27, the flow path selection valve 120 according to an embodiment of the present application is configured to rotate between a first valve position and a second valve position, and the flow path selection valve 120 has a common port 162, a plurality of first ports 182, a plurality of second ports 220, and a plurality of communication grooves 212. The communication groove 212 may selectively allow the common port 162 to be in communication with a first port 182, or allow a first port 182 to be in communication with a second port 220.

In the case that the flow path selection valve 120 is at the first valve position, the first port 182 is in communication with the second port 220 through the first communication groove 212. In the case that the flow path selection valve 120 is at the second valve position, the first port 182 is in communication with the common port 162 through the communication groove 212.

In the flow path selection valve 120 and the fluid path system 122 according to an embodiment of the present application, each first port 182 is in communication with the corresponding second port 220 through the corresponding communication groove 212 in the case that the flow path selection valve 120 is at the first valve position, and each first port 182 is in communication with the common port 162 through the corresponding communication groove 212 in the case that the flow path selection valve 120 is at the second valve position, so that the flow path selection valve 120 has two or more channels. The flow path selection valve 120 is suitable for any fluid path system requiring the use of a three-way valve, in particular a plurality of three-way valves, for example a fluid path system involving the change of the direction of one or more flow paths and/or the dispensing of a plurality of liquids/solutions. The application of the flow path selection valve to the fluid path system 122 including a plurality of fluid paths/flow paths enables independent or parallel three-way control on the plurality of fluid paths, and it may be avoided that a plurality of three-way valves are simultaneously provided in the fluid path system 122, so that the fluid path system 122 has decreased cost, reduced size, less reagent consumption and improved reliability, and is convenient to maintain, repair and control.

Specifically, the number of the common ports 162 is one, and the common port 162 may serve as a liquid inlet or outlet of the flow path selection valve 120. In other words, the liquid may enter or flow out from the flow path selection valve 120 from the common port 162, thereby achieving flow splitting or flow confluence. Here, the common port 162 serves as the liquid inlet of the flow path selection valve 120, and the liquid may enter the flow path selection valve 120 from the common port 162. The shape of the common port 162 may be a regular shape such as circular or polygonal, or may be an irregular shape. In an embodiment of the present application, the common port 162 is circular in shape to facilitate the forming and manufacturing of the common port 162 and/or connecting of it to a common tube.

The plurality of second ports 220 correspond to the plurality of first ports 182 in a one-to-one manner. The plurality of communication grooves 212 correspond to the plurality of first ports 182 in a one-to-one manner. The number of the first ports 182 may be 2, 3, 4, 5, 6, 7, 8, or more. In the embodiment shown in FIG. 25, the number of the first ports 182 is 82 to fit an element or device containing 82 outlets or inlets upstream or downstream of the flow path selection valve 120.

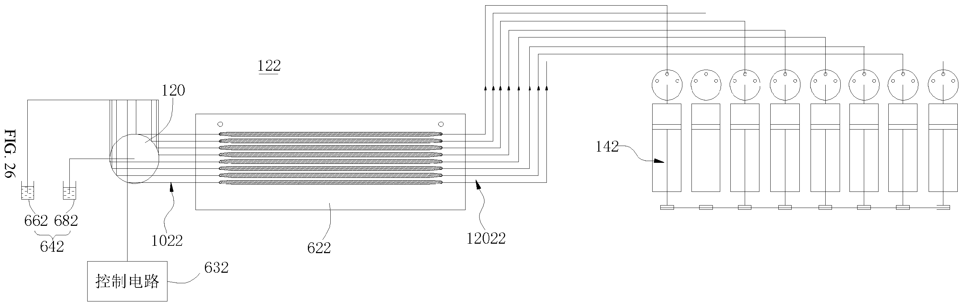

As shown in FIG. 26, the liquid may enter the flow path selection valve 120 from the common port 162, flow out from 82 first ports 182, and then enter a flow cell 622 containing 82 channels or 82 reaction regions, thus enabling the parallel control of the feeding liquid of the 82 channels or 82 reaction regions of the flow cell 622. The shape of the first port 182 may be a regular shape such as circular or polygonal, or may be an irregular shape. In an embodiment of the present application, the first port 182 is circular. The first port 182 may serve as a liquid outlet or an inlet of the flow path selection valve 120.

Similarly, the number of the second ports 220 is not limited in the present application, and the number of the second ports 220 may be 2, 3, 4, 5, 6, 7, 8, or more. In the embodiment shown in FIG. 27, the number of the second ports 220 is 82 to fit an element or device including 82 outlets or inlets upstream or downstream of the flow path selection valve 120, such as the flow cell 622 including 82 channels or 82 reaction regions downstream of the flow path selection valve 120 in FIG. 26, thus enabling the parallel control of the feeding liquid of the 82 channels or 82 reaction regions of the flow cell 622. The number of second ports 220 is equal to the number of first ports 182. The shape of the second port 220 may be a regular shape such as circular or polygonal, or may be an irregular shape. In an embodiment of the present application, the second port 220 is circular. The second port 220 may serve as a liquid inlet or an outlet of the flow path selection valve 120. For ease of manufacture, the second port 220 may have a same size as the first port 182.

The number of the communication grooves 212 is the same as the number of the first ports 182 and/or the second ports 220. In an embodiment of the present application, the number of the communication grooves 212 is also 82. The communication groove 212 is not limited in shape as long as it allows the first port 182 to be in communication with the second port 220 when the flow path selection valve 120 is at the first valve position, and it may be, for example, arc-shaped or rectangular.

In summary, when the flow path selection valve is at the first valve position, each of the first ports 182 is in communication with a corresponding second port 220, and at this time, the liquid enters the flow path selection valve 120 from the second port 220 and flows out from the corresponding first port 182. That is, the flow path selection valve 120 also has a function of simultaneous multi-channel liquid feeding and simultaneous multi-channel liquid discharging.

When the flow path selection valve is at the second valve position, the common port 162 is in communication with each of the first ports 182 through a corresponding communication groove 212, and at this time, the liquid can enter the flow path selection valve 120 from the common port 162, pass through the communication grooves 212, and then flow out from the first ports 182. In other words, when the flow path selection valve is at the second valve position, the liquid enters the flow path selection valve 120 from the common port 162 and flows out from a plurality of first ports 182. That is, the flow path selection valve 120 has a function of one-channel liquid feeding and multi-channel liquid discharging.

In an embodiment of the present application, in the case that the flow path selection valve 120 is at the first valve position, the first ports 182 and the second ports 220 are separated from the common port 162; in the case that the flow path selection valve 120 is at the second valve position, the common port 162 and the first ports 182 are separated from the second ports 220 to form independent first flow channels or second flow channels. The number of the first flow channels and the number of the second flow channels are both more than one, which is beneficial to the parallel control of liquid feeding and liquid discharging of a plurality of reaction regions and also the independent control of liquid feeding and liquid discharging of different reaction processes of the reaction regions.

In some embodiments, the flow path selection valve 120 may be at a third valve position, and in this case, the common port 162, the plurality of first ports 182, and the plurality of second ports 220 are separated from one another. As such, in the case that the flow path selection valve 120 is at the third valve position, the flow path selection valve 120 may also have the function of controlling the liquid shutoff.

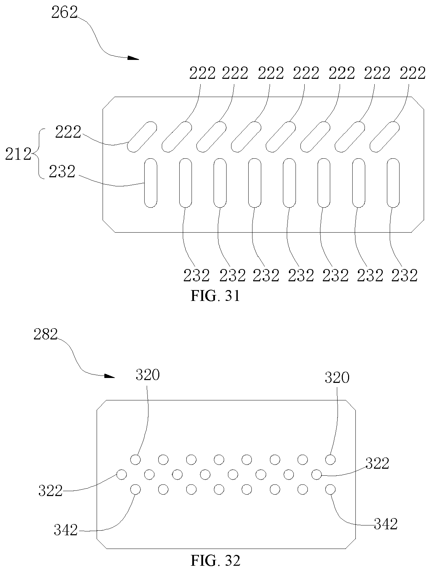

In this embodiment, the flow path selection valve 120 has three modes, that is, a mode in which the first ports 182 is in communication with the second port 220 when the flow path selection valve 120 is at the first valve position; a mode in which the common port 162 is in communication with the first port 182 when the flow path selection valve 120 is at the second valve position; and a mode in which the common port 162, the first ports 182, and the second ports 220 are separated from one another when the flow path selection valve 120 is at the third valve position. A plurality of ports being "separated" means that the plurality of ports cannot be in communication with one another, or that the liquid cannot enter from designated port or ports and flow out from other designated port or ports. Referring to FIGs. 25 and 27, in certain embodiments, the flow path selection valve 120 includes a manifold 242 and a first valve element 262. Referring to FIGs. 28-30, the manifold 242 has a common port 162, a plurality of first ports 182, and a plurality of second ports 220. Referring to FIG. 31, the first valve element 262 is provided with a communication groove 212. The first valve element 262 may rotate or slide relative to the manifold 242 to switch the flow path selection valve 120 between the first valve position and the second valve position. As such, during the rotation or sliding of the first valve element 262, the communication groove 212 may allow the common port 162 to be in communication with the first port 182, or the first port 182 to be in communication with the second port 220, so that liquid may enter the flow path selection valve 120 from the manifold 242 and flow out of the flow path selection valve 120 from the manifold 242.

Specifically, the manifold 242 is a flow splitting or confluence module, and the manifold 242 may split the liquid entering from the common port 162 to allow the liquid to flow out of the plurality of first ports 182, or direct the liquid entering from the second port 220 to flow out of the corresponding first port 182. It will be understood that the interior of the manifold 242 has flow channels in communication with the ports.

In an example of the present application, the first valve element 262 is in a square shape, and the first valve element 262 may slide relative to the manifold 242. The communication groove 212 is in a linear shape. The communication groove 212 may be in a bent shape, a curved shape, or the like, and the specific shape of the communication groove 212 is not limited herein. Illustratively, the number of the communication grooves 212 is the same as the number of the first ports 182. In an embodiment of the present application, the number of the communication grooves 212 is 82. Of course, in other embodiments, the number of the communication grooves 212 may be other numbers such as 22, 32, 42, 52, and 62.

Referring to FIGs. 25, 27, and 32, in certain embodiments, the flow path selection valve 120 includes a second valve element 282 provided on the manifold 242. The first valve element 262 is provided on the second valve element 282, and the second valve element 282 is provided with a first channel 320, a second channel 322, and a third channel 342. The first channel 320 is in communication with the first port 182, the second channel 322 is in communication with the second port 220, and the third channel 342 is in communication with the common port 162.

In the case that the flow path selection valve 120 is at the first valve position, the communication groove 212 allows the first channel 320 to be in communication with the second channel 322 to achieve the communication between the first port 182 and the second port 220. In the case that the flow path selection valve 120 is at the second valve position, the communication groove 212 allows the first channel 320 to be in communication with the third channel 342 to achieve the communication between the first port 182 and the common port 162. As such, the first valve element 262 selectively allows the first port 182 to be in communication with the common port 162, or the first port 182 to be in communication with the second port 220 through the second valve element 282, so that the preparation of the flow path selection valve is facilitated.

Specifically, the second valve element 282 is in a square shape, and the size of both the first valve element 262 and the second valve element 282 is less than the size of the manifold 242. Since the manifold 242 and the first valve element 262 are separately manufactured, the accuracy of both may not allow the manifold 242 and the first valve element 262 to meet the fitting requirements, resulting in liquid leakage. Since the first valve element 262 and the second valve element 282 are in relatively regular shapes and are easy to manufacture, in an embodiment of the present application, connecting the manifold 242 to the first valve element 262 through the second valve element 282 may make the flow path selection valve 120 easier to manufacture and more stable.

In other embodiments, the second valve element 282 may be omitted. At this time, the first valve element 262 forms a fitting connection with the manifold 242.

In an embodiment of the present application, the first channel 320, the second channel 322, and the third channel 342 are independent channels, with each channel separately provided. The first channel 320, the second channel 322, and the third channel 342 are all in a linear shape, and all pass through the second valve element 282 along a thickness direction of the second valve element 282.

In other embodiments, at least one of the first channel 320, the second channel 322, and the third channel 342 may be in a bent shape, a curved shape, or the like, and the specific shapes of the first channel 320, the second channel 322, and the third channel 342 are not limited herein.

In an example of the present application, the number of the first channels 320 is the same as the number of the first ports 182, both of which are 82. The number of the second channels 322 is the same as the number of the second ports 220, both of which are 82. The number of the third channels 342 is 82. Of course, in other embodiments, the number of the first channels 320, second channels 322, and the third channels 342 may be other numbers such as 22, 32, 42, 52, and 62.

As in an example of FIG. 32, the first channels 320, the second channels 322, and the third channels 342 are arranged in a matrix. 82 first channels 320, 82 second channels 322, and 82 third channels 342 are each provided at intervals along a length direction of the second valve element 282, and 82 first channels 320 are arranged along a first straight line, 82 second channels 322 are arranged along a second straight line, and 82 third channels 342 are arranged along the third channels 342. Along a width direction of the second valve element 282, the first channels 320 and the third channels 342 are arranged in a one-to-one alignment manner, and the first channels 320 and the second channels 322 are arranged in a staggered manner.

In other embodiments, the first channels 320, the second channels 322, and the third channels 342 may be arranged in other manners, and the arrangement of the first channels 320, the second channels 322, and the third channels 342 is not limited herein.

In an embodiment of the present application, the second valve element 282 is fixedly provided relative to the manifold 242. The first valve element 262 slides on the surface of the second valve element 282 relative to the second valve element 282 to enable the sliding of the first valve element 262 between the first position and the second position, so that the communication groove 212 may selectively allow the first channel 320 to be in communication with the second channel 322, or the first channel 320 to be in communication with the third channel 342.

Referring to FIGs. 27 and 28, in certain embodiments, the manifold 242 is provided with a cavity 362. The second valve element 282 is at least partially received in the cavity 362. As such, the manifold 242 forms a more compact fit with the second valve element 282, so that the size of the flow path selection valve 120 may be reduced, thereby facilitating the miniaturization of the flow path selection valve 120.

Specifically, the shape of the cavity 362 is adapted to the shape of the second valve element 282. In an embodiment of the present application, the cavity 362 is also in a cuboid shape. As shown in FIG. 27, the second valve element 282 is partially received in the cavity 362, alternatively, a portion of the second valve element 282 protrudes from the cavity 362.

Referring to FIG. 28, in certain embodiments, the bottom surface of the cavity 362 is provided with a first connection port 382, a second connection port 420, and a third connection port 422. The first connection port 382 allows the first port 182 to be in communication with the first channel 320. The second connection port 420 allows the second port 220 to be in communication with the second channel 322. The third connection port 422 allows the common port 162 to be in communication with the third channel 342. As such, the second valve element 282 is in communication with the first port 182, the second port 220, and the common port 162 through the connection ports provided at the bottom surface of the cavity 362, so that the contact area between the second valve element 282 and the cavity 362 may be increased, thereby preventing liquid leakage.

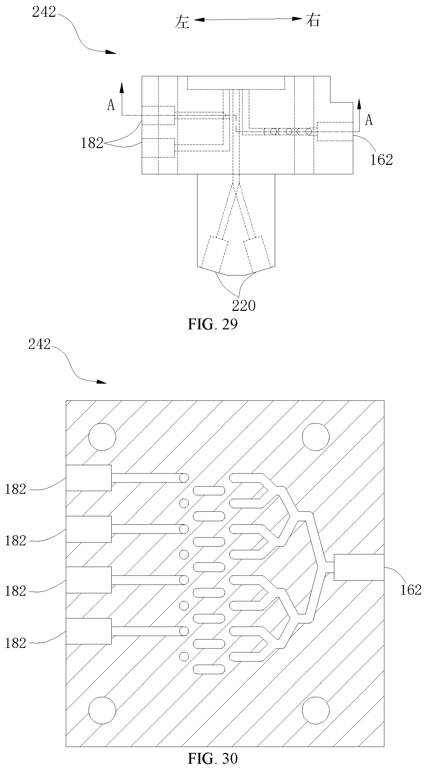

In order to allow the first connection port 382 to be in butt joint with the first channel 320, the second connection port 420 to be in butt joint with the second channel 322, and the third connection port 422 to be in butt joint with the third channel 342, the arrangement of the connection port 382, the second connection port 420, and the third connection port 422 may be the same as the arrangement of the first channel 320, the second channel 322, and the third channel 342, which will not be repeated here. Referring to FIGs. 28 and 29, in certain embodiments, at least two of the first port 182, the second port 220, and the common port 162 are located on different sides of the manifold 242. For example, the first port 182 and the second port 220 are located on one side of the manifold 242, and the common port 162 is located on the other side of the manifold 242. For another example, the first port 182 and the common port 162 are located on one side of the manifold 242, and the second port 220 is located on the other side of the manifold 242. For yet another example, the first port 182, the second port 220, and the common port 162 are located on different sides of the manifold 242.

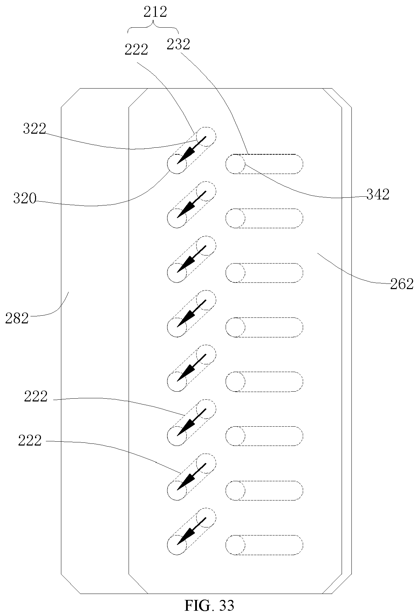

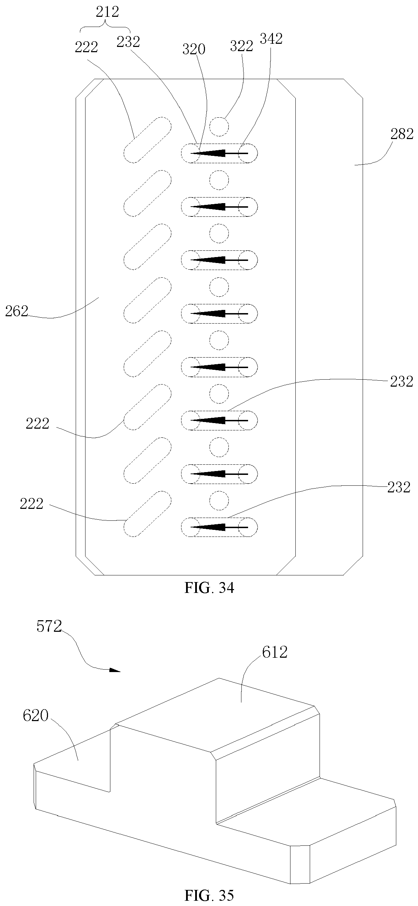

In the orientation illustration of FIG. 29, the first port 182 is located on the left side of the manifold 242, the second port 220 is located on the bottom side of the manifold 242, and the common port 162 is located on the right side of the manifold 242. As such, the ports are provided on different sides of the manifold 242 to make full use of space in the manifold 242, so that the manifold 242 is more miniaturized and the connection of the manifold 242 with other components may be facilitated. Referring to FIG. 31, in certain embodiments, the communication groove 212 includes first communication grooves 222 and second communication grooves 232 provided at intervals. In the case that the flow path selection valve 120 is at the first valve position, the first port 182 is in communication with the second port 220 through the first communication groove 222, as shown in FIG. 33. In the case that the flow path selection valve 120 is at the second valve position, the first port 182 is in communication with the common port 162 through the second communication groove 232, as shown in FIG. 34. Alternatively, the communication groove 212 includes two discontinuous portions, which may facilitate control of the communication of the first port 182 with the second port 220 and the communication of the first port 182 with the common port 162 through the communication groove 212.

The arrows in FIGs. 33 and 34 represent the flow direction of the liquid. As in FIG. 33, after entering the flow path selection valve 120 from the second port 220, the liquid passes through the second channel 322, the first communication groove 222, and the first channel 320 sequentially, and flows out of the first port 182.

In FIG. 34, after entering the flow path selection valve 120 from the common port 162, the liquid passes through the third channel 342, the second communication groove 342, and the first channel 320 sequentially, and flows out of the first port 182.

As in an example of FIG. 31, in order to adapt to the arrangement of the first channel 320, the second channel 322, and the third channel 342, the first communication groove 222 extends linearly and obliquely relative to the width direction of the first valve element 262, and the second communication groove 232 extends linearly along the width direction of the first valve element 262.

It will be understood that FIG. 31 is merely one example of the first communication groove 222 and the second communication groove 232.

In other embodiments, the first communication groove 222 and the second communication groove 232 may be in an arc shape, a bent shape, or the like.

In addition, in other embodiments, one of the first communication groove 222 and the second communication groove 232 may be omitted, alternatively, the communication groove 212 may be a groove of a continuous structure. At this time, in one example, the first channel 320 may be provided between the second channel 322 and the third channel 342, and the first channel 320, the second channel 322, and the third channel 342 are arranged along one straight line. The communication groove 212 is in a linear shape. When the first valve element 262 is located at the first position, one end of the communication groove 212 is in communication with the first channel 320, and the other end is in communication with the second channel 322, so that the first port 182 is in communication with the second port 220. When the second valve element 282 is located at the second position, one end of the communication groove 212 is in communication with the third channel 342, and the other end is in communication with the first channel 320, so that the common port 162 is in communication with the first port 182.

Referring again to FIGs. 25 and 26, in certain embodiments, the flow path selection valve 120 includes a drive component 562. The drive component 562 is configured to drive the first valve element 262 to rotate or slide. As such, the drive component 562 may drive the first valve element 262 to move, so that the first valve element 262 may be located in different positions to enable the flow path selection valve 120 to achieve different functions.

Specifically, the drive component 562 may provide an electromagnetic drive. For example, the drive component 562 may be a motor. When the first valve element 262 slides relative to the manifold 242, the drive component 562 may be a motor having a screw structure. When the first valve element 262 rotates relative to the manifold 242, the drive component 562 may be a motor having a rotation shaft. Referring to FIGs. 25, 27, and 35, in certain embodiments, the flow path selection valve 120 includes a slide block 572 connected to the first valve element 262. The slide block 572 is connected to the drive component 562, and the drive component 562 drives the first valve element 262 to slide through the slide block 572. As such, the drive component 562 is facilitated to drive the first valve element 262 to slide.

Specifically, the slide block 572 is detachably connected to the first valve element 262. For example, the slide block 572 is connected to the first valve element 262 through positioning pins 542. After the driving force of the drive component 562 is applied to the slide block 572, the slide block 572 may drive the first valve element 262 to slide through the positioning pins. Of course, the slide block 572 may be connected to the first valve element 262 through a snap-fit structure, and the specific connection manner between the slide block 572 and the first valve element 262 is not limited herein.

When the drive component 562 is a screw motor, the slide block 572 may be sleeved on a screw of the screw motor. During the rotation of the screw, the slide block 572 may move relative to the screw, thereby driving the first valve element 262.

Referring to FIGs. 25 and 27, in certain embodiments, the flow path selection valve 120 includes a housing 582 detachably connected to the manifold 242. The first valve element 262 and the slide block 572 are received in the housing 582. As such, the housing 582 may protect the first valve element 262 and the slide block 572 and provide a pressing force to the first valve element 262 and the second valve element 282, thereby avoiding undesirable leakage of the first valve element 262 and the second valve element 282.

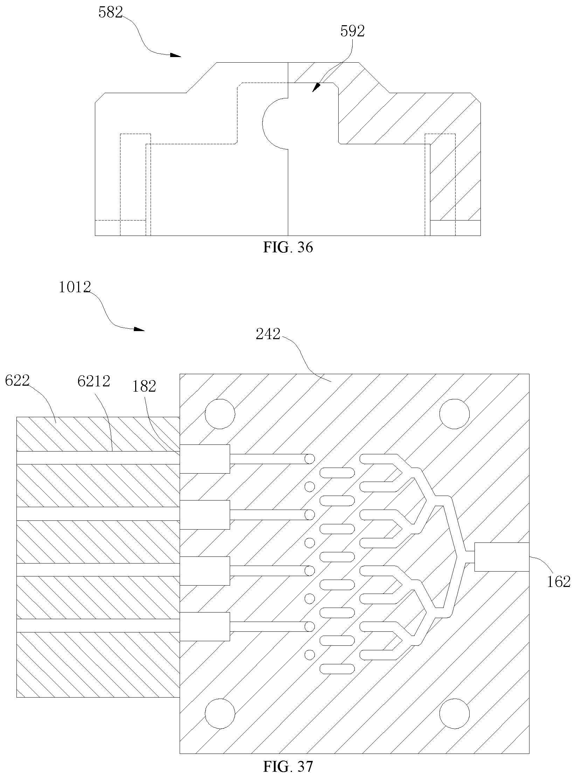

Specifically, the housing 582 may be connected to the manifold 242 through fasteners 552. In one example, during the assembly of the flow path selection valve 120, first the second valve element 282 is installed in the manifold 242, then the first valve element 262 is installed on the second valve element 282, after that, the first valve element 262 and the slide block 572 are limited, then the manifold 242 is covered with the housing 582 such that the slide block 572 and the first valve element 262 are both located in the housing 582, and finally the fasteners 552 pass through the manifold 242 and are tightly thread into the housing 582 such that the housing 582 is fixed to the manifold 242. Referring to FIGs. 35 and 36, in certain embodiments, the housing 582 is provided with a guide groove 592, and the slide block 572 includes a connection part 620 and a guide rail part 612 connected to the connection part 620. The connection part 620 is connected to the first valve element 262. The guide rail part 612 forms a fit with the guide groove 592 to direct the slide block 572 to drive the first valve element 262 to slide. As such, the fitting of the guide rail part 612 with the guide groove 592 may enable the slide block 572 to slide along a predetermined track, thereby driving the first valve element 262 to slide between the first position and the second position.

Specifically, the connection part 620 may be connected to the first valve element 262 through positioning pins 542. The profile of the connection part 620 may be adapted to the shape of the first valve element 262 to facilitate connection with the first valve element 262. The guide rail part 612 protrudes from the surface of the connection part 620. The cross-sectional area of the guide rail part 612 is smaller than the cross-sectional area of the connection part 620.

Referring to FIG. 37, a flow cell assembly 12012 according to an embodiment of the present application includes the flow path selection valve 120 according to any one of the embodiments described above and a flow cell 622. The flow cell 622 is connected to the flow path selection valve 120. The flow cell 622 includes a plurality of channels 6212 arranged in parallel. One end of the channel 6212 is in communication with the first port 182. As such, the flow path selection valve 120 and the flow cell 622 may form an integrated module, thereby facilitating the installation.

The flow cell 622 referred to herein provides a biochemical reaction site. The flow cell 622 may also be referred to as a chip, and the flow cell 622 is detachably connected to the flow path selection valve 120. Specifically, the flow cell 622 may be provided with a plug (manifold). The plug is formed with the channels 6212, and the plug may be inserted into the first port 182, thereby enabling direct connection of the channels 6212 of the flow cell 622 with the first port 182. Of course, in other embodiments, the flow cell 622 may be in communication with the flow path selection valve 120 through pipelines.

In some embodiments, the flow cell assembly 12012 includes two flow path selection valves 120 and one flow cell 622. One end of the channel 6212 is in communication with the first port 182 of one of the flow path selection valves 120, and the other end of the channel 6212 is in communication with the first port 182 of the other flow path selection valve 120.

In some embodiments, one end or the other end of the channel 6212 is connected to the first port 182 without pipelines. As such, the flow cell 622 is directly connected to the flow path selection valve 120, so that the pipelines for connection may be omitted, thereby reducing the risk of leakage.

Referring again to FIG. 26, a fluid path system 122 according to one embodiment of the present application includes the flow path selection valve 120 according to any one of the embodiments described above, a pump 142, a plurality of flow paths 12022, and a control circuit 632. The plurality of flow paths 12022 is in fluid connection with the flow cell 622 to support a target analyte when the flow cell 622 is installed in the fluid path system 122. The pump 142 is in fluid connection with the flow cell 622 when the flow cell 622 is installed in the fluid path system 122 and allows the liquid to flow through a flow path 12022 selected by the flow path selection valve 120 during an analysis operation. The control circuit 632 is operatively coupled to the flow path selection valve 120. The control circuit 632 has one or more processors and memories storing computer executable instructions that, when executed by the processors, control the processors to command the flow path selection valve 120 to select a designated flow path 12022.

In the fluid path system 122 according to an embodiment of the present application, the flow path selection valve 120 may be used to perform independent or parallel three-way control on the plurality of flow paths 12022, and it may be avoided that a plurality of three-way valves are simultaneously provided in the fluid path system 122, so that the fluid path system 122 has decreased cost, reduced size, less reagent consumption, improved reliability, and convenience in maintenance, repair and control. The fluid path system 122 described above is particularly suitable for systems requiring high precision in fluid control and delivery, such as sequencing systems.

Specifically, the pump 142 may provide power for the fluid path system 122, thereby allowing the liquid to flow. The pump 142 may be include a plurality of pumps, e.g., two pumps. One of the pumps 142 may be in communication with the common port 162, and the other pump 142 is in communication with the second port 220, to achieve flow splitting or flow confluence. The pump in communication with the common port 162 may provide power for the liquid to move from the common port 162 to the first port 182, and the pump in communication with the second port 220 may provide power for the liquid to move from the second port 220 to the first port 182.

In a specific embodiment, the number of the first ports 182 is 82, and the pump 142 includes 82 pumps with the number equal to the number of the first ports 182. For example, the pump is a negative pressure octuple pump, which is located downstream of the fluid path system 122, specifically downstream of the flow cell 622, and is capable of independently providing negative pressure to the fluid after passing through the flow path selection valve 120, so that the power of the fluid in flow channels of the plurality of flow paths 12022 can be independently controlled, thereby facilitating the fine control of the flow rate and/or flow velocity of the fluid in the flow channels.

Referring to FIG. 26, in an embodiment of the present application, the fluid path system 122 further includes a reservoir 642. The reservoir 642 may store multiple solutions, including a reaction solution, a buffer solution, a cleaning solution, and/or purified water, and other solutions, including reagents for different reactions or different steps of a reaction. The fluid path system 122 including the pump 142 may allow one or more solutions to flow toward the flow cell 622 sequentially or simultaneously.

In certain embodiments, the reservoir 642 includes a first reservoir 662 and a second reservoir 682. The first reservoir 662 carries a biological sample solution and the second reservoir 682 carries a reaction solution. In the case that the flow path selection valve 120 is at the first valve position, the flow path selection valve 120 allows the first reservoir 662 to be in communication with the flow cell 622, and the pump 142 induces the biological sample solution to flow toward the flow cell 622. In the case that the flow path selection valve 120 is at the second valve position, the flow path selection valve 120 allows the second reservoir 682 to be in communication with the flow cell 622, and the pump 142 induces the reaction solution to flow toward the flow cell 622 through the second flow channel.

That is, the flow path selection valve 120 may allow the reaction solution and the biological sample solution to enter the flow cell 622 independently and in a time-sharing manner to sequentially perform corresponding reactions, such as a sample loading reaction (immobilization and/or hybridization of nucleic acid) and a sequencing reaction.

Illustratively, the first reaction is, for example, an immobilization and/or hybridization reaction, that is, a nucleic acid under test is immobilized on or connected to a channel or a reaction region of the flow cell 622. The biological sample solution is a solution containing the nucleic acid under test, and the biological sample solution may sequentially pass through the second port 220 and the first port 182 and enter the flow cell 622 to perform the first reaction. In one example, the flow cell 622 is a sandwich-like structure with three layers (upper, middle and lower layers), or a structure with upper and lower layers. The upper layer (near the objective lens) is a transparent glass layer, the middle layer or the lower layer is a transparent glass layer or an opaque substrate layer, and the middle layer or the lower layer is provided with a plurality of channels arranged in an array. The channels can receive liquid to provide physical space for reaction, each channel is provided with an independent liquid inlet and an independent liquid outlet, the number of the channels is equal to that of the first ports 182, and a plurality of biological sample solutions may simultaneously and independently enter one channel of the flow cell 622 through one second port 220 and one first port 182. Thus, the flow path selection valve 120 or the fluid path system 122 containing the flow path selection valve 120 can perform loading and detection assays of a plurality of biological samples without the need to incorporate other means (e.g., labeling different biological samples). Specifically, the flow cell 622 herein is, for example, a solid substrate having a functional group on the surface and/or a solid substrate having a probe attached to the surface, and the solid substrate having a functional group on the surface and/or the solid substrate having a probe attached to the surface are also generally referred to as a chip or a microsphere. For example, the channel has a functional group on or a probe (oligonucleotide) attached to the upper surface (the lower surface of the upper glass layer) and/or the lower surface (the upper surface of the middle or lower layer structure), the functional group is capable of binding to the nucleic acid under test, and/or at least a portion of the probe is capable of complementarily pairing with the nucleic acid under test, to immobilize the nucleic acid under test or attach it to the surface of the solid substrate, and to perform a subsequent detection and analysis, such as a sequencing reaction, on the nucleic acid under test immobilized on or attached to the surface of the solid substrate.

The second reaction is, for example, a sequencing reaction, i.e., a nucleic acid sequence determination reaction, and more specifically, a sequencing-by-synthesis reaction using a reversible terminator based on chip detection, and accordingly, the reaction solution includes one or more reagents including a substrate (reversible terminator), a polymerase catalyst, a cleavage reagent (group cleavage reagent), an imaging reagent, a cleaning reagent, and the like. The reagents/reaction solutions may sequentially or simultaneously enter the flow cell 622 through the common port 162 and the first port 182 to perform the second reaction. Specifically, the corresponding reagents may sequentially or simultaneously pass through the flow path selection valve 120 and flow into a flow cell 622 to perform a plurality of reaction steps in the flow cell 622 and thereby achieve the sequencing reaction; the flow cell 622 is, for example, a solid substrate having a nucleic acid under test attached to the surface, and the solid substrate having a nucleic acid under test attached to the surface is, for example, a chip or a microsphere.

The first reaction and the second reaction described above may be performed in the flow cell 622 by controlling one flow path selection valve 120 to switch the flow channels, so that a nucleic acid sequence determination system (integrated system) including the first reaction and the second reaction may have a simpler structure. Thus, the first reaction and the second reaction do not need to be performed in different systems/apparatuses/devices, and the user operation is simpler and more convenient. Besides, the cost of constructing the integrated nucleic acid sequence determination system is far lower than the sum of the cost of constructing a nucleic acid sequence determination system (a sample loading device) for independently performing the first reaction and the cost of constructing a nucleic acid sequence determination system for independently performing the second reaction.

In an embodiment of the present application, the first reaction includes a reaction for attaching a biomolecule to the flow cell 622, including, for example, an immobilization, hybridization, or sampling reaction. The biomolecule includes DNA and/or RNA and the like, including ribonucleotides, deoxyribonucleotides and analogs thereof, including A, T, C, G, and U and analogs thereof. Among them, C represents cytosine or a cytosine analog, G represents guanine or a guanine analog, A represents adenine or an adenine analog, T represents thymine or a thymine analog, and U represents uracil or a uracil analog.

The second reaction includes a reaction for detecting the biomolecule attached to the flow cell 622. For example, the biomolecule is a nucleic acid, and the second reaction may be a sequence determination reaction, i.e., generally so-called sequencing, including the determination of the primary structure or sequence of DNA, RNA, or the like, including the determination of the order of nucleotides/bases of a given nucleic acid fragment. The second reaction may include one or more sub-reactions. In one example, a DNA is subjected to sequence determination. The second reaction is sequencing based on sequencing by synthesis or sequencing by ligation, specifically, for example, sequencing-by-synthesis based on chip detection using a modified nucleotide with a detectable label, such as dNTP or a dNTP analog with a detectable label. The sequencing includes a plurality of sub-reactions, including base extension, signal acquisition, and detection group cleavage, to determine the base type of a position on the nucleic acid sequence under test, and performing the plurality of sub-reactions once may be referred to as performing one repeated reaction or one cycle of reaction. A sequencing includes multiple repeated reactions or multiple cycles of reactions to determine the order of nucleotides/bases of at least a fragment of the nucleic acid molecule (template). The modified nucleotide carries a fluorescent molecule capable of being excited to fluoresce in a particular situation for detection by an optical system. When bound to a nucleic acid under test, the modified nucleotide label is capable of preventing a base/nucleotide from binding to the next position in the nucleic acid under test. For example, the modified nucleotide label is a dNTP with a chemically cleavable moiety at the 32' hydroxyl terminus or a dNTP with a molecular conformation that interferes with the binding of the next nucleotide to the nucleic acid under test. The dNTP or dNTP analog is four deoxyribonucleotides containing bases A, T/U, C, and G, respectively.

For sequencing by synthesis (SBS) or sequencing by ligation (SBL) based on chip detection, under the action of polymerase or ligase, the base extension includes enabling nucleotides (including modified nucleotides) to bind to a nucleic acid molecule under test based on the complementary base pairing rule in a flow cell 622 on which the nucleic acid molecule under test is immobilized, and acquiring corresponding reaction signals. The modified nucleotide may be a nucleotide with a detectable label that allows the modified nucleotide to be detected under certain circumstances, e.g., a nucleotide with a fluorescent molecular label that fluoresces when excited by a laser of a particular wavelength. Generally, for SBS, the modified nucleotide also has the function of inhibiting the binding of other nucleotides to the next position of the same nucleic acid molecule. For example, the modified nucleotide carries a blocking group that can prevent the binding of other nucleotides to the next position of the template, so that each extension is a single base extension, thus enabling the acquisition of the corresponding signal from each single base extension. The blocking group is, for example, a modified azido group (-N₃) attached to the 3' position of the glycosyl of the nucleotide.

In the detection and analysis of a biomolecule, generally, first the biomolecule is attached to the flow cell 622, and then the biomolecule attached to the flow cell 622 is detected. Specifically, in any one of the specific embodiments described above, the first reaction is performed before the second reaction, that is, the sequencing reaction is performed after the nucleic acid under test is attached to the flow cell 622. As such, using the fluid path system 122, multiple types of reactions, including sampling and sample detection, may be performed in one nucleic acid sequence determination system.

In certain specific embodiments, the first reaction is a sampling reaction, the second reaction is a sequencing reaction, and the first reaction is performed before the second reaction. The nucleic acid molecule under test is contained in a trace amount of biological sample solution, for example, a biological sample solution at the microliter level, for example, 220 microliters of a biological sample solution. Before the first reaction is performed, the fluid path system 122 is cleaned using a cleaning solution, for example, a solution that does not affect the subsequent reaction, and the fluid path system 122 is filled with the cleaning solution. Before the first reaction is started, air is injected to fill a section of the fluid path system to separate the biological sample solution which will flow into the fluid path system subsequently from the cleaning solution already in the fluid path system, thereby avoiding a situation where the trace amount of the biological sample is diffused and/or diluted and thereby the attachment of the nucleic acid molecule under test to the flow cell 622 and the subsequent detection of the nucleic acid molecule under test is affected. Moreover, the separation of the biological sample solution which will flow into the fluid path system subsequently from the cleaning solution already in the fluid path system facilitates the observation of the sample injection condition, the observation of whether the flow cell 622, such as a chip containing a plurality of channels, is normal or not, and whether the fluid path system 122 is normal or not, and the like.

The substrate may be any solid support useful for immobilizing nucleic acid sequences, such as nylon membranes, glass slides, plastics, silicon wafers, magnetic beads, and the like. Probes may be randomly distributed on the surface of the substrate. The probe may be a fragment of DNA and/or RNA sequence and the like, and it also is called a primer, a capture chain or an immobilization chain. The first reaction may allow the biomolecule to be attached to the probe, for example, based on the complementary base pairing rule, so that the biomolecule is attached to the flow cell 622.

Acquiring signals includes acquiring signals emitted by the modified nucleotide bound to the nucleic acid molecule. For example, a laser irradiation is performed on a particular region in the flow cell 622 after the base extension using an optical imaging assembly/system, a fluorescent molecular label in the particular region is excited to emit fluorescence, and then photographing/image acquisition is performed on the region to record the biochemical reaction signals as image information. Sequence determination involves converting the image information obtained from multiple cycles of /multiple repeated reactions into sequence information, i.e., determining the base type based on the image information, commonly referred to as base-calling.

Group cleavage includes removing the detectable label and/or blocking group on the modified nucleotide bound to the nucleic acid molecule after the base extension to allow other nucleotides (including modified nucleotides) to bind to the next position of the nucleic acid molecule for the next repeated reaction or the next cycle of reaction.

After the previous cycle or previous sub-reaction or previous step is completed and before the next cycle or subsequent sub-reaction or next step is started, a cleaning reagent may also be introduced to remove residual unreacted substances or substances that will interfere with the reaction or signal acquisition in the flow cell 622 or in the fluid path system 122.

In certain embodiments, the flow path selection valve 120 is provided upstream of the flow cell 622. As such, the flow path selection valve 120 may control the entering of the solution into the flow cell 622, and the fluid path system 122 may control the entering and exiting of multiple solutions to achieve multiple types of reactions only by using one power assembly (e.g., a pump) or only by providing power in one direction, which is beneficial to further reducing the size and improving the integration degree of the fluid path system 122, thereby being beneficial to industrialization.

In certain embodiments, the pump 142 is provided downstream of the flow cell 622 to provide negative pressure. As such, the pump 142 may generate negative pressure in elements such as the flow cell 622 and the flow path selection valve 120, thereby allowing the solution to enter the flow cell 622. In addition, the negative pressure generated by the pump 142 may remove the air in the flow cell 622, thereby preventing the air from affecting the normal reaction in the flow cell 622. The fluid path system 122 including the pump 142 located downstream of the flow cell 622 may provide a uniform power direction for the entering and exiting of the liquid in multiple types of reactions, and is particularly suitable for the fluid path system 122 including pressure-sensitive elements/assemblies. For example, the flow cell 622 is a chip containing thin glasses with a multi-layer sheet structure bonded by glue, the chip may further contain independent multiple reaction regions/channels, and the change of the power/pressure direction is easy to deform the chip or cause fluid leakage or cross-channel.

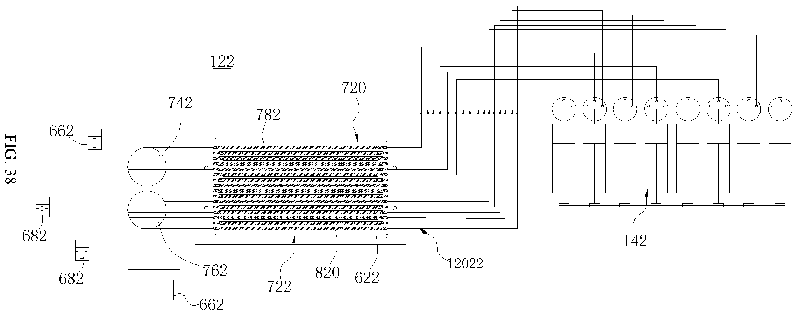

Referring to FIG. 38, in certain embodiments, the flow cell 622 includes a first flow cell 720 and a second flow cell 722. The flow path selection valve 120 includes a first flow path selection valve 742 and a second flow path selection valve 762. The first flow path selection valve 742 and the second flow path selection valve 762 are in communication with the first flow cell 720 and the second flow cell 722, respectively. As such, the first flow path selection valve 742 and the second flow path selection valve 762 may independently control the reactions in the first flow cell 720 and the second flow cell 722, which facilitates the alternation of different reactions or different steps/sub-reactions of a reaction in the first flow cell 720 and the second flow cell 722, thereby improving the reaction efficiency. In addition, the fluid path system 122 containing the flow path selection valve 120, in combination with the plurality of flow cells 622 or the flow cell 622 having a plurality of independent reaction regions, is beneficial to increasing the detection throughput and/or enabling the detection of a plurality of samples at a time.

Specifically, the first flow cell 720 and the second flow cell 722 may be of separate structures or may be of an integrated structure. As in an example shown in FIG. 38, the first flow cell 720 and the second flow cell 722 are of an integrated structure. The first flow cell 720 and/or the second flow cell 722 may include one or more reaction regions. Each reaction region is capable of enabling reactions, and the reaction regions can be intersecting, sequential, or separate regions.

As in an example of FIG. 38, the first flow cell 720 includes eight first reaction regions 782. The eight first reaction regions 782 correspond to the eight first ports 182 of the first flow path selection valve 742 in a one-to-one manner. The eight first reaction regions 782 are separately provided. Similarly, the second flow cell 722 includes eight second reaction regions 820. The eight second reaction regions 820 correspond to the eight second ports of the second flow path selection valve 762 in a one-to-one manner. The eight second reaction regions 820 are separately provided.

The first flow path selection valve 742 and the second flow path selection valve 762 may operate simultaneously, individually, or alternately, so that the reactions or steps in the first flow cell 720 and the second flow cell 722 may be performed alternately and in a time-sharing manner or simultaneously, which is beneficial to improving the reaction efficiency and reducing the consumption of the detection reagent and/or time.

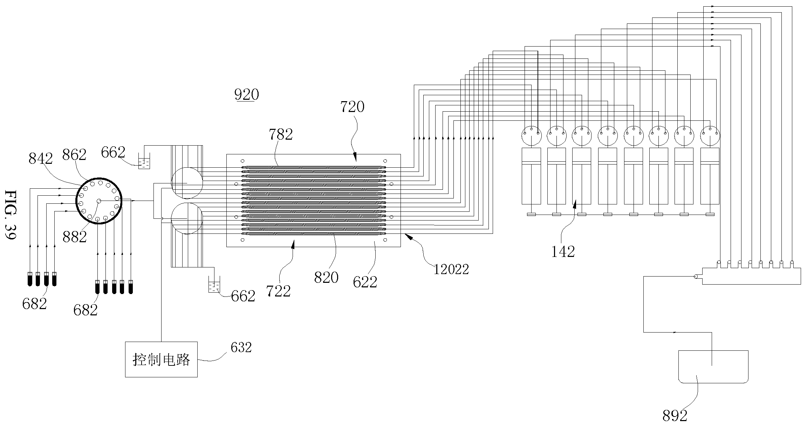

Referring to FIG. 39, in certain embodiments, the fluid path system 122 includes a reagent selection valve 842 and a flow cell 622. The reagent selection valve 842 selects a reagent from a plurality of reagents according to an analysis protocol. The flow path selection valve 120 is fluidly connected between the reagent selection valve 842 and the flow cell 622. The flow path selection valve 120 is configured to select a flow path in the flow cell 622 from a plurality of flow paths passing through the flow cell 622 according to the analysis protocol and to direct the selected reagent to pass through the flow cell 622. The pump 142 allows the selected reagent to flow through the selected flow path according to the analysis protocol.

Specifically, the reagent selection valve 842 is provided with a plurality of liquid inlets 862 and a liquid outlet 882. The liquid outlet 882 is selectively in communication with one of the liquid inlets 862, and the liquid inlet 862 is in communication with the reagent selection valve 822. As such, the plurality of liquid inlets 862 of the reagent selection valve 842 allow different liquids to enter the flow cell, thereby enabling multiple cycles of reactions/multiple repeated reactions.

As shown in FIG. 39, in certain embodiments, the fluid path system 122 includes a liquid trap 892. The liquid trap 892 collects the liquid flowing out of the flow cell 622. For example, the liquid trap 892 collects the liquid after the first reaction and the second reaction.

In one embodiment of the present application, the fluid path system 122 includes a flow path selection valve 120, a pump 142, a plurality of flow paths 12022, and a control circuit 632. The flow path selection valve 120 includes a manifold 242 and a first valve element 262. The manifold 242 is provided with a common port 162, a plurality of first ports 182, and a plurality of second ports 220. The first valve element 262 is provided with a communication groove 212. The first valve element 262 may rotate or slide relative to the manifold 242 to switch the flow path selection valve 120 between the first valve position and the second valve position. In the case that the flow path selection valve 120 is at the first valve position, the first port 182 is in communication with the second port 220 through the first communication groove 212. In the case that the flow path selection valve 120 is at the second valve position, the first port 182 is in communication with the common port 162 through the communication groove 212. The plurality of flow paths 12022 is in fluid connection with the flow cell 622 to support a target analyte when the flow cell 622 is installed in the fluid path system 122. The pump 142 is in fluid connection with the flow cell 622 when the flow cell 622 is installed in the fluid path system 122 and allows the liquid to flow through a flow path 12022 selected by the flow path selection valve 120 during an analysis operation. The control circuit 632 is operatively coupled to the flow path selection valve 120. The control circuit 632 has one or more processors and memories storing computer executable instructions that, when executed by the processors, control the processors to command the flow path selection valve 120 to select a designated flow path 12022.

Referring to FIG. 39, an embodiment of the present application further provides a sequencing system 920. The sequencing system 920 is, for example, a nucleic acid determination system, and the sequencing system 920 includes the fluid path system 122 according to any one of the embodiments described above.

In one example, the control circuit 632 is configured to control the flow path selection valve 120 to rotate to a first valve position to allow the first reservoir 662 to be in communication with the flow cell 622, the first reservoir 662 carrying a first reaction solution, and the first reaction solution containing a nucleic acid molecule; and configured to allow the first reaction solution to enter the flow cell 622 to perform a first reaction in the case that the flow path selection valve 120 is at the first valve position, the first reaction including attaching at least a portion of the nucleic acid molecule to the flow cell 622; and configured to rotate the flow path selection valve 120 to a second valve position to allow the second reservoir 682 to be in communication with the flow cell 622, the second reservoir 682 carrying a second reaction solution, and the second reaction solution containing components necessary for nucleic acid sequencing; and configured to allow the second reaction solution to enter the flow cell 622 through a second flow channel to perform a second reaction in the case that the flow path selection valve 120 is at the second valve position, the second reaction including allowing the nucleic acid molecule in the flow cell 622 after the first reaction is performed to interact with the second reaction solution to perform a polymerization reaction and detecting a signal from the reaction, so as to achieve sequence determination of the nucleic acid molecule. The sequencing system 920 described herein is, for example, a machine such as a sequencer or a sequencing platform.

In one example, the first reservoir 662 carries a biological sample solution, and the biological sample solution contains a nucleic acid molecule. The second reservoir 682 carries a reaction solution, and the reaction solution contains components necessary for a polymerization reaction. The control circuit 632 is configured to control the flow path selection valve 120 to allow the biological sample solution in the first reservoir 662 to enter the flow cell 622 to perform a first reaction, the first reaction including allowing at least a portion of the nucleic acid molecule to be attached to the flow cell 622, and the control circuit 632 is configured to control the flow path selection valve 120 to allow the reaction solution in the second reservoir 682 to enter the flow cell 622 to perform a second reaction after the first reaction is performed, the second reaction including allowing the nucleic acid molecule in the flow cell 622 to interact with the reaction solution to perform a polymerization reaction.

The sequencing system 920 operates according to commands that implement a prescribed protocol for testing, validation, analysis (e.g., including sequencing), and the like. The prescribed protocol will be established in advance and includes a series of events or operations for activities such as aspirating reagents, aspirating air, aspirating other fluids, and ejecting such reagents, air and fluids. The protocol will allow for coordination of such fluidic operations with other operations of the instrument, such as reactions occurring in flow cell 622 and imaging of flow cell 622 and its site.

The present application further provides a system including a sequencing system 920 according to any one of the embodiments described above.

The present application further provides a method for controlling a system to achieve sequencing. The system may be the above fluid path system 122, for example, the system includes a plurality of flow paths 12022, a flow cell 622 connected to the plurality of flow paths 12022, a flow path selection valve 120, a pump 142, a first reservoir 662, and a second reservoir 682. The flow path selection valve 120 includes a manifold 242 and a first valve element 262. The manifold 242 is provided with a common port 162, a plurality of first ports 182, and a plurality of second ports 220. The first valve element 262 is provided with a communication groove 212. The first valve element 262 may rotate or slide relative to the manifold 242, so that the communication groove 212 selectively allows the common port 162 to be in communication with the first port 182, or allows the first port 182 to be in communication with the second port 220, thereby selecting different flow paths. The first reservoir 662 is connected to the second port 182. The second reservoir 682 is connected to the common port 162. The first reservoir 662 carries a first reaction solution, and the first reaction solution contains a nucleic acid molecule. The second reservoir 682 carries a second reaction solution containing components necessary for nucleic acid sequencing. The pump 142 is configured to allow the liquid to flow through the flow path selected by the flow path selection valve 120.

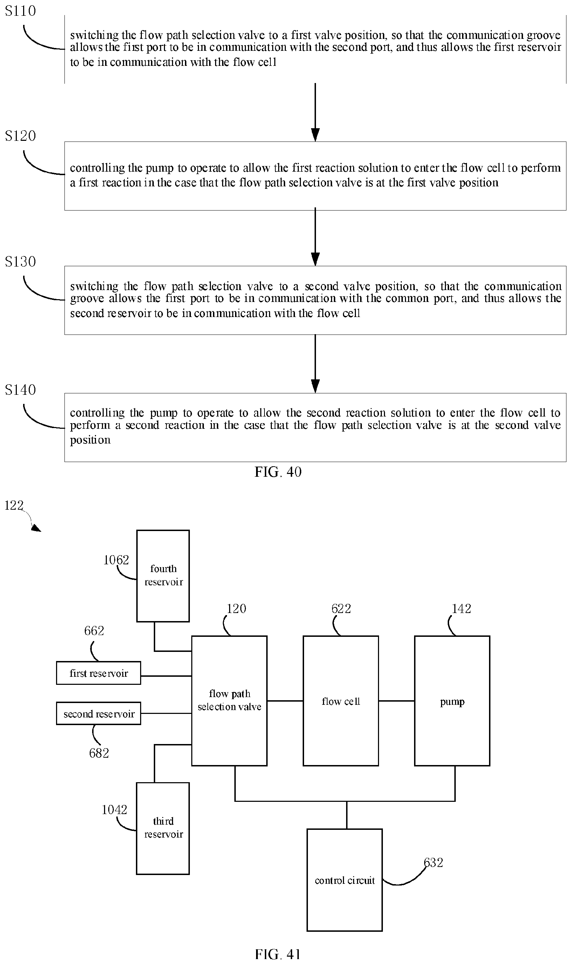

Referring to FIG. 40, the method includes: S 1120, switching the flow path selection valve 120 to the first valve position, so that the communication groove 212 allows the first port 182 to be in communication with the second port 220, and thus allows the first reservoir 662 to be in communication with the flow cell 622; S 1220, controlling the pump 142 to operate to allow the first reaction solution to enter the flow cell 622 to perform a first reaction in the case that the flow path selection valve 120 is at the first valve position, the first reaction including attaching at least a portion of the nucleic acid molecule to the flow cell 622; S 1320, switching the flow path selection valve 120 to the second valve position, so that the communication groove 212 allows the first port 182 to be in communication with the common port 162, and thus allows the second reservoir 682 to be in communication with the flow cell 622; S 1420, controlling the pump 142 to operate to allow the second reaction solution to enter the flow cell 622 to perform a second reaction in the case that the flow path selection valve 120 is at the second valve position, the second reaction including allowing the nucleic acid molecule in the flow cell 622 after the first reaction is performed to interact with the second reaction solution to perform a polymerization reaction and detecting a signal from the reaction, so as to achieve sequence determination of the nucleic acid molecule. The method achieves the first reaction and the second reaction by allowing one flow path selection valve 120 to be at different valve positions to achieve the switching of different flow channels/reagents, and is particularly suitable for the operation control of a highly integrated system or apparatus.

The current next-generation high-throughput sequencing platforms or single-molecule sequencing platforms generally require processing of the sample under test before on-line sequencing. For example, in order to adapt to a designated sequencing platform, it is necessary to process the sample under test to convert it into a library adapted to the sequencing platform, and to load the library into a designated region, such as a flow cell 622, so as to place the flow cell 622 containing the sample under test into a sequencer for automated sequencing. Currently, in commercially available sequencing platforms, the processing of the sample under test before loading on a machine is generally separated from the on-line sequencing. For example, the sample processing/library preparation is performed manually in a reagent tube, or the processing and loading of the sample under test is performed on a sample processing apparatus. The method can achieve sample processing and sequencing before loading on a machine, and achieve the switching and the access control of different flow channels for a plurality of reagents by allowing the flow path selection valve 120 to be at different valve positions, and is particularly suitable for an integrated sequencing platform integrating the functions of sample processing before loading on a machine and sequencing.

Referring to FIG. 41, in certain embodiments, the system further includes a third reservoir 12042. The third reservoir 12042 is connected to the common port 162, the third reservoir 12042 carries a third reaction solution, and the third reaction solution includes components necessary for amplification. The method further includes performing the following step after performing the step S 1220 and before performing the step S 1420 (after performing the first reaction and before performing the second reaction): controlling the pump 142 to operate to allow the third reaction solution in the third reservoir 12042 to enter the flow cell 622 to perform a third reaction, the third reaction including allowing the nucleic acid molecule in the flow cell 622 after the first reaction is performed to interact with the third reaction solution to achieve amplification of the nucleic acid molecule.

Referring to FIG. 41, in certain embodiments, the system further includes a fourth reservoir 12062. The fourth reservoir 12062 is connected to the common port 162, and the fourth reservoir carries a cleaning solution. The method further includes performing the following step before performing the step S 1220 (before performing the first reaction): controlling the pump 142 to operate to allow the cleaning solution in the fourth reservoir 12062 to enter the flow cell 622 in the case that the flow path selection valve 120 is at the first valve position. As such, the cleaning solution may rinse the flow cell 622, thereby preventing the first reaction solution from being contaminated by the residual substance of last reaction and/or reducing unnecessary consumption of the first reaction solution, e.g., a trace amount of biological sample solution (e.g., filling the pipeline of the fluid path system 122).

In certain embodiments, the method further includes performing the following step before performing the step S 1220 (before performing the first reaction): controlling the pump 142 to operate to introduce air into the flow cell 622 in the case that the flow path selection valve 120 is at the first valve position. As such, before the first reaction is started, air is injected to fill a section of the fluid path system to separate the first reaction solution which will flow into the fluid path system subsequently from the cleaning solution already in the fluid path system, thereby avoiding a situation where the trace amount of biological sample is diffused and/or diluted and thereby the attachment of the nucleic acid molecule under test to the flow cell 622 and the subsequent detection of the nucleic acid molecule under test is affected.

In certain embodiments, the method further includes performing the following step before performing the step S1220 and/or before performing the step S1420 (before performing the first reaction and/or before performing the second reaction): controlling the pump 142 to operate to allow the cleaning solution in the fourth reservoir 12062 to enter the flow cell 622. As such, the cleaning solution may clean the flow cell 622, so that the subsequent first reaction solution and/or second reaction solution may be prevented from being affected by the residue of the last reaction or the last step.

In certain embodiments, the flow cell 622 has a solid carrier surface on which a first sequencing primer is immobilized. At least one end of the nucleic acid molecule includes at least a portion of a sequence capable of complementarily pairing with at least a portion of the first sequencing primer, and the first reaction includes complementary pairing of at least a portion of the nucleic acid molecule with the first sequencing primer for attachment to the flow cell 622. The "first sequencing primer" is a fragment of oligonucleotide (a short nucleic acid sequence with known sequence), and such a known sequence immobilized on the surface of the chip is also commonly referred to as a "probe".

In certain embodiments, the second reaction solution includes a first nucleotide, a first polymerase, and a cleavage reagent. The step S 1420 includes: (a) controlling the pump 142 to operate to allow the first nucleotide and the first polymerase to enter the flow cell 622, and subjecting the flow cell 622 to conditions suitable for a polymerization reaction to bind the first nucleotide to a nucleic acid molecule by extending a first sequencing primer, the first nucleotide containing a base, a sugar unit, a cleavable blocking group, and a detectable label; (b) exciting the detectable label and acquiring a signal from the detectable label; (c) controlling the pump 142 to operate to allow the cleavage reagent to enter the flow cell 622, so as to remove the cleavable blocking group and the detectable label of the first nucleotide; and (d) repeating (a)-(c) at least once. Specifically, by "subjected to conditions suitable for a polymerization reaction", temperature conditions are generally involved in addition to the components/reagents required for the polymerization reaction (e.g., a polymerase, a reaction substrate, i.e., a nucleotide, and/or a sequencing primer). For example, the nucleic acid sequence determination system 920 may further include a temperature control system to control the temperature of the flow cell 622/reaction chamber to achieve "conditions suitable for a polymerization reaction". The detectable label is, for example, an optically detectable label, such as a fluorescent molecule. The cleavable blocking group may prevent/inhibit the binding of the other nucleotides (first nucleotide) in the reaction system to the next position of the nucleic acid molecule under test, either in a physically blocking manner, e.g., by attaching an azido group (-N₃) to the 3' position of the glycosyl, or in a non-physical blocking (virtual blocking) manner, e.g., by forming a spatial conformation that can prevent the extension from proceeding in the extension solution system.

In certain embodiments, the second reaction solution further includes a second nucleotide. The step S1420 further includes performing the following step after (a): controlling the pump 142 to operate to allow the second nucleotide and the first polymerase to enter the flow cell, and subjecting the flow cell to conditions suitable for a polymerization reaction to allow the second nucleotide to bind to the nucleic acid molecule by proceeding to extend a product after (a), the second nucleotide containing a base, a sugar unit, and a cleavable blocking group. Compared with the first nucleotide, the second nucleotide is a reversible terminator without a detectable label, and the reaction efficiency of the second nucleotide is generally higher than that of the first nucleotide for the same polymerase. This step facilitates the synchronization of the reactions of multiple nucleic acid molecules in a cluster, that is, it can eliminate or reduce the prephasing or phasing nucleic acid molecules in a cluster to some extent, and thus facilitates the sequencing reaction.

Referring to FIG. 33, in certain embodiments, the second reaction solution includes a third nucleotide, a fourth nucleotide, a second polymerase, a third polymerase, a cleavage reagent, and a second sequencing primer, and at least one end of the nucleic acid molecule includes at least a portion of a sequence capable of complementarily pairing with at least a portion of the second sequencing primer.

Controlling the pump 142 to operate to allow the second reaction solution to enter the flow cell 622 through the second flow channel to perform the second reaction includes: (i) controlling the pump 142 to operate to allow the third nucleotide and the second polymerase to enter the flow cell 622, and subjecting the flow cell 622 to conditions suitable for a polymerization reaction to bind the third nucleotide to the nucleic acid molecule by extending the first sequencing primer to obtain a nascent strand, the third nucleotide being a nucleotide that carries neither a cleavable blocking group nor a detectable label; (ii) controlling the pump 142 to operate to allow the fourth nucleotide, the third polymerase, and the second sequencing primer to enter the flow cell 622, and subjecting the flow cell 622 to conditions suitable for a polymerization reaction to bind the second sequencing primer to the nascent strand and bind the fourth nucleotide to the nascent strand by extending the second sequencing primer, the fourth nucleotide containing a base, a sugar unit, a cleavable blocking group and a detectable label; (iii) exciting the detectable label and acquiring a signal from the detectable label; (iv) controlling the pump 142 to operate to allow the cleavage reagent to enter the flow cell 622 to remove the cleavable blocking group and the detectable label of the fourth nucleotide; and (v) repeating (ii)-(iv) at least once. As such, the second reaction can be performed through the steps (ii)-(v) to achieve determination of the nucleic acid sequence.

The third nucleotide may be, for example, a natural nucleotide. The fourth nucleotide may be identical to the first nucleotide. The first to third polymerases may be the same or different. For example, they are different types of DNA polymerases or different mutants of the same type of DNA polymerase, and they are each capable of independently binding to a designated nucleotide and thereby effectively catalyzing the performance of a designated extension/polymerization reaction.

Compared with the sequencing method of the previous embodiment, the method can determine the sequence of the other end of the nucleic acid molecule under test by synthesizing the complementary strand of the nucleic acid molecule under test, thus achieving sequencing of the other end of the nucleic acid molecule under test.

Similarly, in certain embodiments, the second reaction solution further includes a fifth nucleotide, and the method further includes after (ii), controlling the pump 142 to operate to allow the fifth nucleotide and the third polymerase to enter the flow cell, and subjecting the flow cell to conditions suitable for a polymerization reaction to bind the fifth nucleotide to the nascent strand by proceeding to extend a product after (ii), the fifth nucleotide containing a base, a sugar unit, and a cleavable blocking group. Similar to, for example, the second nucleotide, the fifth nucleotide is a reversible terminator without a detectable label. This step facilitates the synchronization of the reactions of multiple nucleic acid molecules in a cluster, that is, it can eliminate or reduce the prephasing or phasing nucleic acid molecules in a cluster to some extent, and thus facilitates the sequencing reaction and the obtaining of longer reads.

It should be noted that the explanations of the technical features such as the structure, connection relationship and operation control of the flow path selection valve 120 and/or the fluid path system 122 in the embodiments described above are also applicable to the method for controlling a system to achieve sequencing according to any one of the embodiments, and how to implement the method for controlling a system to achieve sequencing according to any one of the embodiments by using the flow path selection valve 120 and the related elements/structural assemblies is not shown here. Those skilled in the art can understand how to utilize and control the flow path selection valve 120 and/or the fluid path system 122 and/or the sequencing system 920 in the embodiments described above to implement the corresponding sequencing method by way of the above description of the structure, connection relationship, function and operation manner of the flow path selection valve 120 and/or the fluid path system 122 in the above embodiments and the current illustration of the sequencing method. The present application further provides a method, which includes a method for controlling a system to achieve sequencing according to any one of the embodiments described above.

In the description of this specification, the description of the terms "certain embodiments", "one embodiment", "some embodiments", "schematic embodiments", "examples", "specific examples" or "some examples" means that the specific features, structures, materials, or characteristics described in reference to the embodiments or examples are included in at least one embodiment or example of the present application. In this specification, the schematic description of the terms described above does not necessarily refer to the same embodiment or example. Moreover, the specific features, structures, materials, or characteristics described may be combined in any embodiment or example in any appropriate manner.

Logic and/or steps shown in the flowcharts or described herein in other manners, for example, may be considered as a program list of executable instructions that are used to implement logical functions, and may be specifically implemented on any computer-readable storage medium, for use by an instruction execution system, device, or apparatus (for example, a computer-based system, a system including a processor, or another system that can fetch instructions from the instruction execution system, device, or apparatus and execute the instructions), or for use in combination with the instruction execution system, device or apparatus. As used herein, the "computer-readable storage medium" may be any device that may include, store, communicate, propagate, or transmit a program for use by an instruction execution system, device, or apparatus, or for use in combination with the instruction execution system, device, or apparatus.

In addition, the functional units in the embodiments of the present application may be integrated in one processing module, or the units may physically exist alone, or two or more than two units may be integrated in one module. The integrated module described may be implemented in the form of hardware or in the form of a software functional module. The integrated module may also be stored in a computer-readable storage medium if it is implemented in the form of a software functional module and is sold or used as standalone products.

Although the embodiments of the present application have been shown and described above, it will be understood that the embodiments described above are exemplary and are not to be construed as limiting the present application, and that those of ordinary skill in the art may make changes, modifications, replacements, and variations to such embodiments without departing from the scope of the present application.

## Claims

1. A fluid path system, comprising:
a fluid network comprising a reservoir, a first flow channel, a second flow channel, and a reaction device, wherein the first flow channel and the second flow channel each independently allow the reservoir to be in fluid communication with the reaction device;
a pump assembly in communication with the fluid network; and
a flow path selection valve configured to rotate between a first valve position and a second valve position, wherein in the case that the flow path selection valve is at the first valve position, the pump assembly induces liquid in the reservoir to flow toward the reaction device through the first flow channel, and in the case that the flow path selection valve is at the second valve position, the pump assembly induces liquid in the reservoir to flow toward the reaction device through the second flow channel.

2. The fluid path system according to claim 1, wherein the flow path selection valve is provided with a common port, a plurality of first ports, a plurality of second ports, and a plurality of communication grooves, wherein the communication groove selectively allows the common port to be in communication with the first port, or allows the first port to be in communication with the second port; in the case that the flow path selection valve is at the first valve position, the first port is in communication with the second port through the communication groove to form the first flow channel; and
in the case that the flow path selection valve is at the second valve position, the first port is in communication with the common port through the communication groove to form the second flow channel.

3. The fluid path system according to claim 2, wherein the flow path selection valve comprises a stator and a rotor provided opposite to the stator, wherein the stator is provided with the common port, the plurality of first ports, and the plurality of second ports, and the rotor is provided with at least a portion of the plurality of communication grooves.

4. The fluid path system according to claim 2, wherein the flow path selection valve comprises a stator and a rotor provided opposite to the stator, wherein the rotor is provided with the common port, the plurality of first ports, and the plurality of second ports, and the stator is provided with at least a portion of the plurality of communication grooves.

5. The fluid path system according to claim 3 or 4, wherein the communication groove comprises a first communication groove and a second communication groove capable of being in communication with each other, and the first communication groove and the second communication groove are provided on the rotor and the stator, respectively.

6. The fluid path system according to claim 5, wherein one communication groove is composed of one first communication groove and one second communication groove, wherein one first communication groove has two ends, one end of each second communication groove is in communication with one first port, and the other end is in selective communication with one second port; and one second communication groove has two ends, one end of each second communication groove is in communication with the common port, and the other end is in selective communication with one first port.

7. The fluid path system according to claim 5 or 6, wherein the stator comprises a stator end face, and the rotor comprises a rotor end face, wherein the stator end face and the rotor end face are abutted together, the first communication groove is formed in the rotor end face, and the second communication groove is formed in the stator end face.

8. The fluid path system according to any one of claims 2-7, wherein the plurality of first ports and the plurality of second ports are all provided around the common port.

9. The fluid path system according to any one of claims 2-7, wherein the flow path selection valve has a central axis, the plurality of first ports are arranged at intervals on a circular plane having the central axis as an axis, and/or
the plurality of second ports are arranged at intervals on a circular plane having the central axis as an axis.

10. The fluid path system according to claim 9, wherein the plurality of first ports and the plurality of second ports are located on the same circular plane.

11. The fluid path system according to any one of claims 3-10, wherein the plurality of first ports are arranged at intervals along a circumferential direction of the stator or the rotor; and/or
the plurality of second ports are arranged at intervals along a circumferential direction of the stator or the rotor.

12. The fluid path system according to claim 11, wherein the plurality of first ports and the plurality of second ports are located on the same circumference.

13. The fluid path system according to any one of claims 3-12, wherein the flow path selection valve comprises a valve body, and the stator and the rotor are both received in the valve body.

14. The fluid path system according to claim 13, wherein the flow path selection valve comprises a valve head covering the stator and provided on the valve body, and the valve head has a first interface, a second interface, and a third interface, the first interface being in communication with the common port, the second interface being in communication with the first port, and the third interface being in communication with the second port.

15. The fluid path system according to claim 14, wherein the flow path selection valve comprises a positioning structure configured to position the valve head and the stator.

16. The fluid path system according to claim 15, wherein the positioning structure comprises a positioning pin inserted in the valve head and the stator.

17. The fluid path system according to claim 13, wherein the flow path selection valve further comprises a drive component connected to the rotor, and the drive component is provided on the valve body and is configured to drive the rotor to rotate.

18. The fluid path system according to any one of claims 2-17, wherein the common port, the plurality of first ports, and the plurality of second ports are separated from one another when the flow path selection valve is at a third valve position.

19. The fluid path system according to any one of claims 1-18, wherein the reservoir comprises a first reservoir and a second reservoir, the first reservoir carrying a biological sample solution, and the second reservoir carrying a reaction solution;
in the case that the flow path selection valve is at the first valve position, the flow path selection valve allows the first reservoir to be in communication with the reaction device, and the pump assembly induces the biological sample solution to flow toward the reaction device through the first flow channel; and
in the case that the flow path selection valve is at the second valve position, the flow path selection valve allows the second reservoir to be in communication with the reaction device, and the pump assembly induces the reaction solution to flow toward the reaction device through the second flow channel.

20. The fluid path system according to any one of claims 1-19, wherein the flow path selection valve is provided upstream of the reaction device.

21. The fluid path system according to any one of claims 1-20, wherein the pump assembly is provided downstream of the reaction device.

22. The fluid path system according to any one of claims 1-21, wherein the reaction device comprises a first reaction device and a second reaction device, and the flow path selection valve comprises a first flow path selection valve and a second flow path selection valve, the first flow path selection valve and the second flow path selection valve being in communication with the first reaction device and the second reaction device, respectively.

23. The fluid path system according to claim 22, further comprising a three-way valve provided between the reservoir and the flow path selection valve, wherein the three-way valve is configured to switch between a first position and a second position, the three-way valve allows the reservoir to be in communication with the first flow path selection valve in the case that the three-way valve is at the first position, and the three-way valve allows the reservoir to be in communication with the second flow path selection valve in the case that the three-way valve is at the second position.

24. The fluid path system according to claim 23, wherein the three-way valve allows the reservoir to be in communication with a common port of the first flow path selection valve in the case that the three-way valve is at the first position, and the three-way valve allows the reservoir to be in communication with a common port of the second flow path selection valve in the case that the three-way valve is at the second position.

25. A detection system, comprising the fluid path system according to any one of claims 1-24, a detection assembly, and a controller, wherein
the detection assembly is configured to detect a signal from the reaction device during a designated reaction, and
the controller is configured to control the pump assembly and the flow path selection valve to allow liquid in the reservoir to flow toward the reaction device through the first flow channel, to control the pump assembly and the flow path selection valve to allow liquid in the reservoir to flow toward the reaction device through the second flow channel, and to control the detection assembly to detect a signal from the reaction device when the liquid in the reservoir flows through the reaction device or after the liquid in the reservoir flows through the reaction device.

26. The detection system according to claim 25, wherein the reservoir comprises a first reservoir and a second reservoir, the first reservoir carrying a biological sample solution comprising a nucleic acid molecule, and the second reservoir carrying a reaction solution comprising components necessary for a polymerization reaction;
the controller is configured to control the pump assembly and the flow path selection valve to allow the biological sample solution in the first reservoir to enter the reaction device to perform a first reaction, the first reaction comprising allowing at least a portion of the nucleic acid molecule to be attached to the reaction device, and
the controller is configured to control the pump assembly and the flow path selection valve to allow the reaction solution in the second reservoir to enter the reaction device to perform a second reaction after the first reaction is performed, and to control the detection assembly to acquire a signal produced by the second reaction, the second reaction comprising allowing the nucleic acid molecule in the reaction device to interact with the reaction solution to perform a polymerization reaction.

27. The detection system according to claim 25 or 26, wherein the second reaction produces an optical signal, and the detection assembly comprises an imaging detector that detects the optical signal.

28. Use of the fluid path system according to any one of claims 1-24 or the detection system according to any one of claims 25-27 in nucleic acid sequence determination.

29. A flow path selection valve, wherein the flow path selection valve is configured to switch between a first valve position and a second valve position, and the flow path selection valve is provided with a common port, a plurality of first ports, a plurality of second ports, and a plurality of communication grooves, wherein the communication groove selectively allows the common port to be in communication with the first port, or allows the first port to be in communication with the second port;
in the case that the flow path selection valve is at the first valve position, the first port is in communication with the second port through the communication groove; and
in the case that the flow path selection valve is at the second valve position, the first port is in communication with the common port through the communication groove.

30. The flow path selection valve according to claim 29, wherein the flow path selection valve comprises:
a manifold provided with the common port, the plurality of first ports, and the plurality of second ports; and
a first valve element provided with the communication groove, the first valve element being rotatable or slidable relative to the manifold to switch the flow path selection valve between the first valve position and the second valve position.

31. The flow path selection valve according to claim 30, comprising a second valve element provided on the manifold, wherein the first valve element is provided on the second valve element, and the second valve element is provided with a first channel, a second channel, and a third channel, the first channel being in communication with the first port, the second channel being in communication with the second port, and the third channel being in communication with the common port;
in the case that the flow path selection valve is at the first valve position, the communication groove allows the first channel to be in communication with the second channel to achieve communication between the first port and the second port; and
in the case that the flow path selection valve is at the second valve position, the communication groove allows the first channel to be in communication with the third channel to achieve communication between the first port and the common port.

32. The flow path selection valve according to claim 31, wherein the manifold is provided with a cavity, and the second valve element is at least partially received in the cavity.

33. The flow path selection valve according to claim 32, wherein a bottom surface of the cavity is provided with a first connection port, a second connection port, and a third connection port, the first connection port allowing the first port to be in communication with the first channel, the second connection port allowing the second port to be in communication with the second channel, and the third connection port allowing the common port to be in communication with the third channel.

34. The flow path selection valve according to any one of claims 30-33, wherein at least two of the first port, the second port, and the common port are located on different sides of the manifold.

35. The flow path selection valve according to any one of claims 30-34, wherein the communication groove comprises first communication grooves and second communication grooves provided at intervals, wherein in the case that the flow path selection valve is at the first valve position, the first port is in communication with the second port through the first communication groove, and in the case that the flow path selection valve is at the second valve position, the first port is in communication with the common port through the second communication groove.

36. The flow path selection valve according to any one of claims 30-35, comprising a drive component configured to drive the first valve element to rotate or slide.

37. The flow path selection valve according to claim 36, comprising a slide block connected to the first valve element, wherein the slide block is connected to the drive component, and the drive component drives the first valve element to slide through the slide block; optionally, the drive component provides an electromagnetic drive.

38. The flow path selection valve according to claim 37, comprising a housing detachably connected to the manifold, wherein the first valve element and the slide block are received in the housing.

39. The flow path selection valve according to claim 38, wherein the housing is provided with a guide groove, and the slide block comprises a connection part and a guide rail part connected to the connection part, the connection part being connected to the first valve element, and the guide rail part forming a fit with the guide groove to direct the slide block to drive the first valve element to slide.

40. The flow path selection valve according to claim 29, wherein the flow path selection valve is configured to rotate between the first valve position and the second valve position, wherein in the case that the flow path selection valve is at the first valve position, the first port is in communication with the second port through the communication groove to form a first flow channel; and
in the case that the flow path selection valve is at the second valve position, the first port is in communication with the common port through the communication groove to form a second flow channel.

41. The flow path selection valve according to claim 40, comprising a stator and a rotor provided opposite to the stator, wherein the stator is provided with the common port, the plurality of first ports, and the plurality of second ports, and the rotor is provided with at least a portion of the plurality of communication grooves.

42. The flow path selection valve according to claim 40, comprising a stator and a rotor provided opposite to the stator, wherein the rotor is provided with the common port, the plurality of first ports, and the plurality of second ports, and the stator is provided with at least a portion of the plurality of communication grooves.

43. The flow path selection valve according to claim 41 or 42, wherein the communication groove comprises a first communication groove and a second communication groove capable of being in communication with each other, and the first communication groove and the second communication groove are provided on the rotor and the stator, respectively.

44. The flow path selection valve according to claim 43, wherein one communication groove is composed of one first communication groove and one second communication groove, wherein one first communication groove has two ends, one end of each second communication groove is in communication with one first port, and the other end is in selective communication with one second port; and one second communication groove has two ends, one end of each second communication groove is in communication with the common port, and the other end is in selective communication with one first port.

45. The flow path selection valve according to claim 43 or 44, wherein the stator comprises a stator end face, and the rotor comprises a rotor end face, wherein the stator end face and the rotor end face are abutted together, the first communication groove is formed in the rotor end face, and the second communication groove is formed in the stator end face.

46. The flow path selection valve according to any one of claims 40-45, wherein the plurality of first ports and the plurality of second ports are all provided around the common port.

47. The flow path selection valve according to any one of claims 42-46, wherein the flow path selection valve has a central axis, the plurality of first ports are arranged at intervals on a circular plane having the central axis as an axis, and/or
the plurality of second ports are arranged at intervals on a circular plane having the central axis as an axis.

48. The flow path selection valve according to claim 47, wherein the plurality of first ports and the plurality of second ports are located on the same circular plane.

49. The flow path selection valve according to any one of claims 41-46, wherein the plurality of first ports are arranged at intervals along a circumferential direction of the stator or the rotor; and/or
the plurality of second ports are arranged at intervals along a circumferential direction of the stator or the rotor.

50. The flow path selection valve according to claim 49, wherein the plurality of first ports and the plurality of second ports are located on the same circumference.

51. The flow path selection valve according to any one of claims 41-50, comprising a valve body, wherein the stator and the rotor are both received in the valve body.

52. The flow path selection valve according to claim 51, comprising a valve head covering the stator and provided on the valve body, wherein the valve head has a first interface, a second interface, and a third interface, the first interface being in communication with the common port, the second interface being in communication with the first port, and the third interface being in communication with the second port.

53. The flow path selection valve according to claim 52, comprising a positioning structure configured to position the valve head and the stator.

54. The flow path selection valve according to claim 53, wherein the positioning structure comprises a positioning pin inserted in the valve head and the stator.

55. The flow path selection valve according to claim 51, further comprising a drive component connected to the rotor, wherein the drive component is provided on the valve body and is configured to drive the rotor to rotate.

56. The flow path selection valve according to any one of claims 40-55, wherein the common port, the plurality of first ports, and the plurality of second ports are separated from one another when the flow path selection valve is at a third valve position.

57. A flow cell assembly, comprising:
the flow path selection valve according to any one of claims 29-56; and
a flow cell connected to the flow path selection valve, wherein the flow cell comprises a plurality of channels arranged in parallel, and one end of the channel is in communication with the first port.

58. The flow cell assembly according to claim 57, comprising two flow path selection valves and one flow cell, wherein one end of the channel of the flow cell is in communication with the first port of one of the flow path selection valves, and the other end of the channel of the flow cell is in communication with the first port of the other flow path selection valve.

59. The flow cell assembly according to claim 57 or 58, wherein one end or the other end of the channel is connected to the first port without tubes.

60. A fluid path system, comprising:
a plurality of flow paths being in fluid connection with a flow cell to support a target analyte when the flow cell is installed in the fluid path system;
the flow path selection valve according to any one of claims 29-56 connected to the flow paths, the flow path selection valve selecting between the flow paths;
a pump being in fluid connection with the flow cell when the flow cell is installed in the fluid path system and allowing liquid to flow through a flow path selected by the flow path selection valve during an analysis operation; and
a control circuit operatively coupled to the flow path selection valve, wherein the control circuit has one or more processors and memories storing computer executable instructions that, when executed by the processors, control the processors to command the flow path selection valve to select a designated flow path.

61. The fluid path system according to claim 60, further comprising:
a reagent selection valve selecting a reagent from a plurality of reagents according to an analysis protocol; and
a flow cell carrying a target analyte; wherein
the flow path selection valve is fluidly connected between the reagent selection valve and the flow cell, the flow path selection valve selects a flow path for flowing through the flow cell from a plurality of flow paths passing through the flow cell according to the analysis protocol and directs the selected reagent to pass through the flow cell, and the pump allows the selected reagent to flow through the selected flow path according to the analysis protocol.

62. The fluid path system according to claim 61, wherein the flow cell comprises a plurality of channels arranged in parallel, and one end of the channel is in communication with the first port.

63. The fluid path system according to claim 62, wherein one end of the channel is connected to the first port without tubes.

64. The fluid path system according to any one of claims 60-63, further comprising a first reservoir and a second reservoir, wherein
the first reservoir stores a sample solution under test and is connected to the second port, and
the second reservoir stores a reaction reagent and is connected to the common port.

65. A sequencing system, comprising the fluid path system according to any one of claims 60-64.

66. A method for controlling a system to achieve sequencing, wherein the system comprises a plurality of flow paths, a flow cell connected to the plurality of flow paths, a flow path selection valve and a pump, a first reservoir, and a second reservoir, wherein
the flow path selection valve comprises a manifold and a first valve element, the manifold is provided with a common port, a plurality of first ports, and a plurality of second ports, the first valve element is provided with a communication groove, and the first valve element is rotatable or slidable relative to the manifold, so that the communication groove selectively allows the common port to be in communication with the first port, or allows the first port to be in communication with the second port, thereby selecting different flow paths,
the first reservoir is connected to the second port,
the second reservoir is connected to the common port,
the first reservoir carries a first reaction solution comprising a nucleic acid molecule,
the second reservoir carries a second reaction solution comprising components necessary for nucleic acid sequencing, and
the pump is configured to allow liquid to flow through a flow path selected by the flow path selection valve; and
the method comprises:
switching the flow path selection valve to a first valve position, so that the communication groove allows the first port to be in communication with the second port, and thus allows the first reservoir to be in communication with the flow cell;
controlling the pump to operate to allow the first reaction solution to enter the flow cell to perform a first reaction in the case that the flow path selection valve is at the first valve position, the first reaction comprising attaching at least a portion of the nucleic acid molecule to the flow cell;
switching the flow path selection valve to a second valve position, so that the communication groove allows the first port to be in communication with the common port, and thus allows the second reservoir to be in communication with the flow cell; and
controlling the pump to operate to allow the second reaction solution to enter the flow cell to perform a second reaction in the case that the flow path selection valve is at the second valve position, the second reaction comprising allowing the nucleic acid molecule in the flow cell after the first reaction is performed to interact with the second reaction solution to perform a polymerization reaction and
detecting a signal from the reaction, so as to achieve sequence determination of the nucleic acid molecule.

67. The method according to claim 66, wherein the system further comprises a third reservoir connected to the common port, the third reservoir carries a third reaction solution comprising components necessary for amplification, and the method further comprises, after performing the first reaction and before performing the second reaction:
controlling the pump to operate to allow the third reaction solution in the third reservoir to enter the flow cell to perform a third reaction, the third reaction comprising allowing the nucleic acid molecule in the flow cell after the first reaction is performed to interact with the third reaction solution to achieve amplification of the nucleic acid molecule.

68. The method according to claim 66 or 67, wherein the system further comprises a fourth reservoir connected to the common port, the fourth reservoir carries a cleaning solution, and the method further comprises, before performing the first reaction:
controlling the pump to operate to allow the cleaning solution in the fourth reservoir to enter the flow cell in the case that the flow path selection valve is at the first valve position.

69. The method according to claim 66, further comprising, before performing the first reaction:
controlling the pump to operate to introduce air into the flow cell in the case that the flow path selection valve is at the first valve position.

70. The method according to claim 66, wherein the system further comprises a fourth reservoir connected to the common port, the fourth reservoir carries a cleaning solution, and the method further comprises, before performing the first reaction and/or performing the second reaction:
controlling the pump to operate to allow the cleaning solution in the fourth reservoir to enter the flow cell.

71. The method according to any one of claims 66-70, wherein the flow cell has a solid carrier surface on which a first sequencing primer is immobilized, at least one end of the nucleic acid molecule comprises at least a portion of a sequence capable of complementarily pairing with at least a portion of the first sequencing primer, and the first reaction comprises complementary pairing of at least a portion of the nucleic acid molecule with the first sequencing primer for attachment to the flow cell.

72. The method according to claim 71, wherein the second reaction solution comprises a first nucleotide, a first polymerase, and a cleavage reagent, and the controlling the pump to operate to allow the second reaction solution to enter the flow cell to perform a second reaction comprises:
(a) controlling the pump to operate to allow the first nucleotide and the first polymerase to enter the flow cell, and subjecting the flow cell to conditions suitable for a polymerization reaction to bind the first nucleotide to the nucleic acid molecule by extending the first sequencing primer, the first nucleotide comprising a base, a sugar unit, a cleavable blocking group, and a detectable label;
(b) exciting the detectable label and acquiring a signal from the detectable label;
(c) controlling the pump to operate to allow the cleavage reagent to enter the flow cell, so as to remove the cleavable blocking group and the detectable label of the first nucleotide; and
(d) repeating (a)-(c) at least once.

73. The method according to claim 72, wherein the second reaction solution further comprises a second nucleotide, and the controlling the pump to operate to allow the second reaction solution to enter the flow cell to perform a second reaction comprises, after (a):
controlling the pump to operate to allow the second nucleotide and the first polymerase to enter the flow cell, and subjecting the flow cell to conditions suitable for a polymerization reaction to allow the second nucleotide to bind to the nucleic acid molecule by proceeding to extend a product after (a), the second nucleotide comprising a base, a sugar unit, and a cleavable blocking group.

74. The method according to claim 71, wherein the second reaction solution comprises a first nucleotide, a second nucleotide, a first polymerase, a second polymerase, a cleavage reagent, and a second sequencing primer, at least one end of the nucleic acid molecule comprises at least a portion of a sequence capable of complementarily pairing with at least a portion of the second sequencing primer, and the controlling the pump to operate to allow the second reaction solution to enter the flow cell to perform a second reaction comprises:
(i) controlling the pump to operate to allow the first nucleotide and the first polymerase to enter the flow cell, and subjecting the flow cell to conditions suitable for a polymerization reaction to bind the first nucleotide to the nucleic acid molecule by extending the first sequencing primer to obtain a nascent strand, the first nucleotide being a nucleotide that carries neither a cleavable blocking group nor a detectable label;
(ii) controlling the pump to operate to allow the second nucleotide, the second polymerase, and the second sequencing primer to enter the flow cell, and subjecting the flow cell to conditions suitable for a polymerization reaction to bind the second sequencing primer to the nascent strand and bind the second nucleotide to the nascent strand by extending the second sequencing primer, the second nucleotide comprising a base, a sugar unit, a cleavable blocking group, and a detectable label;
(iii) exciting the detectable label and acquiring a signal from the detectable label;
(iv) controlling the pump to operate to allow the cleavage reagent to enter the flow cell, so as to remove the cleavable blocking group and the detectable label of the second nucleotide; and
(v) repeating (ii)-(iv) at least once.

75. The method according to claim 72, wherein the second reaction solution further comprises a third nucleotide, and the controlling the pump to operate to allow the second reaction solution to enter the flow cell to perform a second reaction comprises, after (ii):
controlling the pump to operate to allow the third nucleotide and the second polymerase to enter the flow cell, and subjecting the flow cell to conditions suitable for a polymerization reaction to bind the third nucleotide to the nascent strand by proceeding to extend a product after (ii), the third nucleotide comprising a base, a sugar unit, and a cleavable blocking group.

76. The method according to any one of claims 66-75, wherein the flow path selection valve comprises
a second valve element provided on the manifold, wherein the first valve element is provided on the second valve element, and the second valve element is provided with a first channel, a second channel, and a third channel, the first channel being in communication with the first port, the second channel being in communication with the second port, and the third channel being in communication with the common port;
in the case that the flow path selection valve is at the first valve position, the communication groove allows the first channel to be in communication with the second channel, and thus allows the first port to be in communication with the second port; and
in the case that the flow path selection valve is at the second valve position, the communication groove allows the first channel to be in communication with the third channel, and thus allows the first port to be in communication with the common port.

77. The method according to claim 76, wherein the manifold is provided with a cavity, and the second valve element is at least partially received in the cavity.

78. The method according to claim 77, wherein a bottom surface of the cavity is provided with a first connection port, a second connection port, and a third connection port, the first connection port allowing the first port to be in communication with the first channel, the second connection port allowing the second port to be in communication with the second channel, and the third connection port allowing the common port to be in communication with the third channel.

79. The method according to any one of claims 66-78, wherein at least two of the first port, the second port, and the common port are located on different sides of the manifold.

80. The method according to any one of claims 66-79, wherein the communication groove comprises first communication grooves and second communication grooves provided at intervals, wherein in the case that the flow path selection valve is at the first valve position, the first port is in communication with the second port through the first communication groove, and in the case that the flow path selection valve is at the second valve position, the first port is in communication with the common port through the second communication groove.

81. The method according to any one of claims 66-80, wherein the flow path selection valve comprises a drive component configured to drive the first valve element to rotate or slide.

82. The method according to claim 81, wherein the flow path selection valve comprises a slide block connected to the first valve element, wherein the slide block is connected to the drive component, and the drive component drives the first valve element to slide through the slide block; optionally, the drive component provides an electromagnetic drive.

83. The method according to claim 82, wherein the flow path selection valve comprises a housing detachably connected to the manifold, and the first valve element and the slide block are received in the housing.

84. The method according to claim 83, wherein the housing is provided with a guide groove, and the slide block comprises a connection part and a guide rail part connected to the connection part, the connection part being connected to the first valve element, and the guide rail part forming a fit with the guide groove to direct the slide block to drive the first valve element to slide.

85. The method according to any one of claims 66-84, wherein the system further comprises:
a reagent selection valve selecting a reagent from a plurality of reagents according to an analysis protocol; wherein
the flow path selection valve is fluidly connected between the reagent selection valve and the flow cell, the flow path selection valve selects a flow path for flowing through the flow cell from a plurality of flow paths passing through the flow cell according to the analysis protocol and directs the selected reagent to pass through the flow cell, and the pump allows the selected reagent to flow through the selected flow path according to the analysis protocol.

86. The method according to claim 85, wherein the flow cell comprises a plurality of channels arranged in parallel, and one end of the channel is in communication with the first port.

87. The method according to claim 86, wherein one end of the channel is connected to the first port without tubes.

88. A method for determining a nucleic acid sequence, comprising:
rotating a flow path selection valve provided with a first flow channel and a second flow channel to a first valve position to allow a first reservoir to be in communication with a reaction device, the first reservoir carrying a first reaction solution comprising a nucleic acid molecule;
allowing the first reaction solution to enter the reaction device through the first flow channel to perform a first reaction in the case that the flow path selection valve is at the first valve position, the first reaction comprising attaching at least a portion of the nucleic acid molecule to the reaction device;
rotating the flow path selection valve to a second valve position to allow a second reservoir to be in communication with the reaction device, the second reservoir carrying a second reaction solution comprising components necessary for nucleic acid sequencing; and
allowing the second reaction solution to enter the reaction device through the second flow channel to perform a second reaction in the case that the flow path selection valve is at the second valve position,
the second reaction comprising allowing the nucleic acid molecule in the reaction device after the first reaction is performed to interact with the second reaction solution to perform a polymerization reaction and detecting a signal from the reaction, so as to achieve sequence determination of the nucleic acid molecule.

89. The method according to claim 88, further comprising, after performing the first reaction and before performing the second reaction:
allowing a third reaction solution in a third reservoir to enter the reaction device through the first flow channel or the second flow channel to perform a third reaction, the third reaction comprising allowing the nucleic acid molecule in the reaction device after the first reaction is performed to interact with the third reaction solution to achieve amplification of the nucleic acid molecule, and the third reaction solution comprising components necessary for the amplification.

90. The method according to claim 88 or 89, further comprising, before performing the first reaction:
allowing a cleaning solution in a fourth reservoir to enter the reaction device through the first flow channel.

91. The method according to claim 90, further comprising, before performing the first reaction:
introducing air into the reaction device through the first flow channel.

92. The method according to claim 88, further comprising, before performing the first reaction and/or before performing the second reaction:
allowing a cleaning solution in a fourth reservoir to enter the reaction device through the first flow channel or the second flow channel.

93. The method according to any one of claims 88-92, wherein the reaction device has a solid carrier surface on which a first sequencing primer is immobilized, at least one end of the nucleic acid molecule comprises at least a portion of a sequence capable of complementarily pairing with at least a portion of the first sequencing primer, and the first reaction comprises complementary pairing of at least a portion of the nucleic acid molecule with the first sequencing primer for attachment to the reaction device.

94. The method according to claim 93, wherein the second reaction solution comprises a first nucleotide, a first polymerase, and a cleavage reagent, and the allowing the second reaction solution to enter the reaction device through the second flow channel to perform a second reaction comprises:
(a) allowing the first nucleotide and the first polymerase to enter the reaction device through the second flow channel, and subjecting the reaction device to conditions suitable for a polymerization reaction to bind the first nucleotide to the nucleic acid molecule by extending the first sequencing primer, the first nucleotide comprising a base, a sugar unit, a cleavable blocking group, and a detectable label;
(b) exciting the detectable label and acquiring a signal from the detectable label;
(c) allowing the cleavage reagent to enter the reaction device through the second flow channel, so as to remove the cleavable blocking group and the detectable label of the first nucleotide; and
(d) repeating (a)-(c) at least once.

95. The method according to claim 94, wherein the second reaction solution further comprises a second nucleotide, and the allowing the second reaction solution to enter the reaction device through the second flow channel to perform a second reaction further comprises, after (a):
allowing the second nucleotide and the first polymerase to enter the reaction device through the second flow channel, and subjecting the reaction device to conditions suitable for a polymerization reaction to allow the second nucleotide to bind to the nucleic acid molecule by proceeding to extend a product after (a), the second nucleotide comprising a base, a sugar unit, and a cleavable blocking group.

96. The method according to claim 93, wherein the second reaction solution comprises a third nucleotide, a fourth nucleotide, a second polymerase, a third polymerase, a cleavage reagent, and a second sequencing primer, at least one end of the nucleic acid molecule comprises at least a portion of a sequence capable of complementarily pairing with at least a portion of the second sequencing primer, and the allowing the second reaction solution to enter the reaction device through the second flow channel to perform a second reaction comprises:
(i) allowing the third nucleotide and the second polymerase to enter the reaction device through the second flow channel, and subjecting the reaction device to conditions suitable for a polymerization reaction to bind the third nucleotide to the nucleic acid molecule by extending the first sequencing primer to obtain a nascent strand, the third nucleotide being a nucleotide that carries neither a cleavable blocking group nor a detectable label;
(ii) allowing the fourth nucleotide, the third polymerase, and the second sequencing primer to enter the reaction device through the second flow channel, and subjecting the reaction device to conditions suitable for a polymerization reaction to bind the second sequencing primer to the nascent strand and bind the fourth nucleotide to the nascent strand by extending the second sequencing primer, the fourth nucleotide comprising a base, a sugar unit, a cleavable blocking group, and a detectable label;
(iii) exciting the detectable label and acquiring a signal from the detectable label;
(iv) allowing the cleavage reagent to enter the reaction device through the second flow channel, so as to remove the cleavable blocking group and the detectable label of the fourth nucleotide; and
(v) repeating (ii)-(iv) at least once.

97. The method according to claim 96, wherein the second reaction solution further comprises a fifth nucleotide, and the allowing the second reaction solution to enter the reaction device through the second flow channel to perform a second reaction further comprises, after (ii):
allowing the fifth nucleotide and the third polymerase to enter the reaction device through the second flow channel, and subjecting the reaction device to conditions suitable for a polymerization reaction to bind the fifth nucleotide to the nascent strand by proceeding to extend a product after (ii), the fifth nucleotide comprising a base, a sugar unit, and a cleavable blocking group.

98. A nucleic acid sequence determination system, comprising a fluid path system, a detection assembly, and a controller connected to each other, wherein the fluid path system comprises a fluid network and a pump assembly in communication with the fluid network, the fluid network comprises a first reservoir, a second reservoir, a flow path selection valve, and a reaction device, the flow path selection valve is provided with a first flow channel and a second flow channel, the first flow channel allows the first reservoir to be in communication with the reaction device, and the second flow channel allows the second reservoir to be in communication with the reaction device; the detection assembly is configured to detect a signal from the reaction device during a designated reaction; and the controller is configured to control the fluid path system and the detection assembly to perform the steps of the method according to any one of claims 88-97.

99. The system according to claim 103, wherein the flow path selection valve is provided with a common port, a plurality of first ports, a plurality of second ports, and a plurality of communication grooves, wherein the communication groove selectively allows the common port to be in communication with the first port, or allows the first port to be in communication with the second port; in the case that the flow path selection valve is at a first valve position, the first port is in communication with the second port through the communication groove to form the first flow channel; and
in the case that the flow path selection valve is at a second valve position, the first port is in communication with the common port through the communication groove to form the second flow channel.

100. The system according to claim 99, wherein the flow path selection valve comprises a stator and a rotor provided opposite to the stator, wherein the stator is provided with the common port, the plurality of first ports, and the plurality of second ports, and the rotor is provided with at least a portion of the plurality of communication grooves.

101. The system according to claim 99, wherein the flow path selection valve comprises a stator and a rotor provided opposite to the stator, wherein the rotor is provided with the common port, the plurality of first ports, and the plurality of second ports, and the stator is provided with at least a portion of the plurality of communication grooves.

102. The system according to claim 100 or 101, wherein the communication groove comprises a first communication groove and a second communication groove capable of being in communication with each other, and the first communication groove and the second communication groove are provided on the rotor and the stator, respectively.

103. The system according to claim 102, wherein one communication groove is composed of one first communication groove and one second communication groove, wherein one first communication groove has two ends, one end of each second communication groove is in communication with one first port, and the other end is in selective communication with one second port; and one second communication groove has two ends, one end of each second communication groove is in communication with the common port, and the other end is in selective communication with one first port.

104. The system according to claim 102 or 103, wherein the stator comprises a stator end face, and the rotor comprises a rotor end face, wherein the stator end face and the rotor end face are abutted together, the first communication groove is formed in the rotor end face, and the second communication groove is formed in the stator end face.

105. The system according to any one of claims 99-104, wherein the plurality of first ports and the plurality of second ports are all provided around the common port.

106. The system according to any one of claims 99-104, wherein the flow path selection valve has a central axis, the plurality of first ports are arranged at intervals on a circular plane having the central axis as an axis, and/or
the plurality of second ports are arranged at intervals on a circular plane having the central axis as an axis.

107. The system according to claim 106, wherein the plurality of first ports and the plurality of second ports are located on the same circular plane.

108. The system according to any one of claims 99-107, wherein the plurality of first ports are arranged at intervals along a circumferential direction of the stator or the rotor; and/or
the plurality of second ports are arranged at intervals along a circumferential direction of the stator or the rotor.

109. The system according to claim 108, wherein the plurality of first ports and the plurality of second ports are located on the same circumference.

110. The system according to any one of claims 100-109, wherein the flow path selection valve comprises a valve body, and the stator and the rotor are both received in the valve body.

111. The system according to claim 110, wherein the flow path selection valve comprises a valve head covering the stator and provided on the valve body, and the valve head has a first interface, a second interface, and a third interface, the first interface being in communication with the common port, the second interface being in communication with the first port, and the third interface being in communication with the second port.

112. The system according to claim 111, wherein the flow path selection valve comprises a positioning structure configured to position the valve head and the stator.

113. The system according to claim 112, wherein the positioning structure comprises a positioning pin inserted in the valve head and the stator.

114. The system according to claim 110, wherein the flow path selection valve further comprises a drive component connected to the rotor, and the drive component is provided on the valve body and is configured to drive the rotor to rotate.

115. The system according to any one of claims 99-114, wherein the common port, the plurality of first ports, and the plurality of second ports are separated from one another when the flow path selection valve is at a third valve position.

116. The system according to any one of claims 98-115, wherein the flow path selection valve is provided upstream of the reaction device.

117. A computer-readable storage medium configured to store a program executed by a computer, wherein executing the program comprises implementing the method according to any one of claims 94-102.

118. A system configured to perform the method according to any one of claims 88-97.

119. A computer program product comprising instructions, wherein the method according to any one of claims 88-97 is implemented when the program is executed by a computer.

120. A nucleic acid sequence determination system, comprising the computer program product according to claim 119.

121. A nucleic acid sequence determination system, comprising:
a first control module configured to rotate a rotary valve provided with a first flow channel and a second flow channel to a first valve position to allow a first reservoir to be in communication with a reaction device, the first reservoir carrying a first reaction solution comprising a nucleic acid molecule;
a second control module configured to allow the first reaction solution to enter the reaction device through the first flow channel to perform a first reaction in the case that the rotary valve is at the first valve position, the first reaction comprising attaching at least a portion of the nucleic acid molecule to the reaction device;
a third control module configured to rotate the rotary valve to a second valve position to allow a second reservoir to be in communication with the reaction device, the second reservoir carrying a second reaction solution comprising components necessary for nucleic acid sequencing; and
a fourth control module configured to allow the second reaction solution to enter the reaction device through the second flow channel to perform a second reaction in the case that the rotary valve is at the second valve position, the second reaction comprising allowing the nucleic acid molecule in the reaction device after the first reaction is performed to interact with the second reaction solution to perform a polymerization reaction and detecting a signal from the reaction, so as to achieve sequence determination of the nucleic acid molecule.
